# EUROPÄISCHE PATENTSCHRIFT

(11) **EP 0 501 129 B1**
(45) Veröffentlichungstag und Bekanntmachung des Hinweises auf die Patenterteilung: **14.07.1999**
(21) Anmeldenummer: 92100826.4
(22) Anmeldetag: 20.01.1992
(51) Int. Cl.: C07D 487/04, C07D 491/20, A01N 43/90, C07F 9/6561

(54) **Substituierte bicyclische 3-Aryl-pyrrolidin-2,4-dion-Derivate**
Substituted bicyclic 3-arylpyrrolidin-2,4-dion derivatives
Dérivés bicycliques substitués de la 3-aryl-pyrrolidin-2,4-dione

(30) Priorität: 31.01.1991 DE 4102778
(43) Veröffentlichungstag der Anmeldung: 02.09.1992
(73) Patentinhaber: BAYER AG, 51368 Leverkusen (DE)
(72) Erfinder: Santel, Hans-Joachim, Dr., W-5090 Leverkusen (DE); Schmidt, Robert R., Dr., W-5060 Bergisch Gladbach 2 (DE); Wachendorff-Neumann, Ulrike, Dr., W-4019 Monheim (DE); Erdelen, Christoph, Dr., W-5653 Leichlingen (DE); Bretschneider, Thomas, Dr., W-5200 Siegburg (DE); Fischer, Reiner, Dr., W-4019 Monheim 2 (DE); Hagemann, Hermann, Dr., W-5090 Leverkusen (DE); Krüger, Bernd-Wieland, Dr., W-5060 Bergisch Gladbach 2 (DE); Lürssen, Klaus, Dr., W-5060 Bergisch Gladbach 2 (DE)

(56) Entgegenhaltungen:
- EP-A- 0 355 599

## Beschreibung

Die Erfindung betrifft neue bicyclische 3-Aryl-pyrrolidin-2,4-dion-Derivate, mehrere Verfahren zu ihrer Herstellung und ihre Verwendung als Schädlingsbekämpfungsmittel (insbesondere als Insektizide und Akarizide) und als Herbizide.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger Liebigs Ann. Chem. 1985 1095 synthetisiert. Eine biologische Wirksamkeit dieser Verbindungen wurde nicht beschrieben.

In EP-A 0 262 399 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt sind außerdem unsubstituierte, bicyclische 3-Aryl-pyrrolidin-2,4-dion-Derivate (EP-A 355 599), sowie substituierte monocyclische 3-Aryl-pyrrolidin-2,4-dion-Derivate (EP-A 377 893).

Es wurden nun neue substituierte bicyclische 3-Aryl-pyrrolidin-2,4-dion-Derivate der Formel (I) 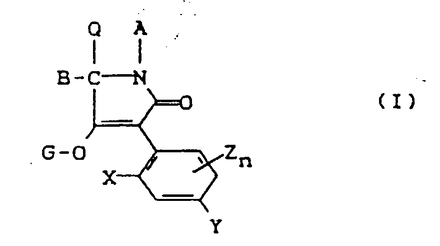 gefunden,
in welcher
- A und Q: gemeinsam für eine jeweils einfach bis dreifach, gleich oder verschieden substituierte Alkandiyl-, Alkendiyl-, Alkendienyl- oder Alkentrienylgruppierung mit 3 bis 6 Kohlenstoffatomen stehen, welche jeweils durch Halogen, Hydroxy, Mercapto, jeweils gegebenenfalls durch Halogen substituiertes Alkyl mit 1 bis 10 Kohlenstoffatomen, Alkylcarbonyloxy mit 1 bis 8 Kohlenstoffatomen, Alkoxy mit 1 bis 6 Kohlenstoffatomen, Alkylthio mit 1 bis 6 Kohlenstoffatomen, Cycloalkyl mit 3 bis 7 Kohlenstoffatomen oder Aryl mit 6 bis 10 Kohlenstoffatomen substituiert ist und welche außerdem gegebenenfalls durch eine der nachstehenden Gruppierungen 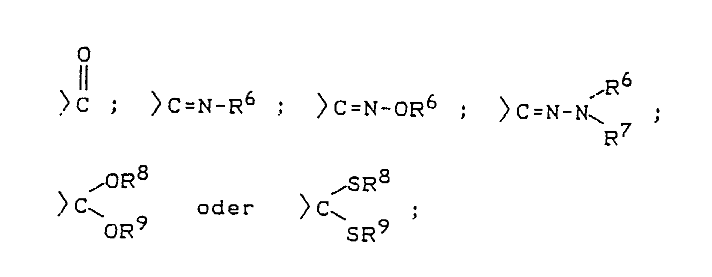 unterbrochen ist.
- B: für Wasserstoff oder Alkyl mit 1 bis 6 Kohlenstoffatomen steht,
- X: für C₁-C₆-Alkyl, Halogen oder C₁-C₆-Alkoxy steht,
- Y: für Wasserstoff, C₁-C₆-Alkyl, Halogen, C₁-C₆-Alkoxy oder C₁-C₃-Halogenalkyl steht,
- Z: für C₁-C₆-Alkyl; Halogen oder C₁-C₆-Alkoxy steht,
- n: für eine Zahl 0, 1, 2 oder 3 steht,
- G: für Wasserstoff (a) oder für die Gruppen 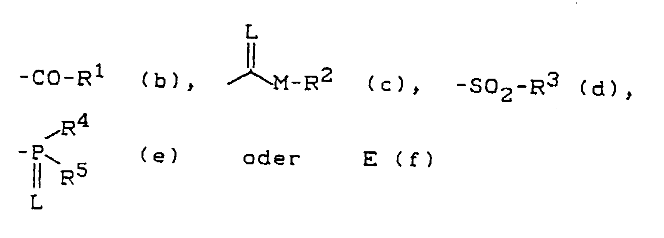 steht,
in welchen
- E: für ein Metallionäquivalent oder ein Ammoniumion steht,
- L und M: für Sauerstoff und/oder Schwefel steht,
- R¹: für gegebenenfalls durch Halogen substituiertes C₁-C₂₀-Alkyl, C₂-C₂₀-Alkenyl, C₁-C₈-Alkoxy-C₂-C₈-alkyl, C₁-C₈-Alkylthio-C₂-C₈-alkyl, C₁-C₈-Polyalkoxy-C₂-C₈-alkyl oder Cycloalkyl mit 3 bis 8 Ringatomen, das durch Sauerstoff- und/oder Schwefelatome unterbrochen sein kann, steht,
für gegebenenfalls durch Halogen, Nitro, C₁-C₆-Alkyl, C₁-C₆-Alkoxy, C₁-C₆-Halogenalkyl, C₁-C₆-Halogenalkoxy substituiertes Phenyl,
für gegebenenfalls durch Halogen, C₁-C₆-Alkyl, C₁-C₆-Alkoxy, C₁-C₆-Halogenalkyl, C₁-C₆-Halogenalkoxy substituiertes Phenyl-C₁-C₆-alkyl steht,
für gegebenenfalls durch Halogen und/oder C₁-C₆-Alkyl substituiertes Hetaryl steht,
für gegebenenfalls durch Halogen und C₁-C₆-Alkyl substituiertes Phenoxy-C₁-C₆-alkyl steht,
für gegebenenfalls durch Halogen. Amino und C₁-C₆-Alkyl substituiertes Hetaryloxy-C₁-C₆-Alkyl steht,
- R²: für gegebenenfalls durch Halogen substituiertes C₁-C₂₀-Alkyl, C₂-C₂₀-Alkenyl, C₁-C₈-Alkoxy-C₂-C₈-alkyl, C₁-C₈-Polyalkoxy-C₂-C₈-alkyl steht,
für gegebenenfalls durch Halogen, Nitro, C₁-C₆-Alkyl, C₁-C₆-Alkoxy, C₁-C₆-Halogenalkyl substituiertes Phenyl oder Benzyl steht,
- R³, R⁴ und R⁵: unabhängig voneinander für gegebenenfalls durch Halogen substituiertes C₁-C₈-Alkyl, C₁-C₈-Alkoxy, C₁-C₈-Alkylamino, Di-(C₁-C₈)-alkylamino, C₁-C₈-Alkylthio, C₂-C₅-Alkenylthio, C₂-C₅-Alkinylthio; C₃-C₇-Cycloalkylthio, für gegebenenfalls durch Halogen, Nitro, Cyano, C₁-C₄-Alkoxy, C₁-C₄-Halogenalkaxy, C₁-C₄-Alkylthio, C₁-C₄-Halogenalkylthio, C₁-C₄-Alkyl, C₁-C₄-Halogenalkyl substituiertes Phenyl, Phenoxy oder Phenylthio stehen,
- R⁶ und R⁷: unabhängig voneinander für Wasserstoff, gegebenenfalls durch Halogen substituiertes C₁-C₂₀-Alkyl, C₁-C₂₀-Alkoxy, C₂-C₈-Alkenyl, C₁-C₂₀-Alkoxy-C₁-C₂₀-alkyl, für gegebenenfalls durch Halogen, C₁-C₂₀-Halogenalkyl, C₁-C₂₀-Alkyl oder C₁-C₂₀-Alkoxy substituiertes Phenyl, für gegebenenfalls durch Halogen, C₁-C₂₀-Alkyl, C₁-C₂₀-Halogenalkyl oder C₁-C₂₀-Alkoxy substituiertes Benzyl steht oder zusammen für einen gegebenenfalls durch Sauerstoff unterbrochenen C₂-C₆-Alkylenring stehen,
- R⁸ und R⁹: unabhängig voneinander für Wasserstoff oder Alkyl mit 1 bis 6 Kohlenstoffatomen stehen oder
- R⁸ und R⁹: zusammen für einen Alkandiylrest mit 2 bis 4 Kohlenstoffatomen stehen.

Unter Einbeziehung der verschiedenen Bedeutungen (a), (b), (c), (d), (e) und (f) der Gruppe G der allgemeinen Formel (I) ergeben sich folgende hauptsächlichen Strukturen (Ia) bis (If): 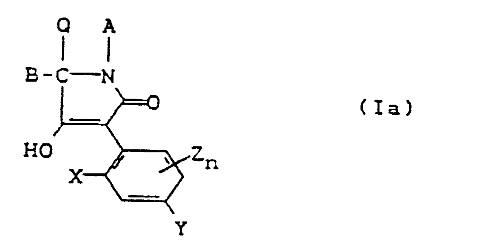 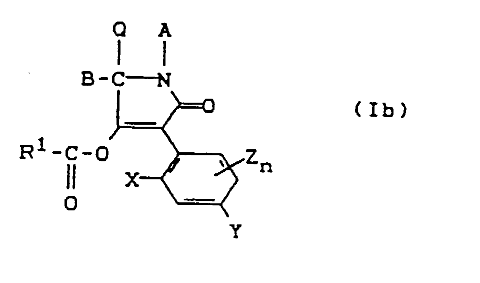 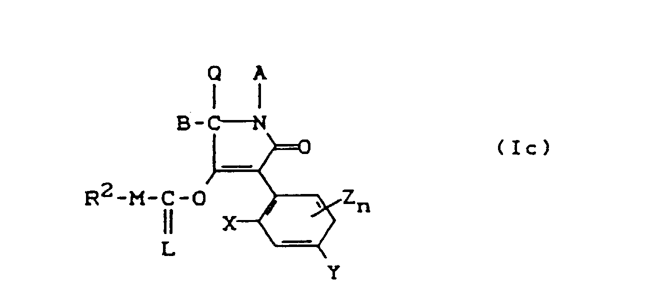 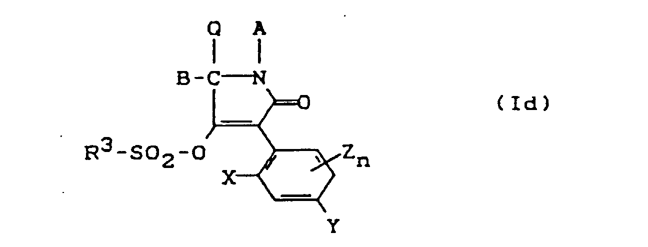 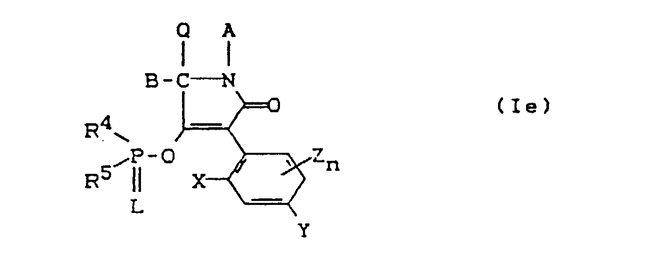 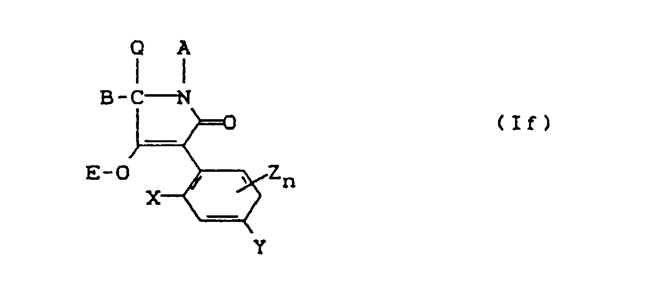 worin
A, B, Q, E, L, M, X, Y, Zₙ, R¹, R², R³, R⁴, R⁵, R⁶ und R⁷
die oben angegebenen Bedeutungen besitzen.

Aufgrund eines oder mehrerer Chiralitätszentren, fallen die Verbindungen der Formel (Ia) - (If) im allgemeinen als Stereoisomerengemisch an. Sie können sowohl in Form ihrer Diastereomerengemische als auch als reine Diastereomere oder Enantiomere verwendet werden.

Weiterhin wurde gefunden, daß man die neuen substituierten bicyclischen 3-Aryl-pyrrolidin-2,4-dion-Derivate der Formel (I) nach einem der im folgenden beschriebenen Verfahren erhält.
(A-α) Man erhält 3-Aryl-pyrrolidin-2,4-dione bzw. deren Enole der Formel (Ia) 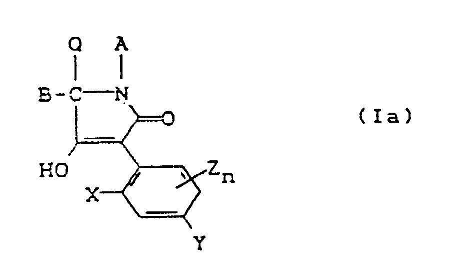 in welcher
   - A, B, Q, X, Y, Z und n: die oben angegebene Bedeutung haben,
   wenn man
   N-Acylaminosäureester der Formel (II) 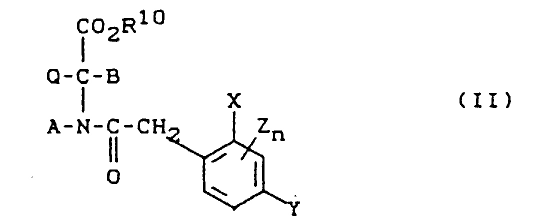 in welcher
   - A, B, Q, X, Y, Z und n: die oben angegebene Bedeutung haben,
   und
   - R¹⁰: für Alkyl steht,
   in Gegenwart eines Verdünnungsmittels und in Gegenwart einer Base intramolekularer kondensiert;
   oder
(A-β) wenn man die nach Verfahren (Aα) erhaltenen Verbindungen der Formel (Ia-a) 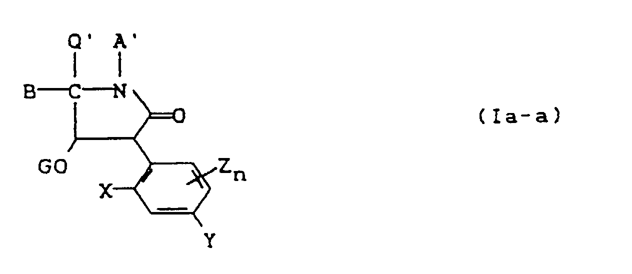 in welcher
   B, X, Y, Z, n und G die oben angegebene Bedeutung haben und
   A' und Q' gemeinsam für eine durch Hydroxysubstituierte Alkandiyl-, Alkendiyl, Alkendienyl- oder Alkentrienyl-gruppierung stehen,
   entweder an der Hydroxy-Gruppierung nach allgemein üblichen Verfahren in Ester oder Ether umwandelt oder die Hydroxy-Gruppierung in einer ersten Stufe mit Hilfe üblicher Oxidationsmethoden, wie beispielsweise der Swern-Oxidation oder der Pfitzner-Moffart-Oxidation in die entsprechenden Ketone überführt und diese in einer zweiten Stufe nach ebenfalls üblichen Methoden mit Anionen, Alkoholen, Diolen, Mercaptanen und Dithiolen weiter umsetzt,
   oder
(B) man erhält Verbindungen der Formel (Ib) 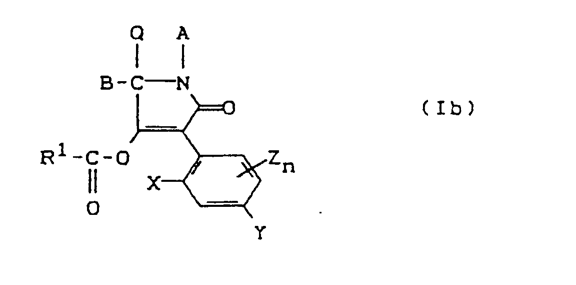 in welcher
   - A, B, Q, X, Y, Z, R¹ und n: die oben angegebene Bedeutung haben,
   wenn man Verbindungen der Formel (Ia), 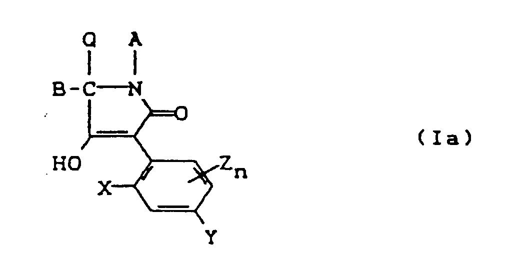 in welcher
   - A, B, Q, X, Y, Z und n: die oben angegebene Bedeutung haben,

   α) mit Säurehalogeniden der allgemeinen Formel (III) 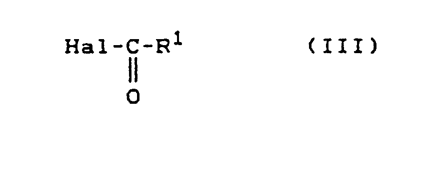 in welcher
      - R¹: die oben angegebene Bedeutung hat und
      - Hal: für Halogen, insbesondere Chlor und Brom steht,
      gegebenenfalls in Gegenwart eines Verdünnungsmittels und gegebenenfalls in Gegenwart eines Säurebindemittels umsetzt
      oder
   β) mit Carbonsäureanhydriden der allgemeinen Formel (IV)

      R¹-CO-O-CO-R¹ (IV)

      in welcher
      - R¹: die oben angegebene Bedeutung hat,
      gegebenenfalls in Gegenwart eines Verdünnungsmittels und gegebenenfalls in Gegenwart eines Säurebindemittels,
   umsetzt;
   oder
(C) man erhält Verbindungen der Formel (Ic-1) 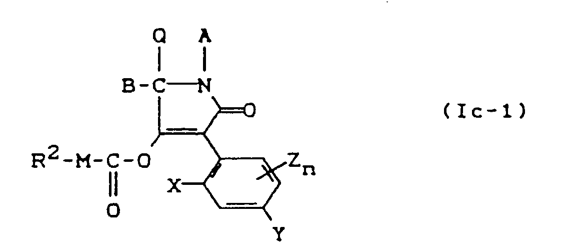 in welcher
   - A, B, Q, X, Y, Z, R² und n: die oben angegebene Bedeutung haben,
   und
   - M: für Sauerstoff oder Schwefel steht,
   wenn man Verbindungen der Formel (Ia) 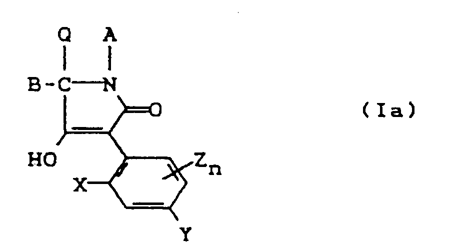 in welcher
   - A, B, Q, X, Y, Z und n: die oben angegebene Bedeutung haben,
   mit Chlorameisensäureester oder Chlorameisensäurethiolester der allgemeinen Formel (V)

   R²-M-CO-Cl (V)

   in welcher
   - R² und M: die oben angegebene Bedeutung haben,
   gegebenenfalls in Gegenwart eines Verdünnungsmittels und gegebenenfalls in Gegenwart eines Säurebindemittels umsetzt;
   oder
(D) man erhält Verbindungen der Formel (Ic-2) 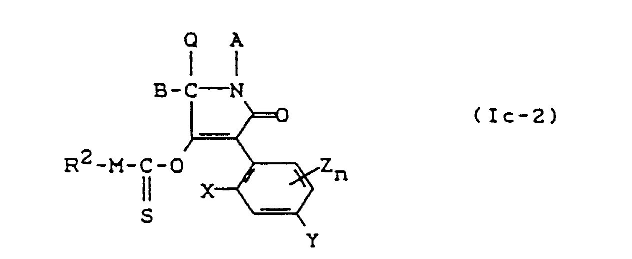 in welcher
   - A, B, Q, R², X, Y, Z und n: die oben angegebene Bedeutung haben
   und
   - M: für Sauerstoff oder Schwefel steht,
   wenn man Verbindungen der Formel (Ia) 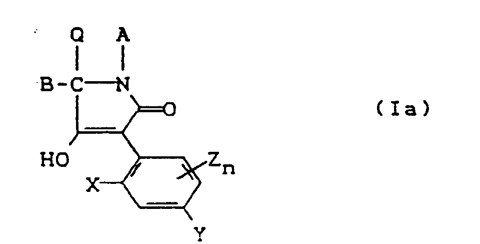 in welcher
   - A, B, Q, X, Y, Z und n: die oben angegebene Bedeutung haben,

   α) mit Chlormonothioameisensäureestern oder Chlordithioameisensäureestern der allgemeinen Formel (VI) 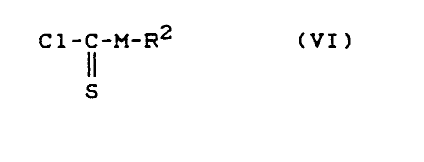 in welcher
      - M und R²: die oben angegebene Bedeutung haben,
      gegebenenfalls in Gegenwart eines Verdünnungsmittels und gegebenenfalls in Gegenwart eines Säurebindemittels umsetzt,
      oder
   β) mit Schwefelkohlenstoff und anschließend mit Alkylhalogeniden der allgemeinen Formel (VII)

      R²-Hal (VII)

      in welcher
      - R²: die oben angegebene Bedeutung hat
      und
      - Hal: für Chlor, Brom, Jod steht,
   umsetzt;
   oder
(E) man erhält Verbindungen der Formel (Id) 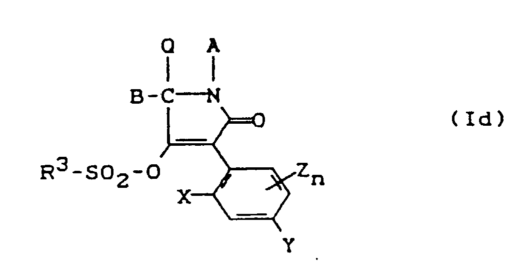 in welcher
   - A, B, Q, X, Y, Z, R³ und n: die oben angegebene Bedeutung haben,
   wenn man Verbindungen der Formel (Ia) 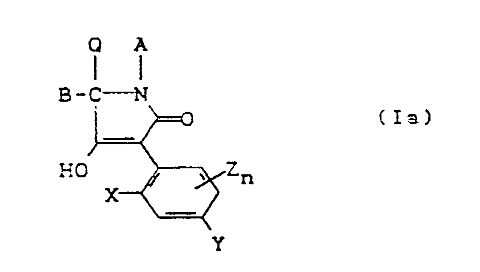 in welcher
   - A, B, Q, X, Y, Z und n: die oben angegebene Bedeutung haben,
   mit Sulfonsäurechloriden der allgemeinen Formel (VIII)

   R³-SO₂-Cl (VIII)

   in welcher
   - R³: die oben angegebene Bedeutung hat,
   gegebenenfalls in Gegenwart eines Verdünnungsmittels und gegebenenfalls in Gegenwart eines Säurebindemittels,
   umsetzt;
   oder
(F) man erhält 3-Aryl-pyrrolidin-2,4-dione der Formel (Ie) 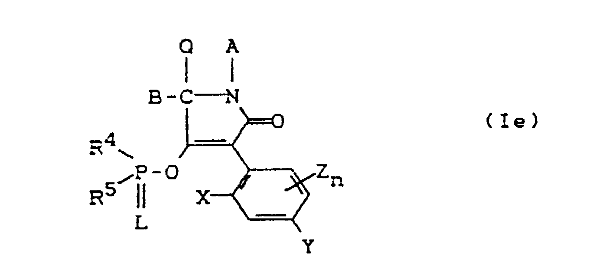 in welcher
   - A, B, Q, L, X, Y, Z, R⁴, R⁵ und n: die oben angegebene Bedeutung haben,
   wenn man
   3-Aryl-pyrrolidin-2,4-dione der Formel (Ia) bzw. deren Enole 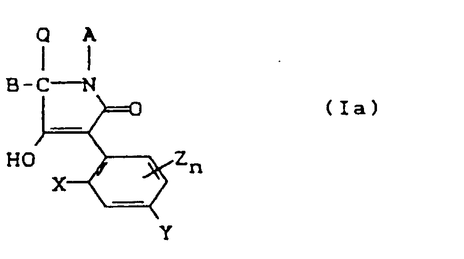 in welcher
   - A, B, Q, X, Y, Z und n: die oben angegebene Bedeutung haben,
   mit Phosphorverbindungen der allgemeinen Formel (IX) 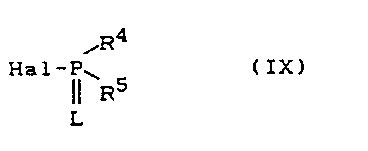 in welcher
   - L, R⁴ und R⁵: die oben angegebene Bedeutung haben
   und
   - Hal: für Halogen, insbesondere Chlor und Brom steht,
   umsetzt;
   oder
(G) man erhält Verbindungen der Formel (If) 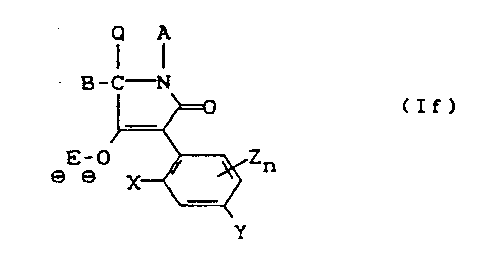 in welcher
   - A, B, Q, X, Y, Z und n: die oben angegebene Bedeutung haben,
   und
   - E: für ein Metallionäquivalent oder für ein Ammoniumion steht,
   wenn man Verbindungen der Formel (Ia) 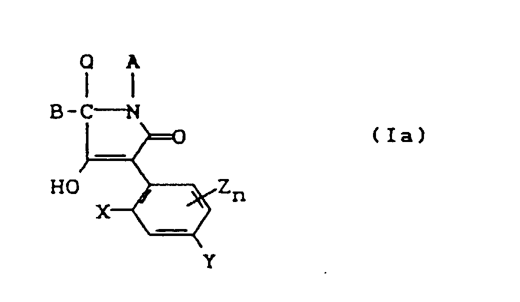 in welcher
   - A, B, Q, X, Y, Z und n: die oben angegebene Bedeutung haben,
   mit Metallhydroxiden oder Aminen der allgemeinen Formeln (X) und (XI) 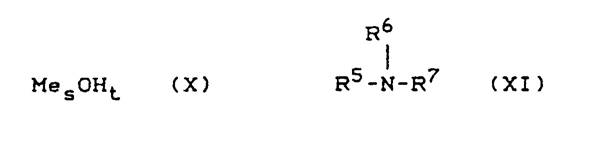 in welchen
   - Me: für ein- oder zweiwertige Metallionen,
   - s und t: für die Zahl 1 und 2 und
   - R⁵, R⁶ und R⁷: unabhängig voneinander für Wasserstoff und Alkyl
   stehen,
   gegebenenfalls in Gegenwart eines Verdünnungsmittels, umsetzt.

Weiterhin wurde gefunden, daß sich die neuen bicyclischen 3-Arylpyrrolidin-2,4-dion-Derivate der Formel (I) durch hervorragende insektizide, akarizide und herbizide Wirkungen auszeichnen.

Für die allgemeinen Formeln der vorliegenden Anmeldung gilt:
- A und Q: stehen besonders bevorzugt gemeinsam für eine jeweils einfach bis dreifach, gleich oder verschieden substituierte Alkandiyl-, Alkendiyl-, Alkendienyl-gruppierung mit 3 bis 5 Kohlenstoffatomen, welche jeweils durch Fluor, Chlor, Brom, Hydroxy, Mercapto, jeweils gegebenenfalls durch Halogen substituiertes Alkyl mit 1 bis 8 Kohlenstoffatomen, Alkylcarbonyloxy mit 1 bis 6 Kohlenstoffatomen, Alkoxy mit 1 bis 4 Kohlenstoffatomen, Alkylthio mit 1 bis 4 Kohlenstoffatomen, Cycloalkyl mit 5 bis 7 Kohlenstoffatomen oder Phenyl substituiert ist und welche außerdem gegebenenfalls durch eine der nachstehenden Gruppierungen 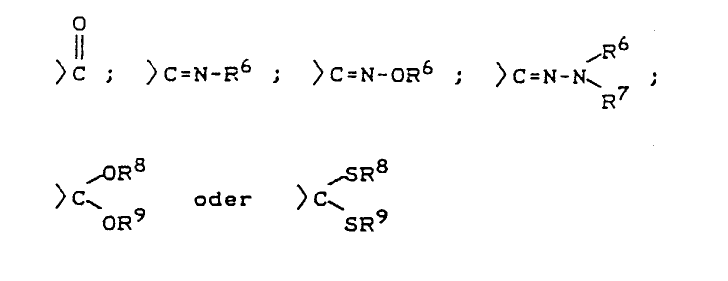 unterbrochen ist, wobei
- R⁶ und R⁷: unabhängig voneinander für Wasserstoff, gegebenenfalls durch Halogen substituiertes C₁-C₂₀-Alkyl, C₁-C₂₀-Alkoxy, C₂-C₈-Alkenyl, C₁-C₂₀-Alkoxy-C₁-C₂₀-alkyl, für gegebenenfalls durch Halogen, C₁-C₅-Halogenalkyl, C₁-C₅-Alkyl oder C₁-C₅-Alkoxy substituiertes Phenyl, für gegebenenfalls durch Halogen, C₁-C₅-Alkyl, C₁-C₅-Halogenalkyl oder C₁-C₅-Alkoxy substituiertes Benzyl steht,
- R⁸ und R⁹: unabhängig voneinander für Wasserstoff oder Alkyl mit 1 bis 4 Kohlenstoffatomen stehen oder
- R⁸ und R⁹: zusammen für einen Alkandiylrest mit 2 oder 3 Kohlenstoffatomen stehen.
- A und Q: stehen ganz besonders bevorzugt gemeinsam für eine jeweils einfach bis dreifach, gleich oder verschieden substituierte Alkandiyl-, Alkendiyl- oder Alkendienylgruppierung mit 3 bis 4 Kohlenstoffatomen, welche durch Fluor, Chlor, Brom, Hydroxy, Mercapto, jeweils gegebenenfalls durch Fluor, Chlor, Brom substituiertes Alkyl mit 1 bis 6 Kohlenstoffatomen, Alkylcarbonyloxy mit 1 bis 4 Kohlenstoffatomen, Alkoxy mit 1 oder 2 Kohlenstoffatomen, Alkylthio mit 1 oder 2 Kohlenstoffatomen oder Cycloalkyl mit 5 oder 6 Kohlenstoffatomen substituiert ist und welche außerdem gegebenenfalls durch eine der nachstehenden Gruppierungen 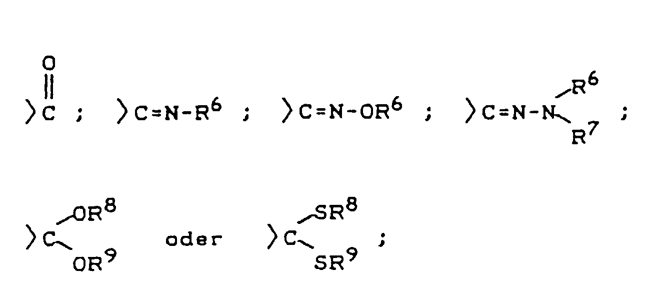 unterbrochen ist,
wobei
- R⁶ und R⁷: unabhängig voneinander für gegebenenfalls durch Fluor, Chlor, Brom substituiertes C₁-C₁₀-Alkyl, C₁-C₁₀-Alkoxy, C₁-C₁₀-Alkoxy-(C₁-C₁₀)-alkyl, für gegebenenfalls durch Fluor, Chlor, Brom, C₁-C₂₀-Halogenalkyl, C₁-C₂₀-Alkyl oder C₁-C₄-Alkoxy substituiertes Phenyl, für gegebenenfalls durch Fluor, Chlor, Brom, C₁-C₄-Alkyl, C₁-C₄-Halogenalkyl oder C₁-C₄-Alkoxy substituiertes Benzyl steht,
- R⁸ und R⁹: unabhängig voneinander für Wasserstoff oder Alkyl mit 1 bis 4 Kohlenstoffatomen stehen oder
- R⁸ und R⁹: zusammen für einen Alkandiylrest mit 2 oder 3 Kohlenstoffatomen stehen.
- B: steht besonders bevorzugt für Wasserstoff oder Alkyl mit 1 bis 4 Kohlenstoffatomen.
- B: steht ganz besonders bevorzugt für Wasserstoff, Methyl oder Ethyl.
- X: steht besonders bevorzugt für C₁-C₄-Alkyl, Halogen oder C₁-C₄-Alkoxy.
- X: steht ganz besonders bevorzugt für Methyl, Ethyl, Propyl, 2-Propyl, Fluor, Chlor, Brom, Methoxy oder Ethoxy.
- Y: steht besonders bevorzugt für Wasserstoff, C₁-C₄-Alkyl, Halogen, C₁-C₄-Alkoxy oder C₁-C₂-Halogenalkyl.
- Y: steht ganz besonders bevorzugt für Wasserstoff, Methyl, Ethyl, Propyl, i-Propyl, Butyl, i-Butyl, tert,-Butyl, Fluor, Chlor, Brom, Methoxy, Ethoxy oder Trifluormethyl.
- Z: steht besonders bevorzugt für C₁-C₄-Alkyl, Halogen oder C₁-C₄-Alkoxy.
- Z: steht ganz besonders bevorzugt für Methyl, Ethyl, Propyl, i-Propyl, Butyl, i-Butyl, tert.-Butyl, Fluor, Chlor, Brom, Methoxy oder Ethoxy.
- G: steht besonders bevorzugt für Wasserstoff (a) oder für die Gruppen 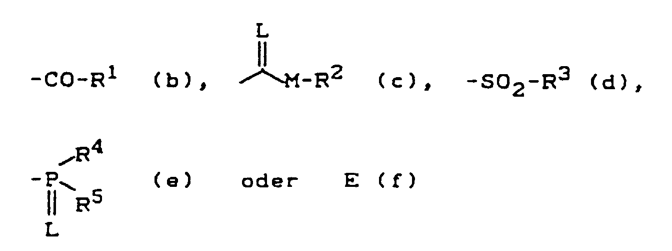
in welchen
- E: für ein Metallionäquivalent oder ein Ammoniumion steht,
- L und M: jeweils für Sauerstoff und/oder Schwefel steht,
- R¹: für gegebenenfalls durch Halogen substituiertes C₁-C₁₆-Alkyl, C₂-C₁₆-Alkenyl, C₁-C₆-Alkoxy-C₂-C₆-alkyl, C₁-C₁₆-Alkylthio-C₂-C₆-alkyl, C₁-C₆-Polyalkoxy-C₂-C₆-alkyl oder Cycloalkyl mit 3 bis 7 Ringatomen, das durch 1-2 Sauerstoff- und/oder Schwefelatome unterbrochen sein kann, steht,
für gegebenenfalls durch Halogen-, Nitro-, C₁-C₄-Alkyl-, C₁-C₄-Alkoxy-, C₁-C₃-Halogenalkyl-, C₁-C₃-Halogenalkoxy-substituiertes Phenyl,
für gegebenenfalls durch Halogen-, C₁-C₄-Alkyl-, C₁-C₄-Alkoxy-, C₁-C₃-Halogenalkyl-, C₁-C₃-Halogenalkoxy-substituiertes Phenyl-C₁-C₄-alkyl steht,
für gegebenenfalls durch Halogen- und/oder C₁-C₆-Alkyl-substituiertes Hetaryl steht,
für gegebenenfalls durch Halogen- und C₁-C₄-Alkyl-substituiertes Phenoxy-C₁-C₅-alkyl steht,
für gegebenenfalls durch Halogen, Amino und C₁-C₄-Alkyl-substituiertes Hetaryloxy-C₁-C₅-alkyl steht,
- R²: für gegebenenfalls durch Halogen substituiertes C₁-C₁₆-Alkyl, C₂-C₁₆-Alkenyl, C₁-C₁₆-Alkoxy-C₂-C₆-alkyl, C₁-C₆-Polyalkoxy-C₂-C₆-alkyl steht,
für gegebenenfalls durch Halogen-, Nitro-, C₁-C₄-Alkyl-, C₁-C₃-Alkoxy-, C₁-C₃-Halogenalkyl-substituiertes Phenyl oder Benzyl steht,
- R³, R⁴ und R⁵: unabhängig voneinander für gegebenenfalls durch Halogen substituiertes C₁-C₆-Alkyl, C₁-C₆-Alkoxy, C₁-C₆-Alkylamino, Di-(C₁-C₆)-alkylamino, C₁-C₆-Alkylthio, C₃-C₄-Alkenylthio, C₂-C₄-Alkinylthio, C₃-C₆-Cycloalkylthio, für gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl, C₁-C₃-Halogenalkyl substituiertes Phenyl, Phenoxy oder Phenylthio stehen.
- G: steht ganz besonders bevorzugt für Wasserstoff (a) oder für die Gruppen 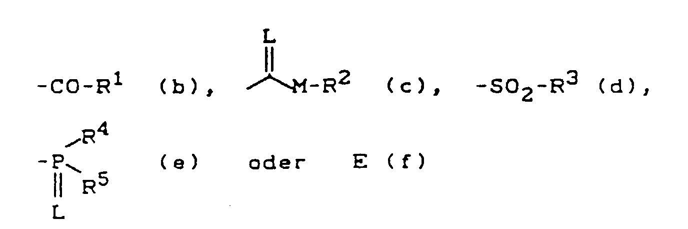
in welchen
- E: für ein Metallionäquivalent oder ein Ammoniumion steht,
- L und M: für Sauerstoff und/oder Schwefel steht,
- R¹: für gegebenenfalls durch Fluor oder Chlor substituiertes C₁-C₁₄-Alkyl, C₂-C₁₄-Alkenyl, C₁-C₄-Alkoxy-C₂-C₆-alkyl,C₁-C₄-Alkylthio-C₂-C₆-alkyl, C₁-C₄-Polyalkoxy-C₂-C₄-alkyl oder Cycloalkyl mit 3 bis 6 Ringatomen, das durch 1 bis 2 Sauerstoff- und/oder Schwefelatome unterbrochen sein kann, steht,
für gegebenenfalls durch Fluor, Chlor, Brom, Methyl, Ethyl, Propyl, i-Propyl, Methoxy, Ethoxy, Trifluormethyl, Trifluormethoxy, Nitrosubstituiertes Phenyl steht,
für gegebenenfalls durch Fluor, Chlor, Brom, Methyl, Ethyl, Propyl, i-Propyl, Methoxy, Ethoxy, Trifluormethyl, Trifluormethoxy-substituiertes Phenyl-C₁-C₃-alkyl steht,
für gegebenenfalls durch Fluor, Chlor, Brom, Methyl, Ethyl-substituiertes Pyridyl, Pyrimidyl, Thiazolyl und Pyrazolyl steht,
für gegebenenfalls durch Fluor, Chlor, Methyl, Ethyl-substituiertes Phenoxy-C₁-C₄-alkyl steht,
für gegebenenfalls durch Fluor, Chlor, Amino, Methyl-, Ethyl, substituiertes Pyridyloxy-C₁-C₄-alkyl, Pyrimidyloxy-C₁-C₄-alkyl und Thiazolyloxy-C₁-C₄-alkyl steht,
- R²: für gegebenenfalls durch Fluor oder Chlor substituiertes C₁-C₁₄-Alkyl, C₂-C₁₄-Alkenyl, C₁-C₄-Alkoxy-C₂-C₆-alkyl, C₁-C₄-Polyalkoxy-C₂-C₆-alkyl steht,
oder für gegebenenfalls durch Fluor, Chlor, Nitro, Methyl, Ethyl, Propyl, i-Propyl, Methoxy, Ethoxy, Trifluormethyl substituiertes Phenyl oder Benzyl steht,
- R³, R⁴ und R⁵: unabhängig voneinander für gegebenenfalls durch Fluor oder Chlor substituiertes C₁-C₄-Alkyl, C₁-C₄-Alkoxy, C₁-C₄-Alkylamino, Di-(C₁-C₄)-alkylamino, C₁-C₄-Alkylthio, für gegebenenfalls durch Fluor, Chlor, Brom, Nitro, Cyano, C₁-C₂-Alkoxy, C₁-C₄-Fluoralkoxy, C₁-C₂-Chloralkoxy, C₁-C₂-Alkylthio, C₁-C₂-Fluoralkylthio, C₁-C₂-Chloralkylthio, C₁-C₃-Alkyl substituiertes Phenyl, Phenoxy oder Phenylthio stehen.

Besonders bevorzugt sind bicyclische 3-Aryl-pyrrolidin-2,4-dione der Formel (I), in welchern für eine Zahl 0, 1, 2 oder 3 steht und die Reste A, Q, B, G, X, Y und Z jeweils die oben angegebenen besonders bevorzugten Bedeutungen haben.

Ganz besonders bevorzugt sind bicyclische 3-Aryl-pyrrolidin-2,4-dione der Formel (I), in welcher n für eine Zahl 0, 1, 2 oder 3 steht und die Reste A, Q, B, G, X, Y und Z jeweils die oben angegebenen ganz besonders bevorzugten Bedeutungen besitzen.

Im einzelnen seien außer den bei den Herstellungsbeispielen genannten Verbindungen die folgenden substituierten bicyclischen 3-Aryl-pyrrolidin-2,4-dion-Derivate der allgemeinen Formel (I) genannt:

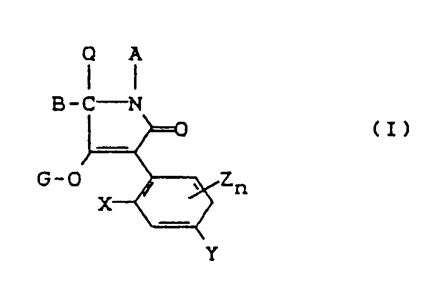

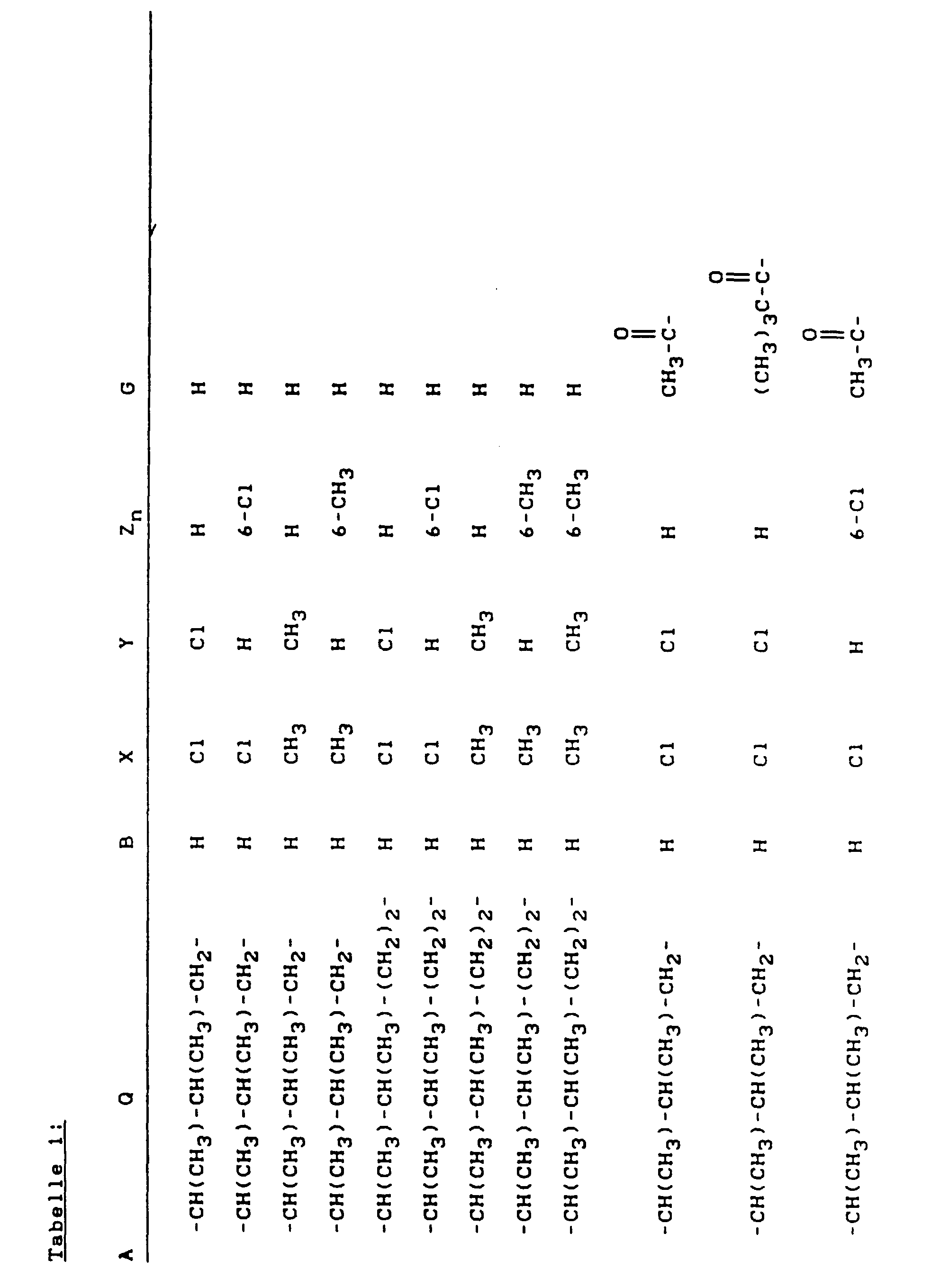

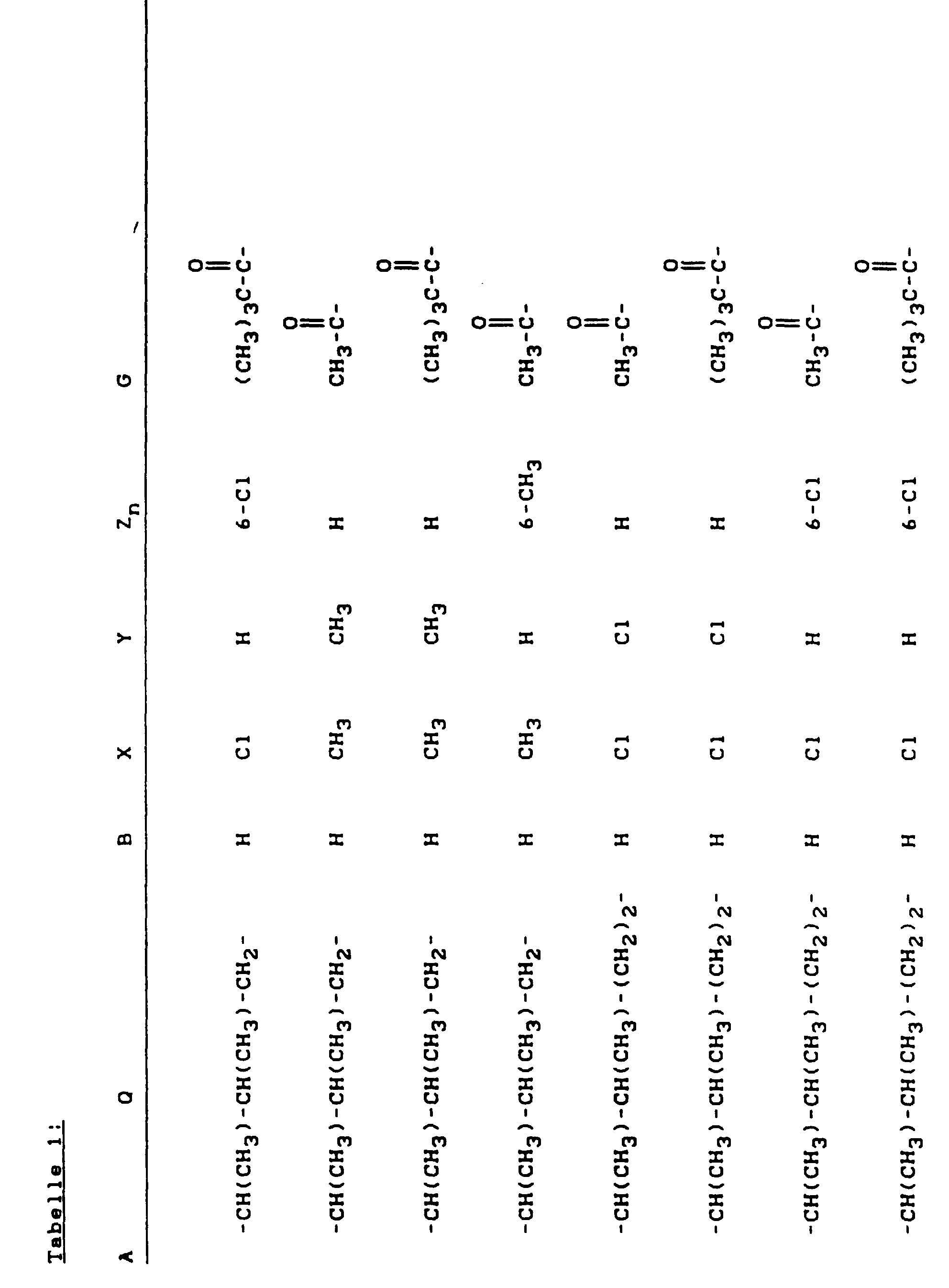

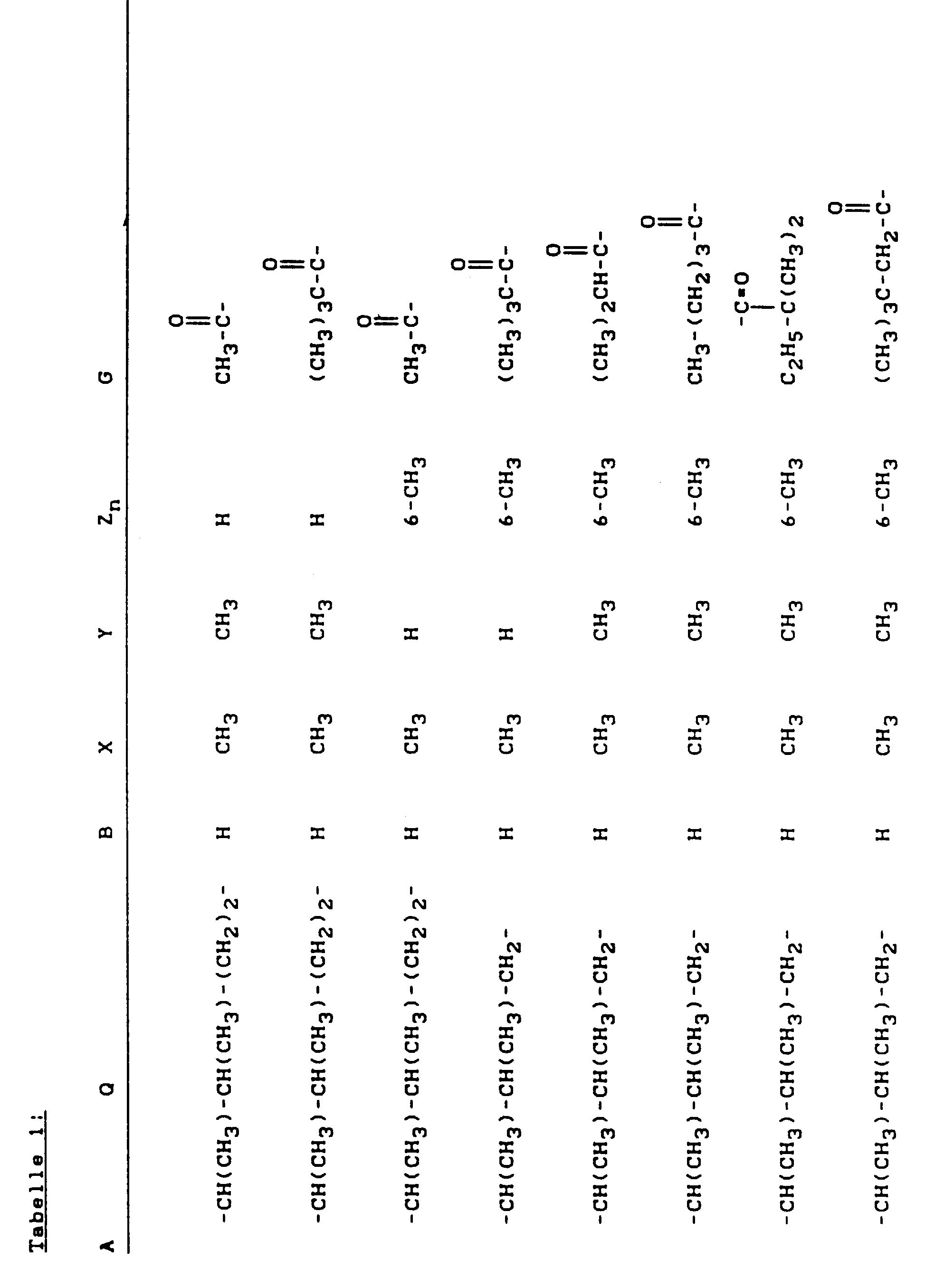

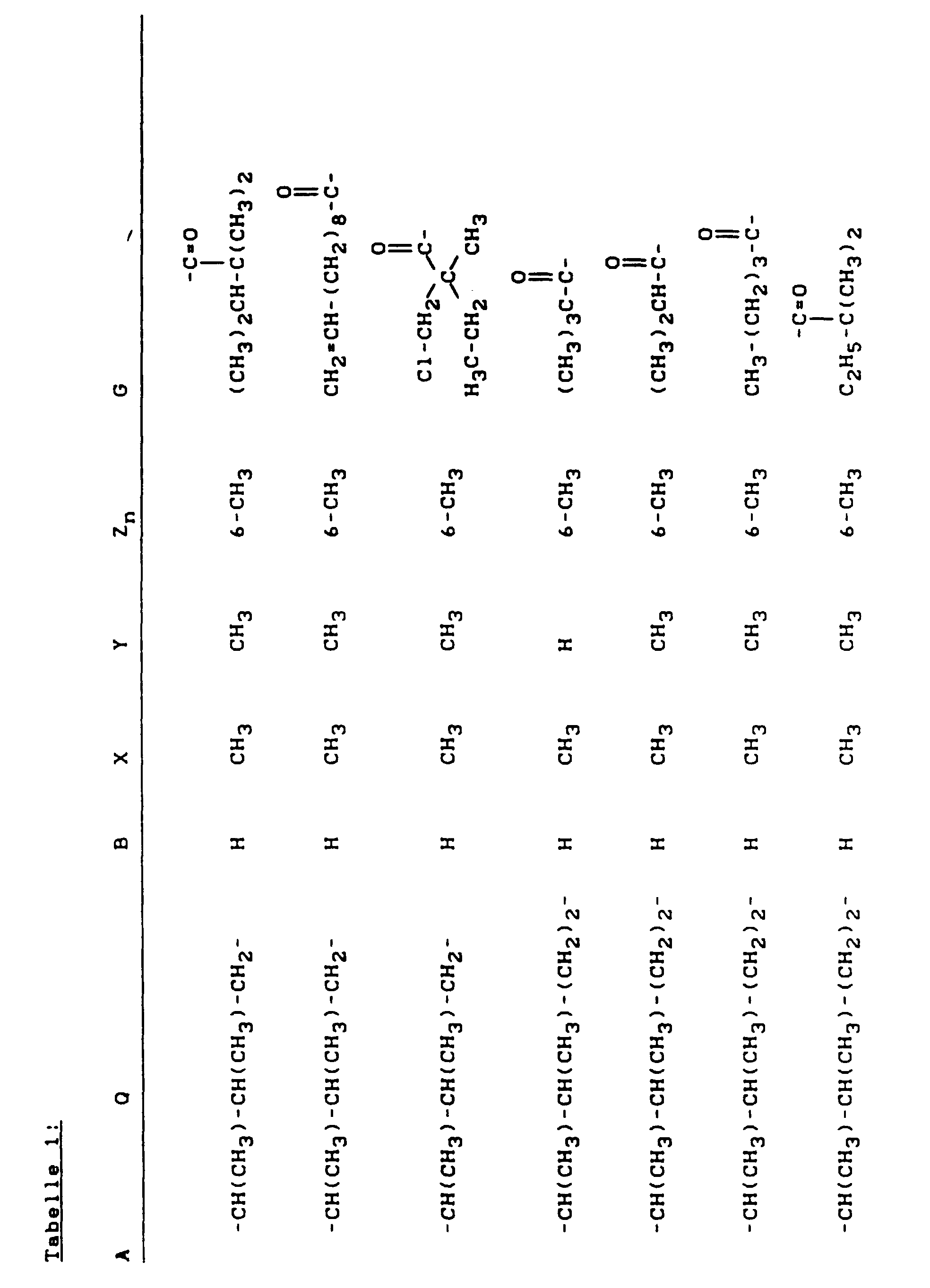

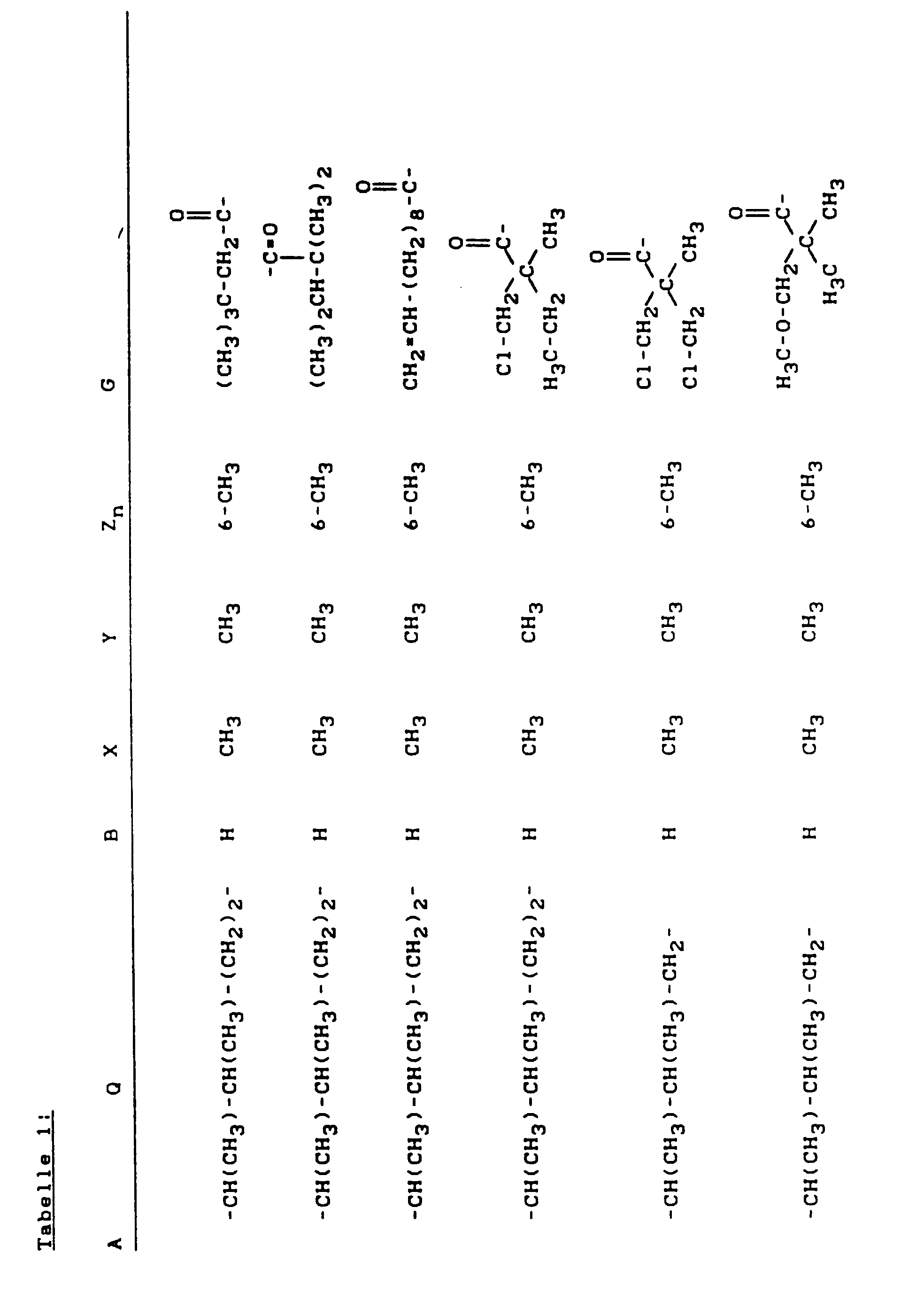

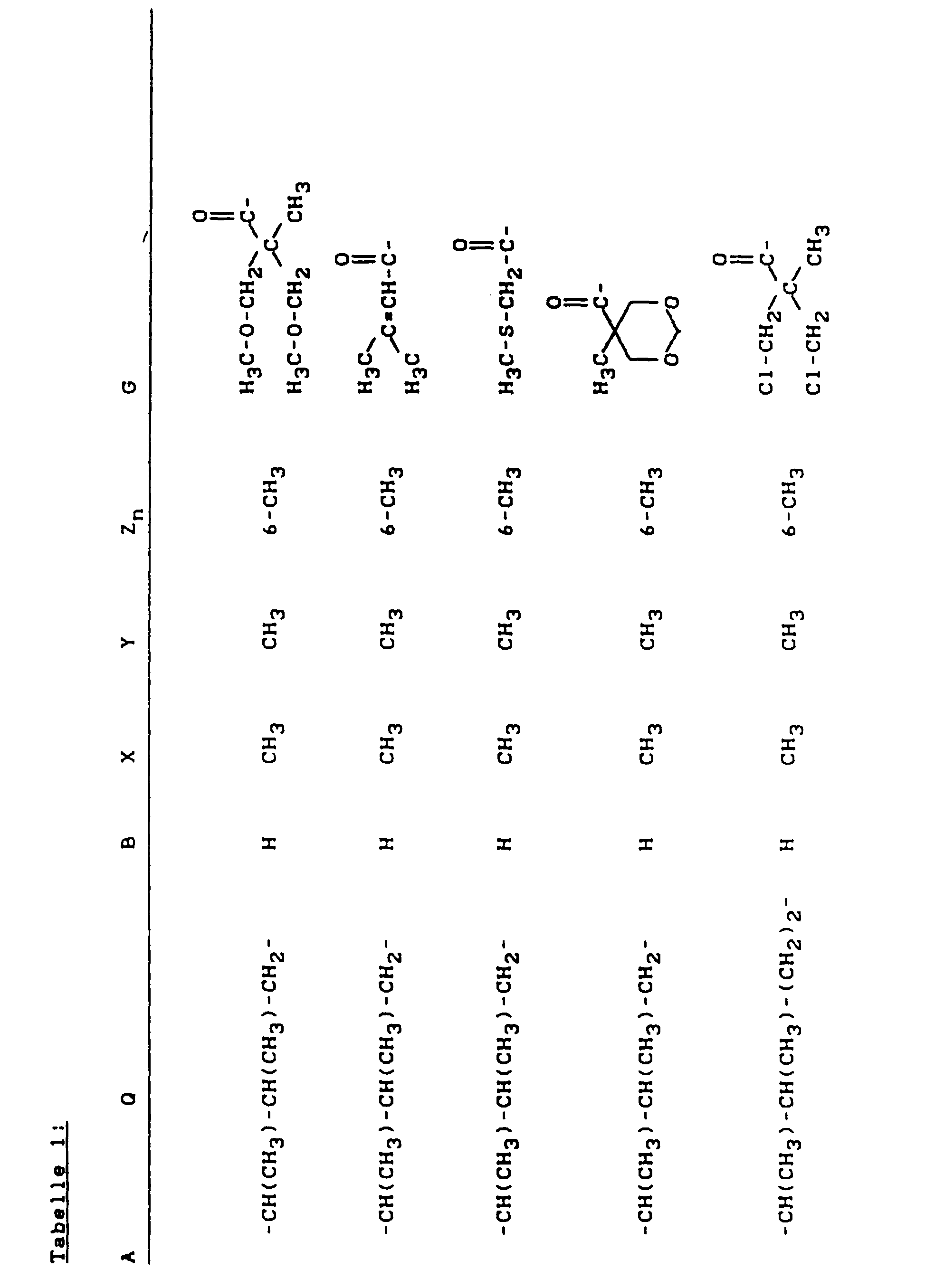

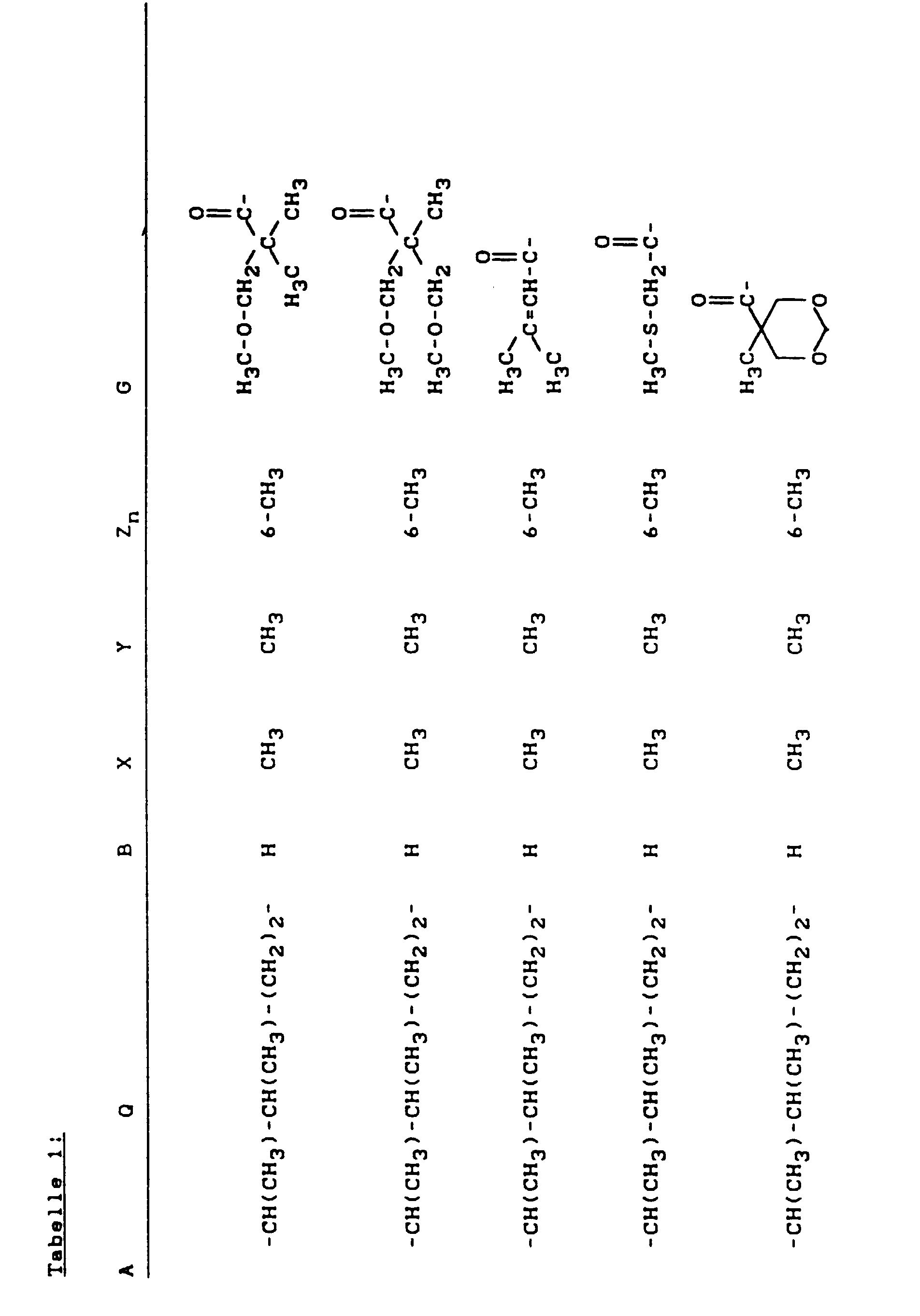

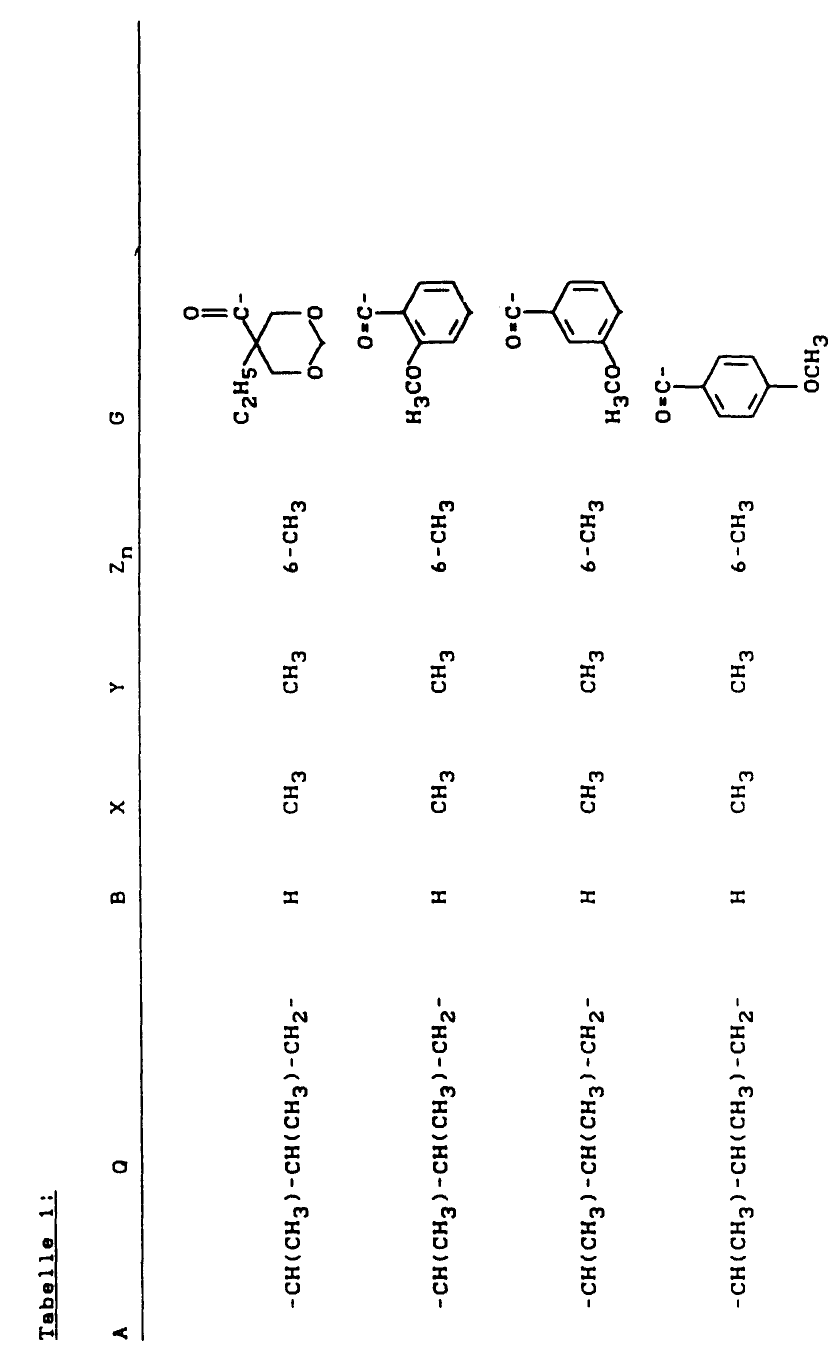

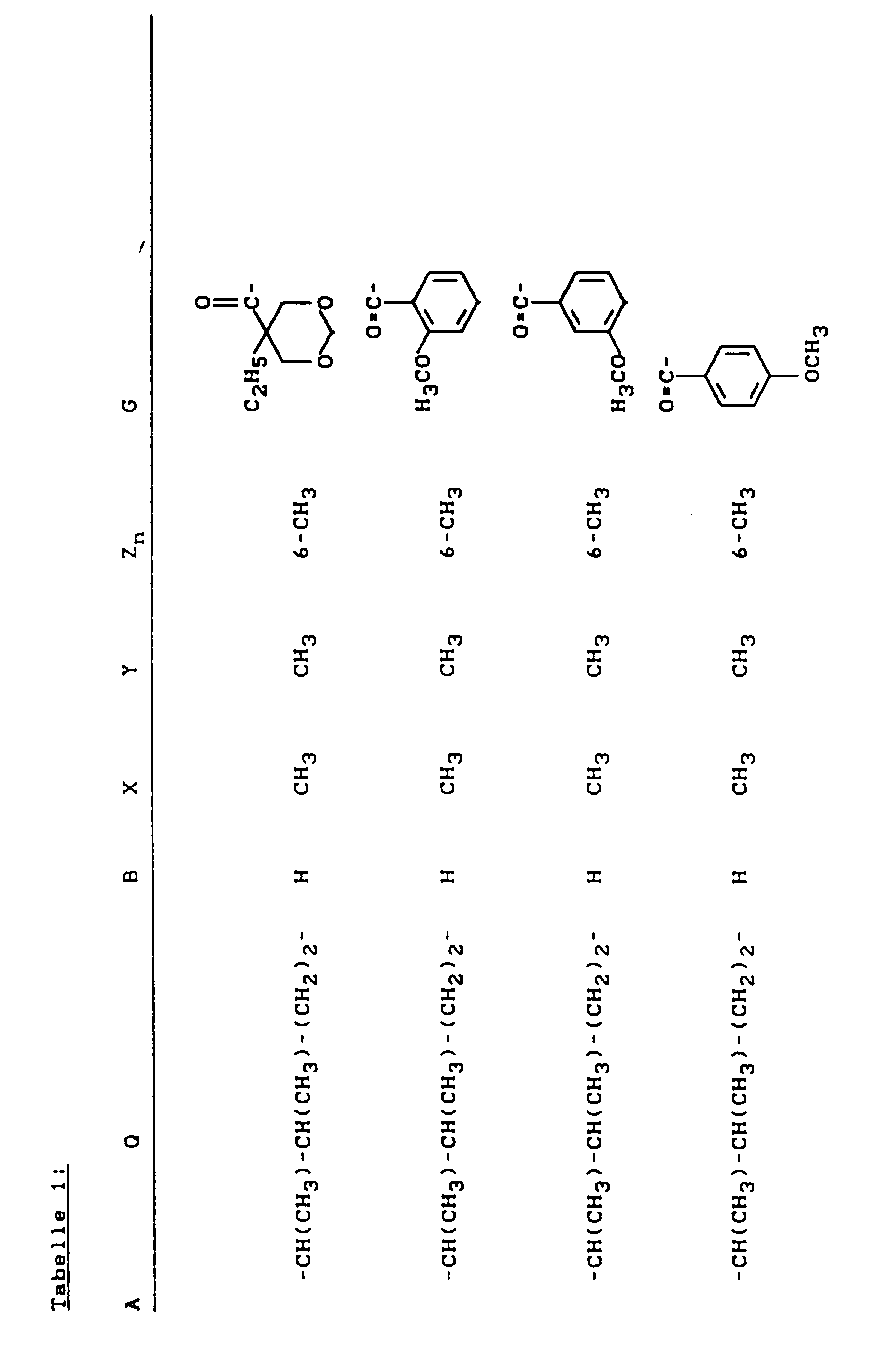

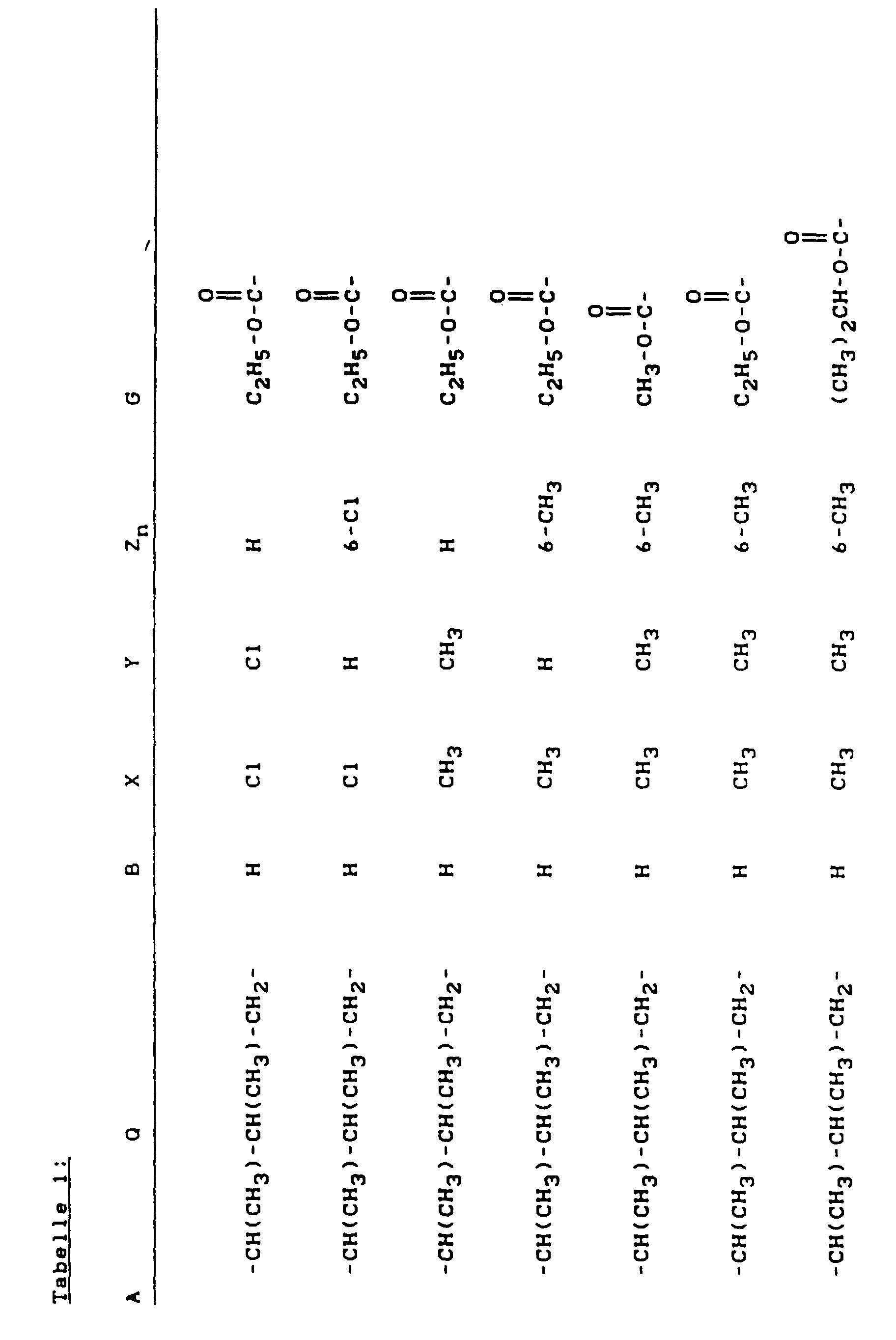

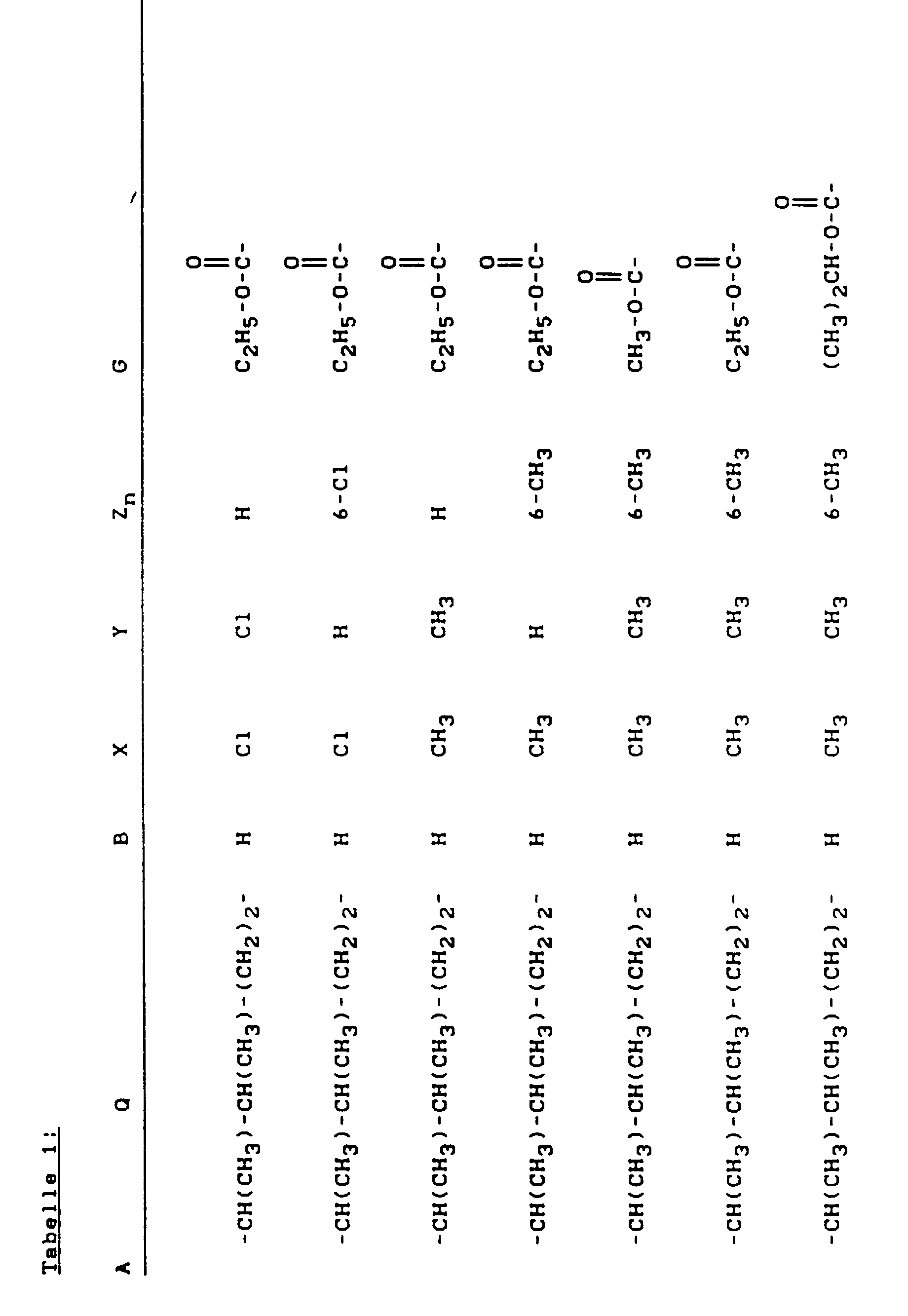

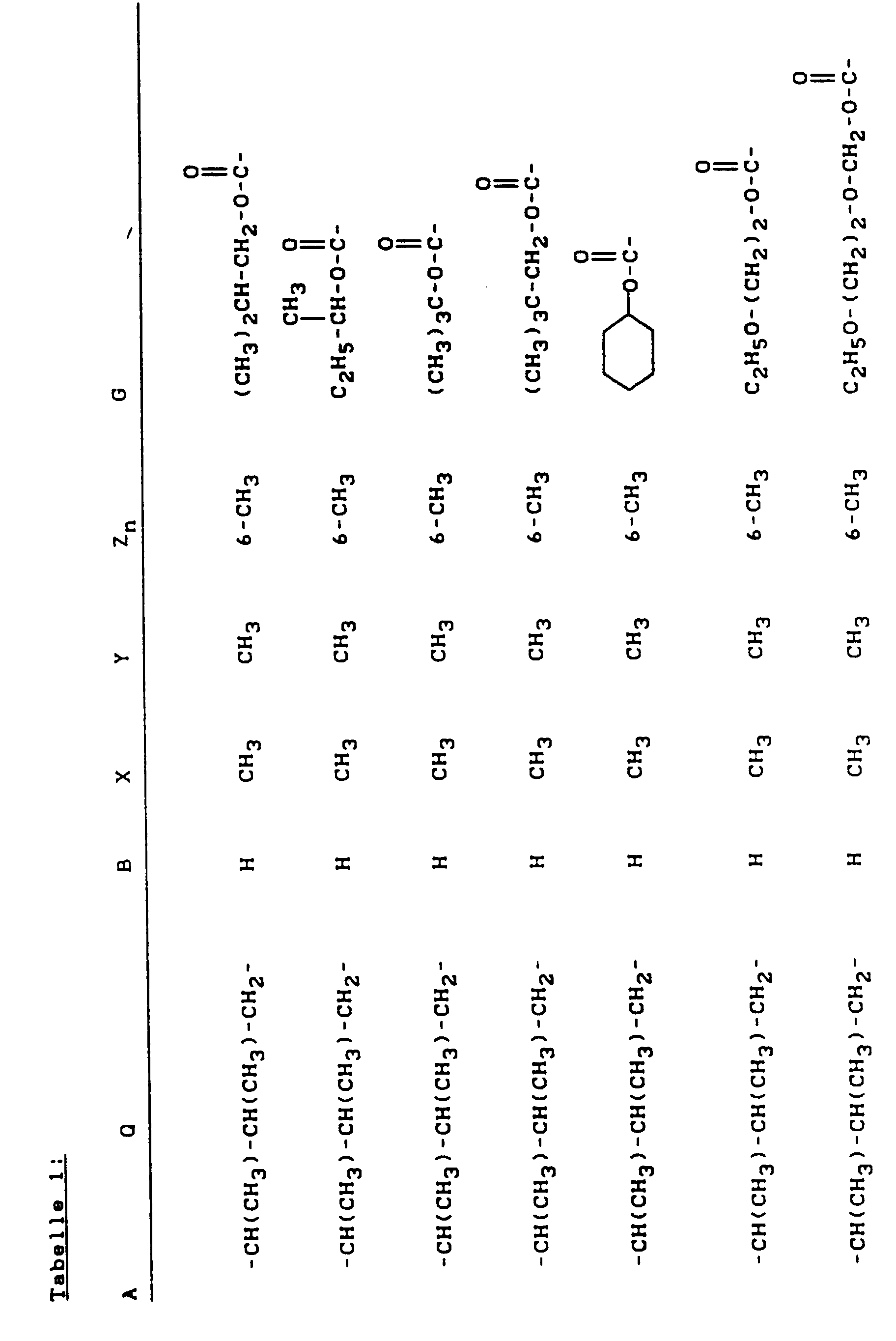

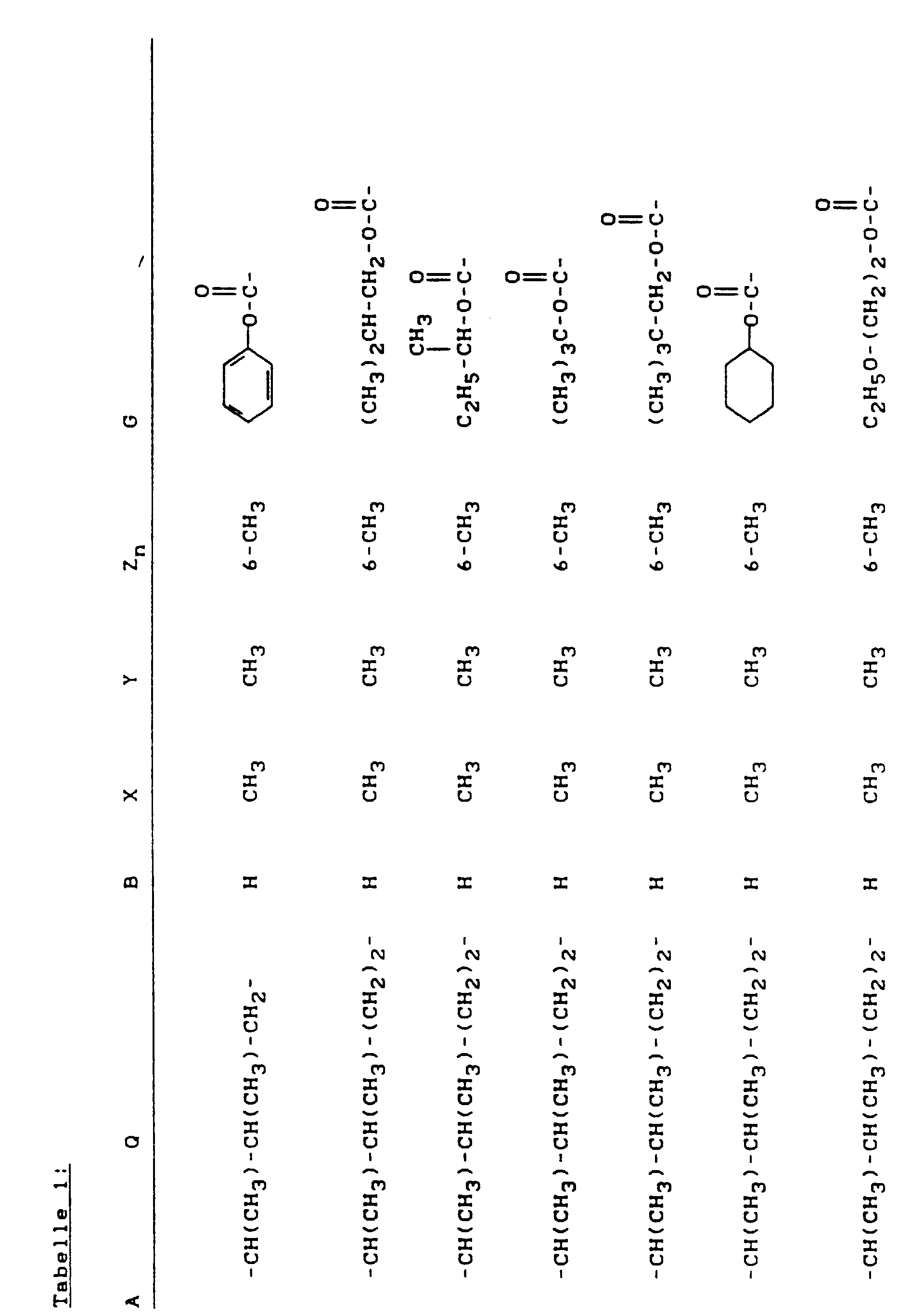

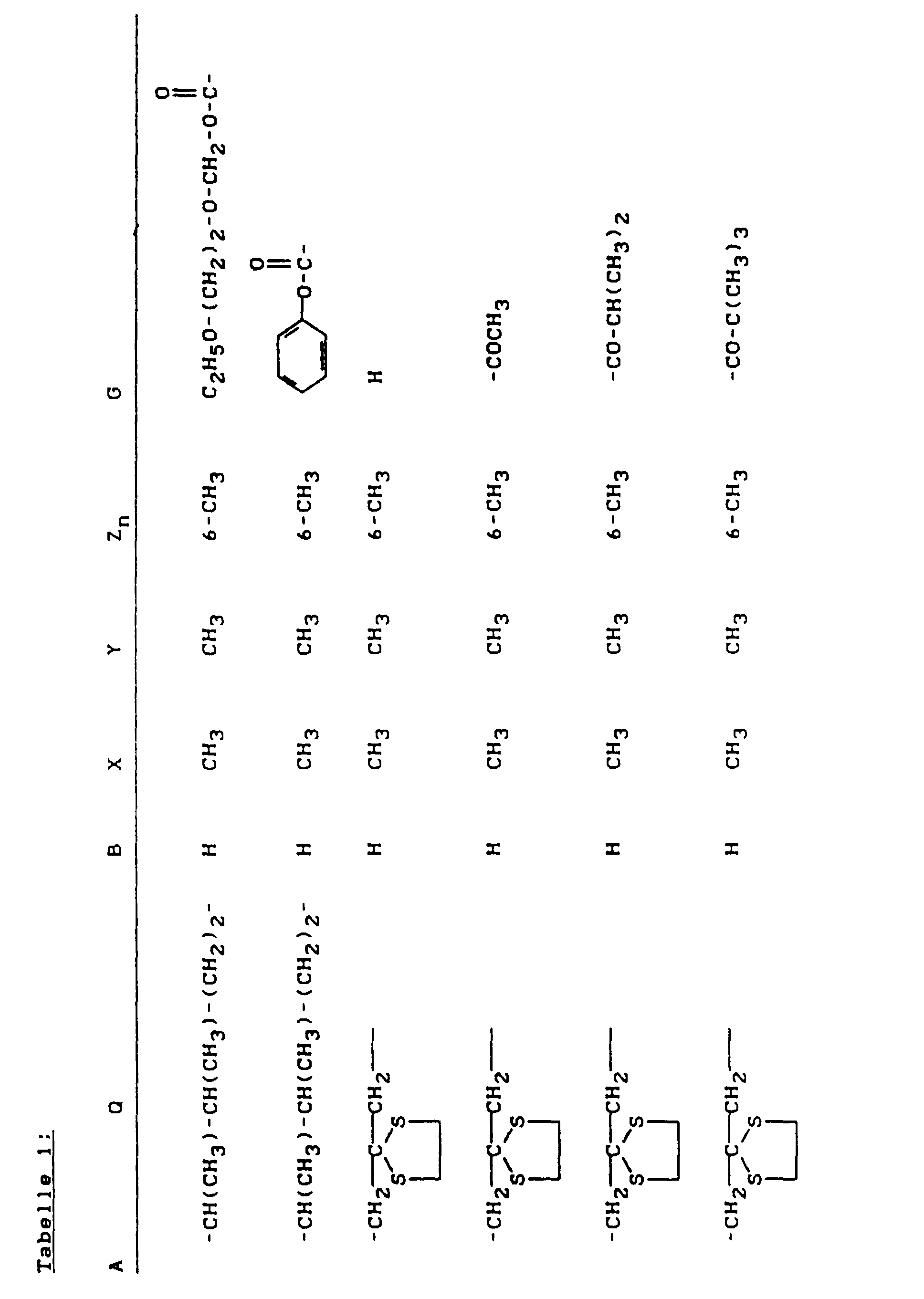

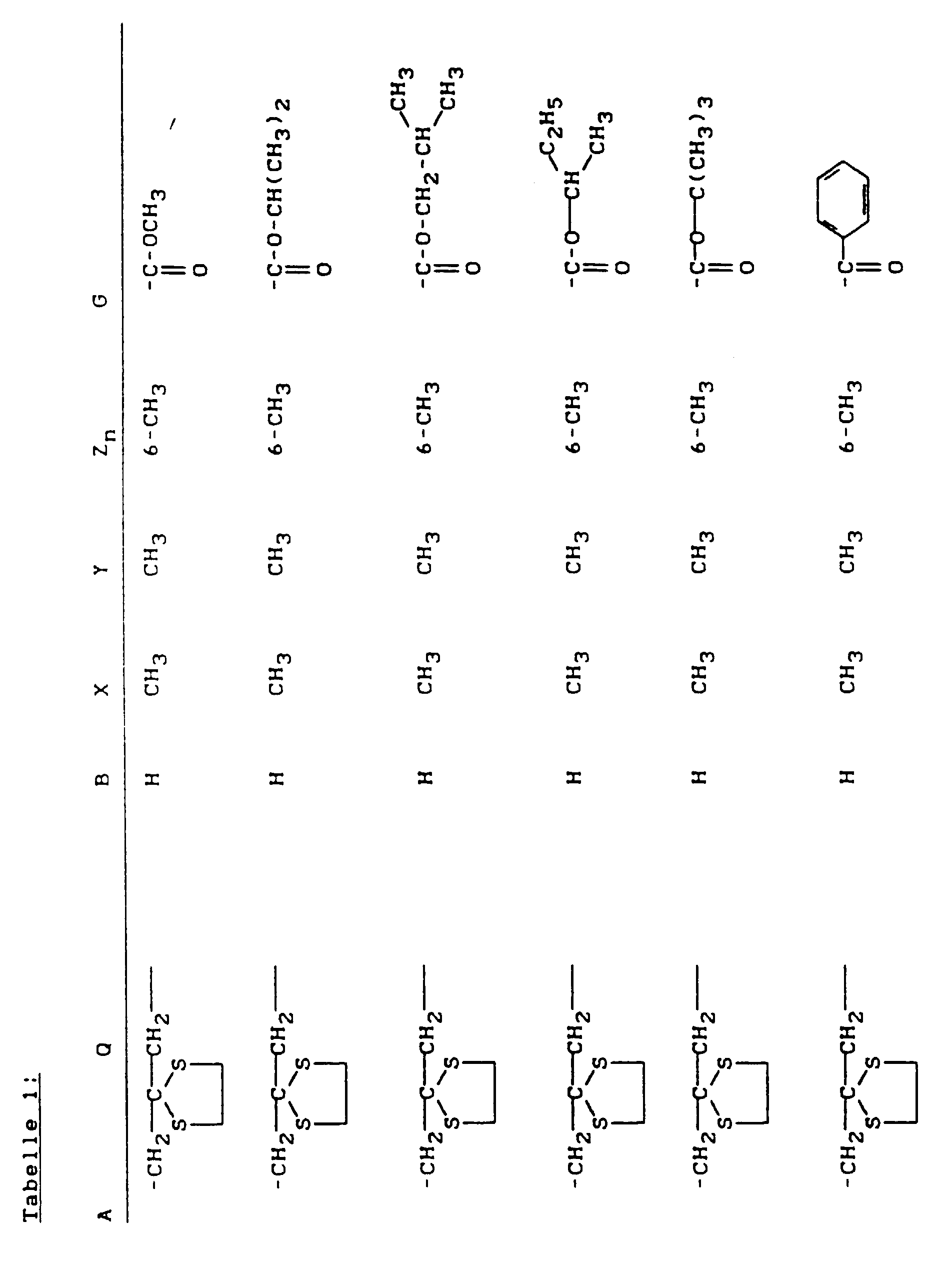

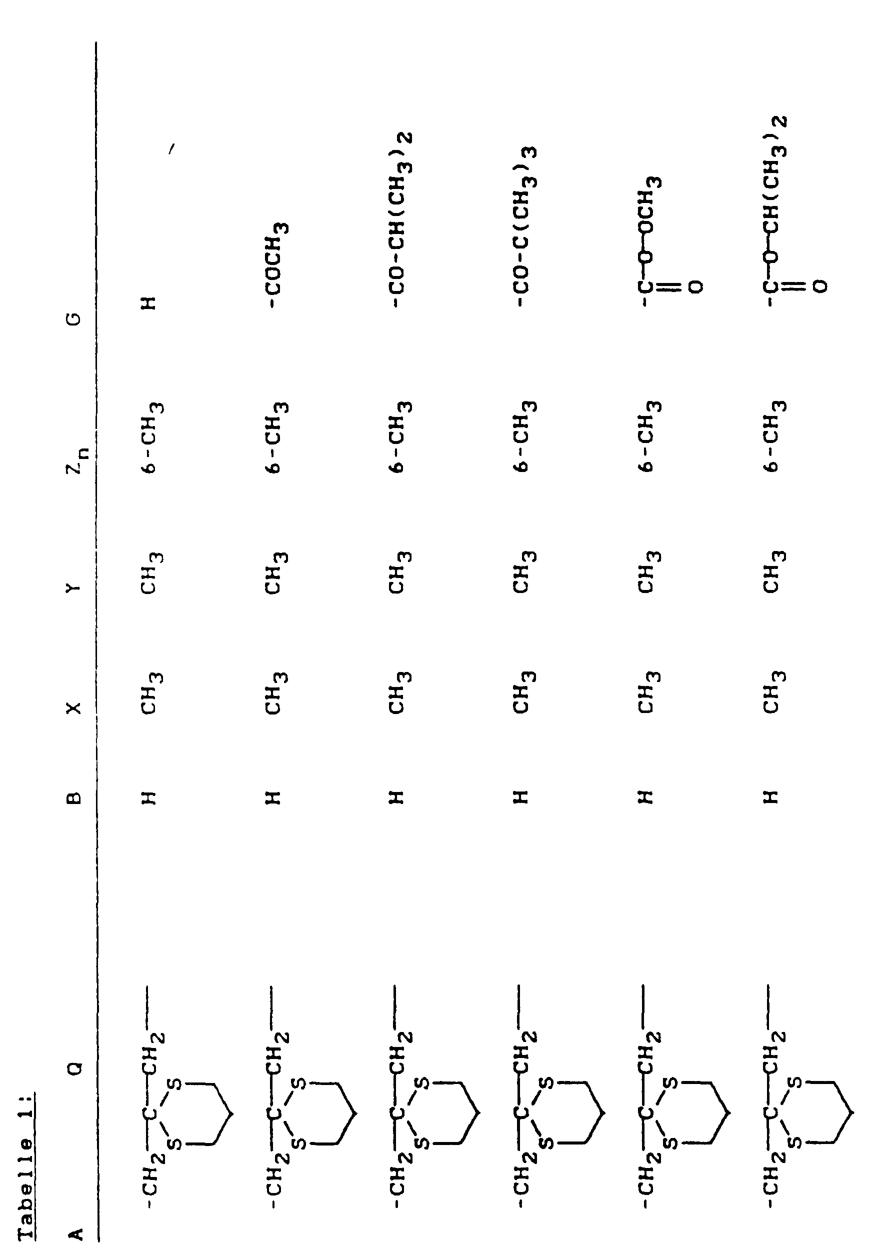

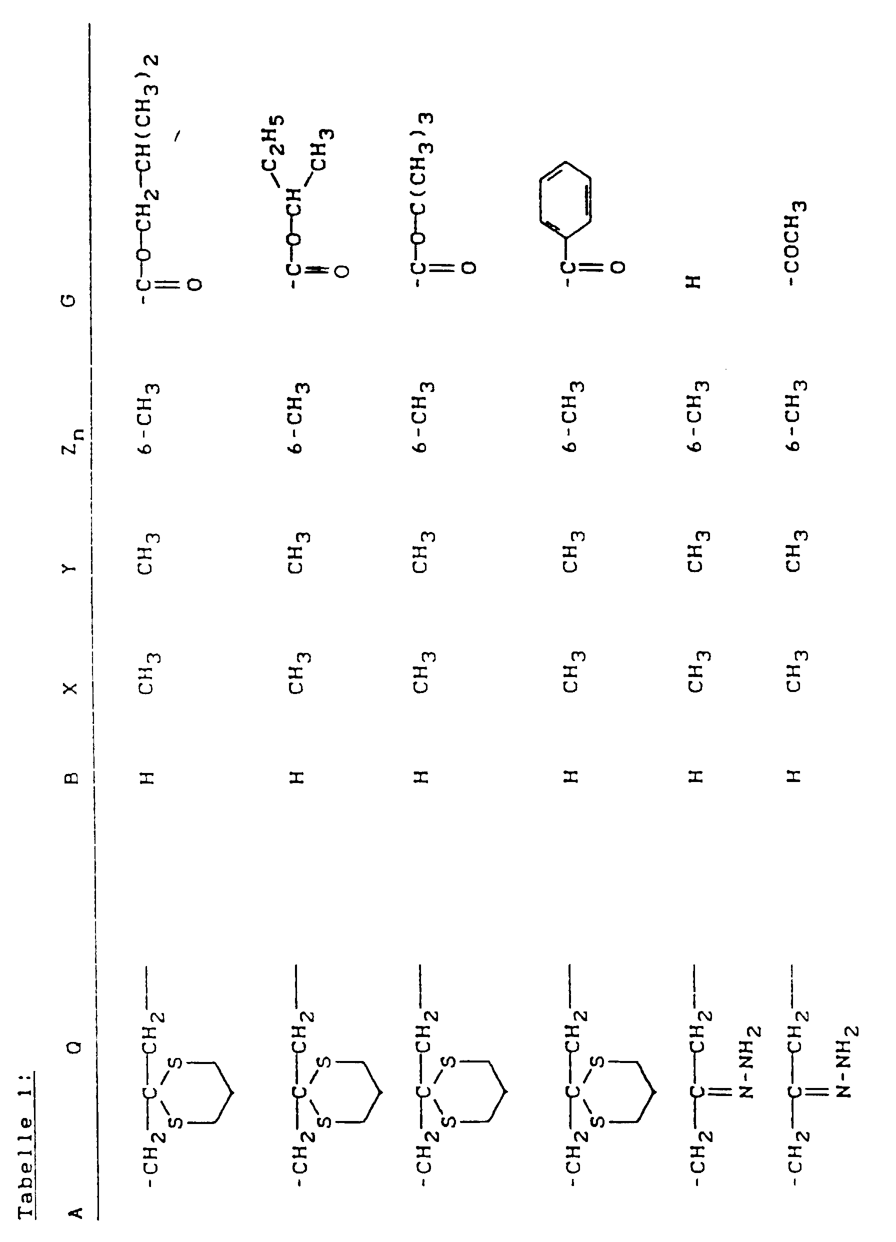

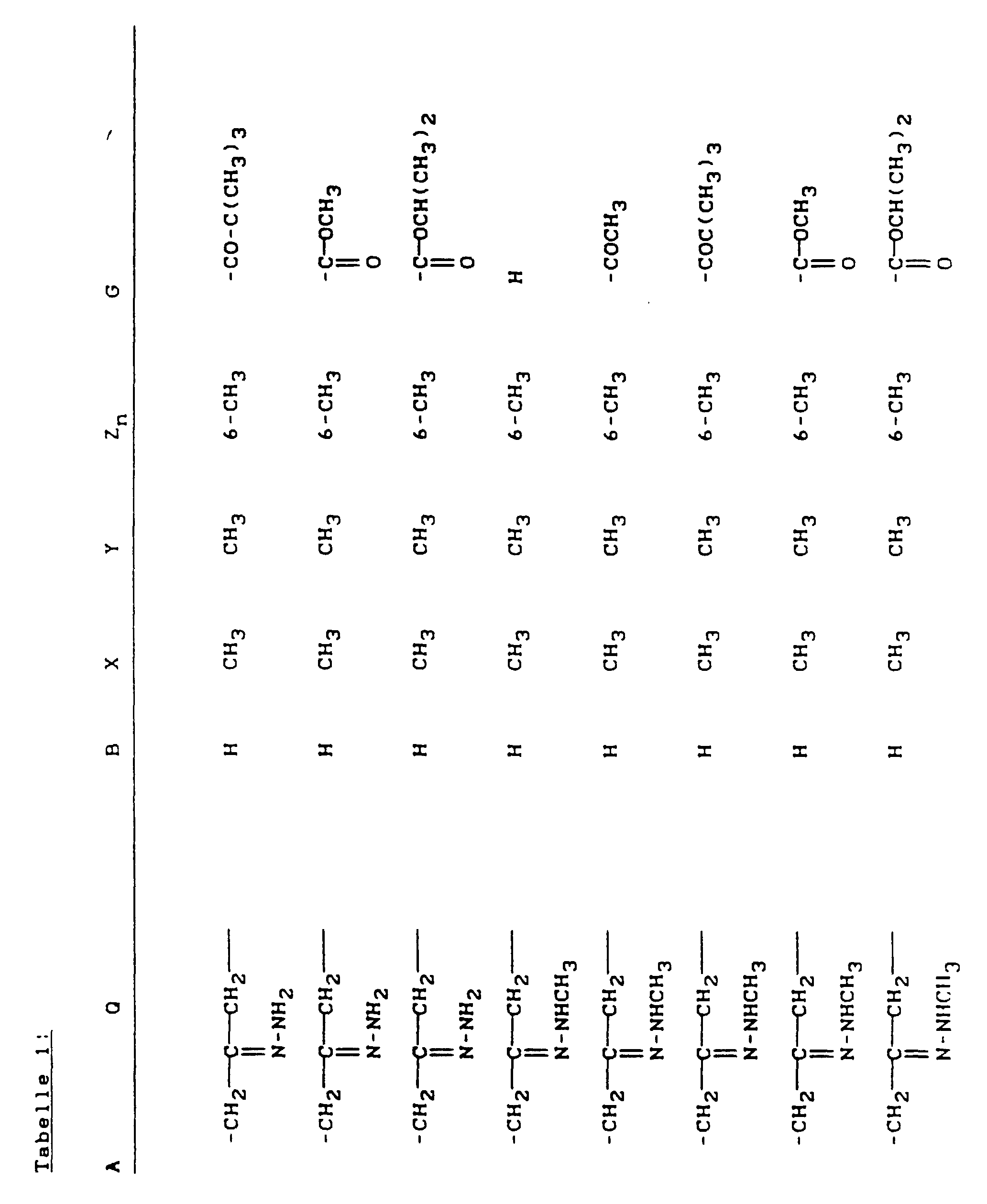

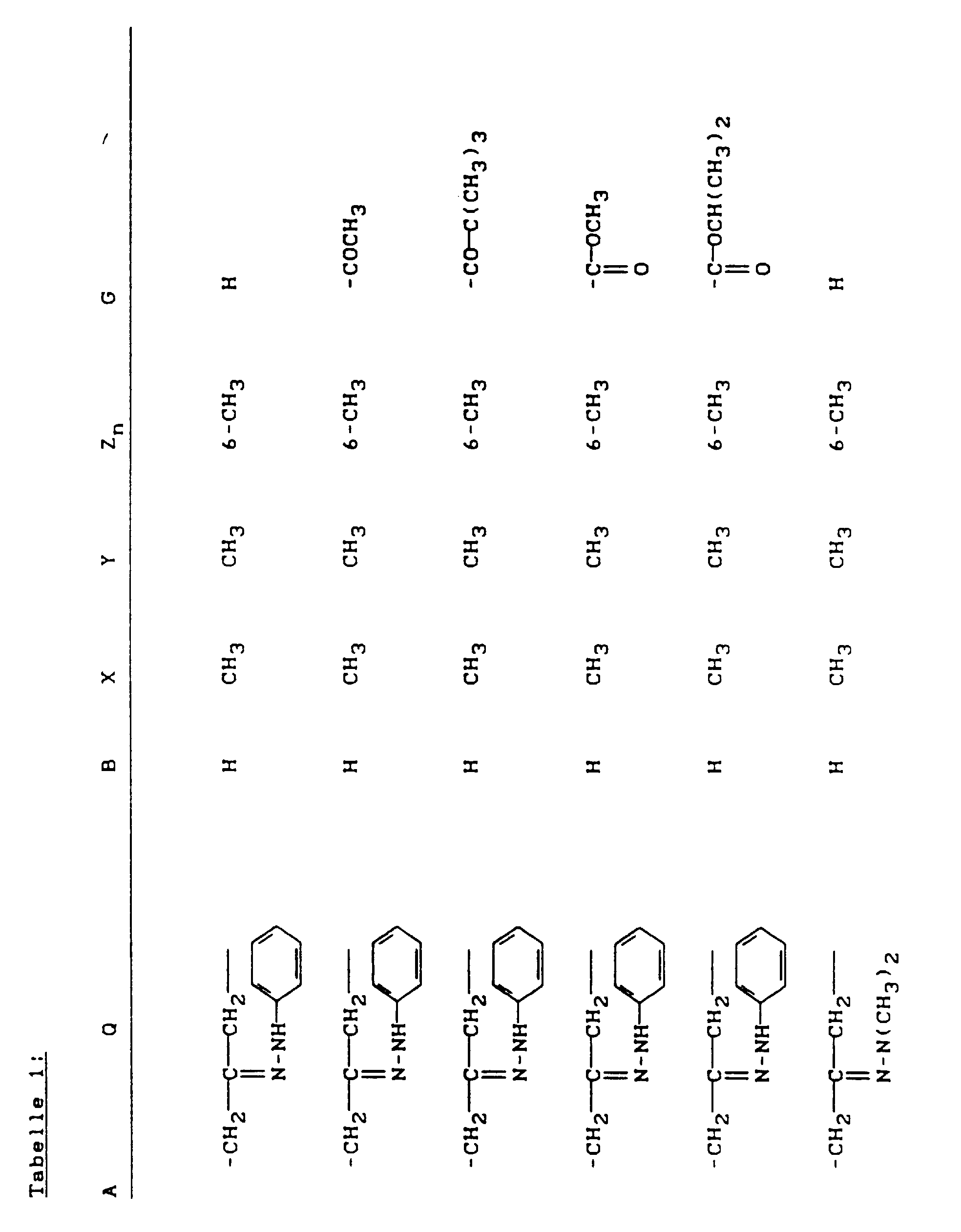

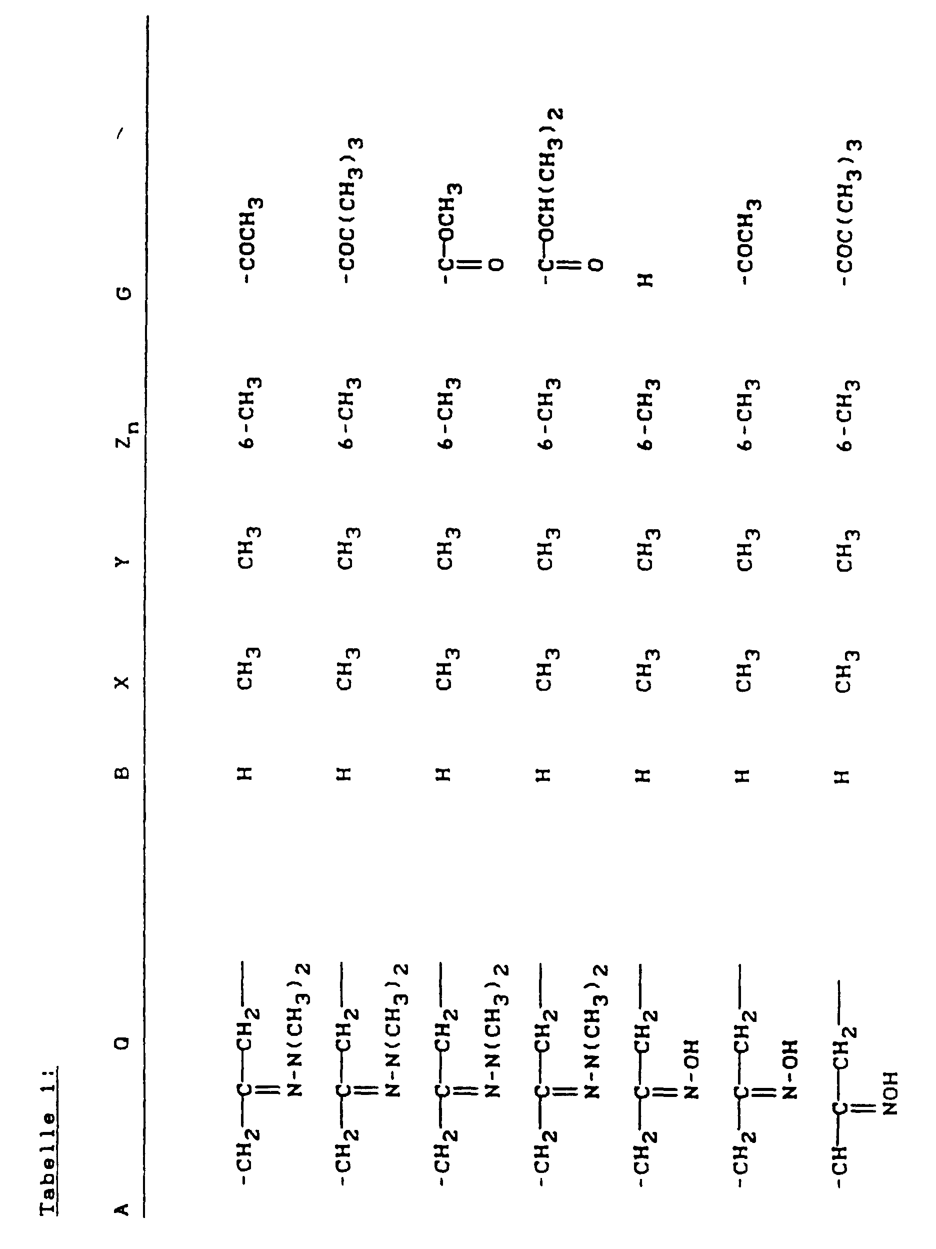

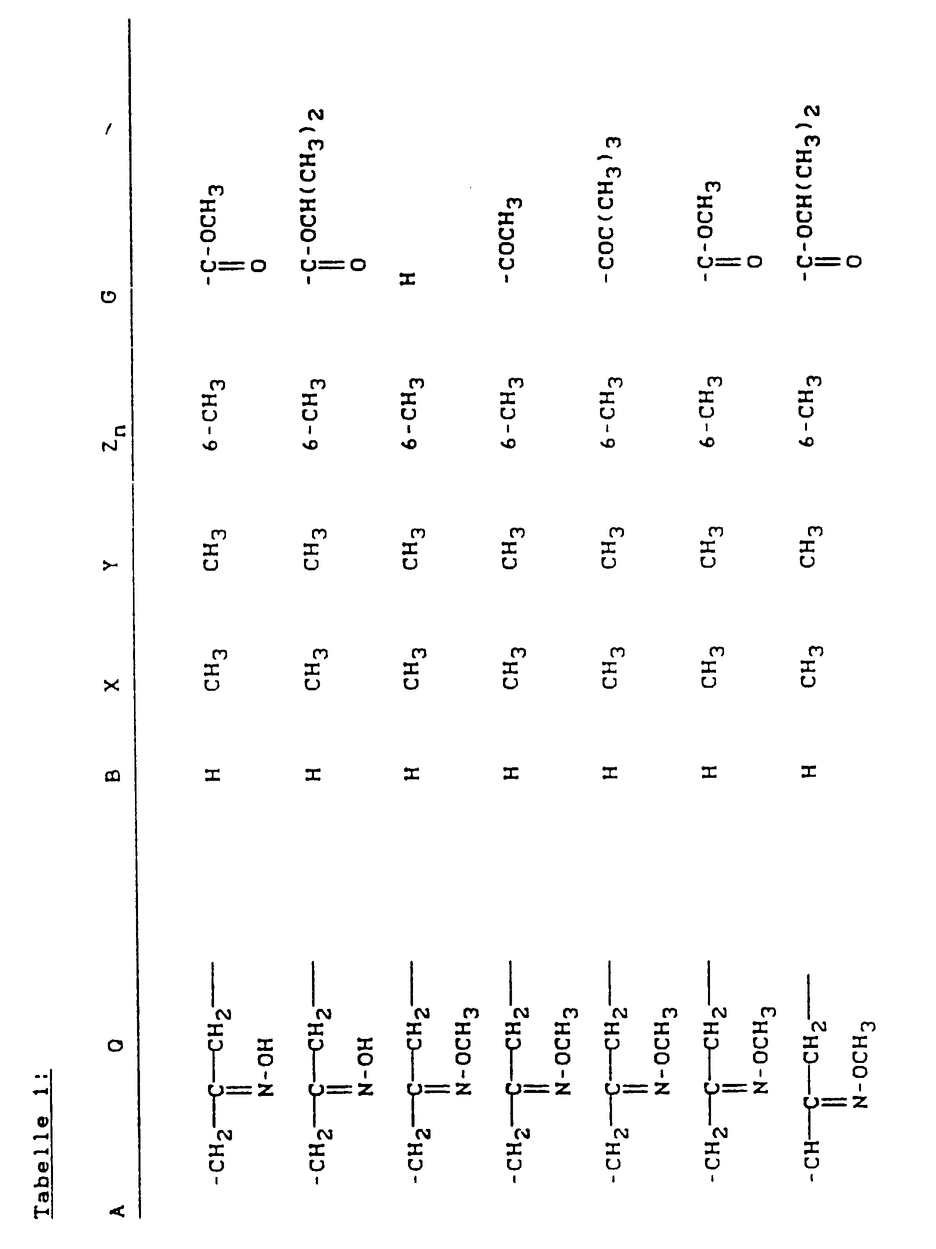

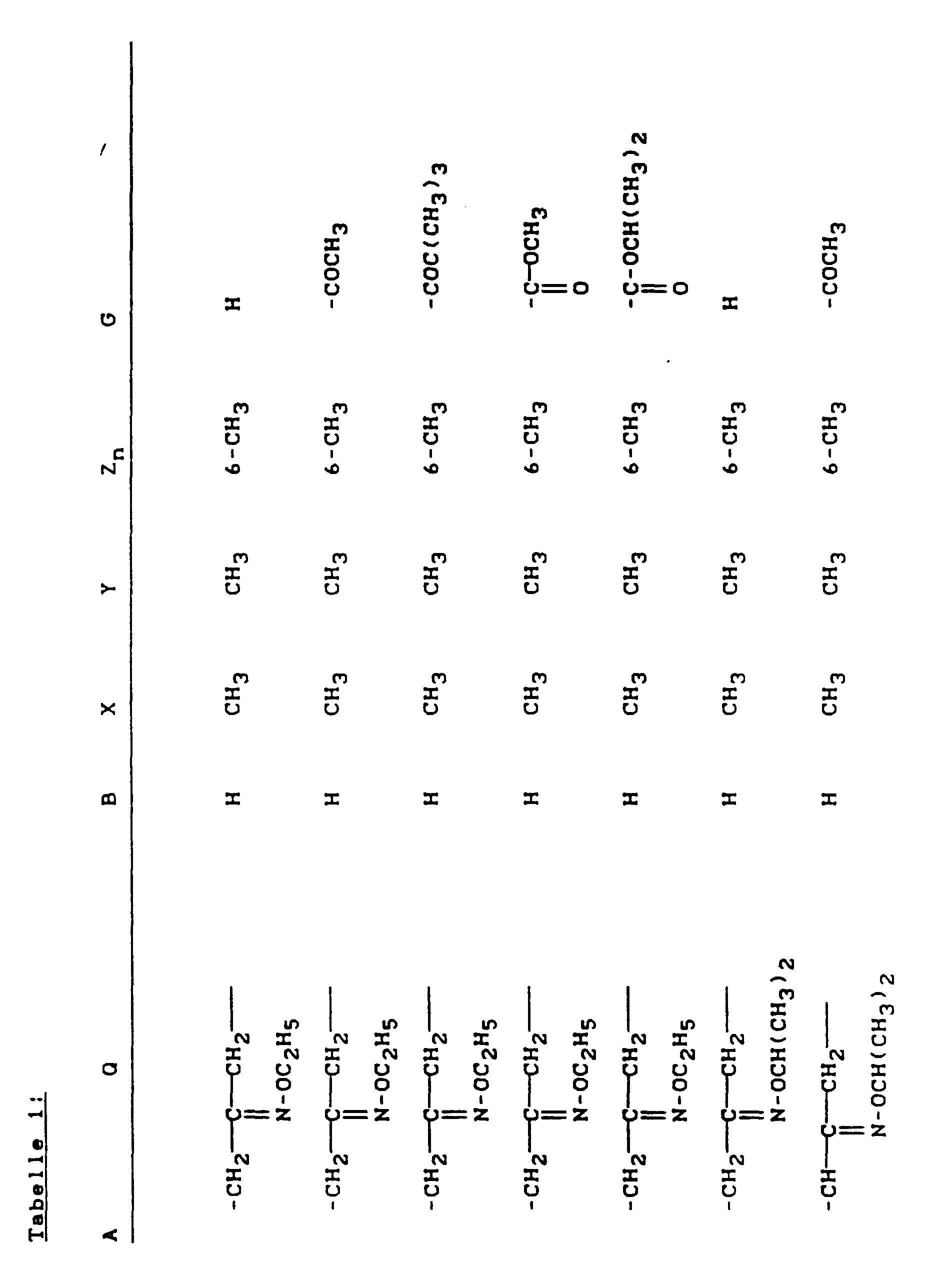

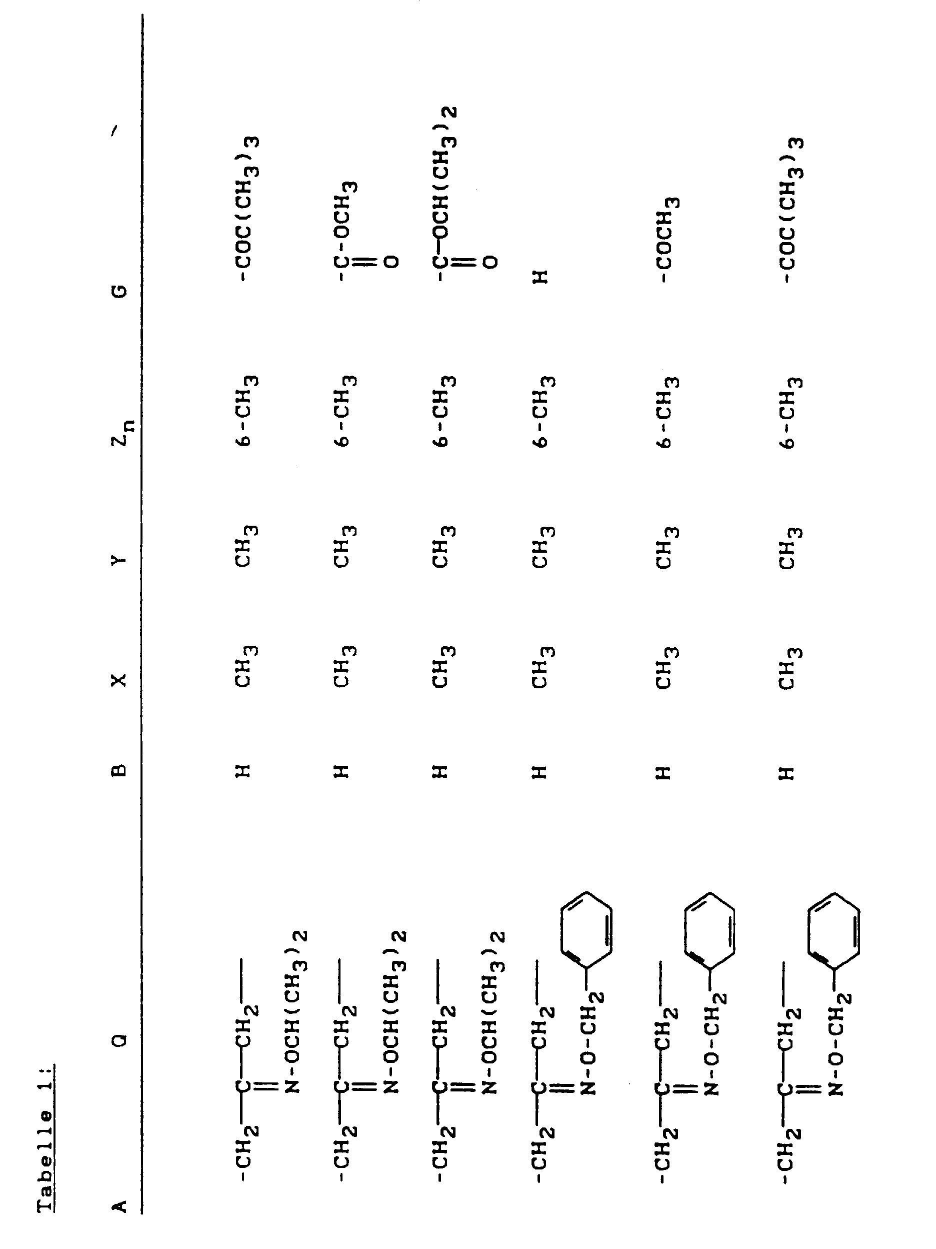

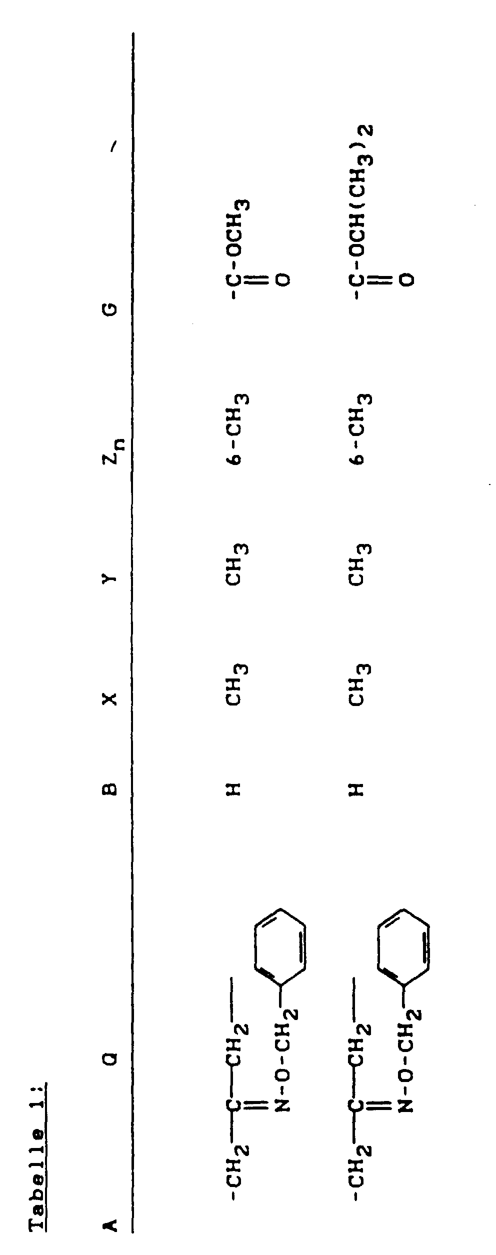

Verwendet man gemäß Verfahren (Aα) N-(2,6-Dichlorphenylacetyl)-6-methyl-piperidin-2-carbonsäureethylester, so kann der Verlauf des erfindungsgemäßen Verfahrens durch folgendes Reaktionsschema wiedergegeben werden:

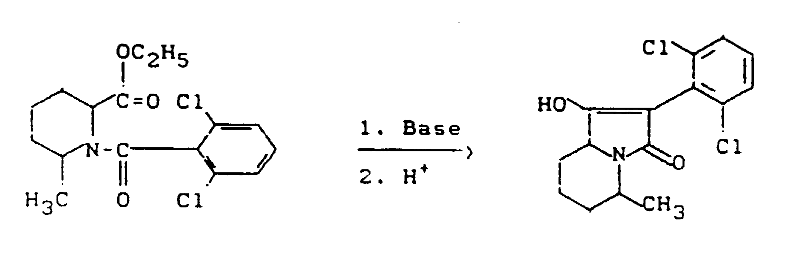

Verwendet man gemäß Verfahren (A-β) 3-(2,4,6-Trimethylphenyl)-1-aza-7-hydroxy-bicyclo-(3,3,0^{1,5})-octan-2,4-dion, Oxalylchlorid und eine Mischung von Dimethylsulfoxid/Dichlormethan, so kann der Verlauf des erfindungsgemäßen Verfahrens durch das folgende Reaktionsschema wiedergegeben werden:

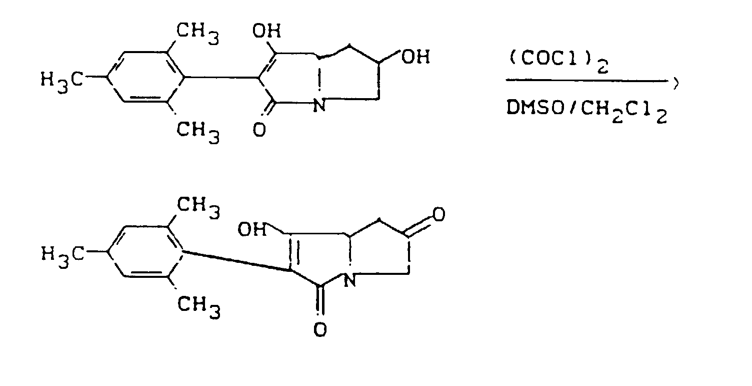

Verwendet man gemäß Verfahren (A-β) 3-(2,4,6-Trimethylphenyl)-1-aza-7-hydroxy-bicyclo-(3,30^{1,5})-octan-2,4-dion und Bromwasserstoff in Eisessig, so kann der Verlauf des erfindungsgemäßen Verfahrens durch das folgende Reaktionsschema wiedergegeben werden:

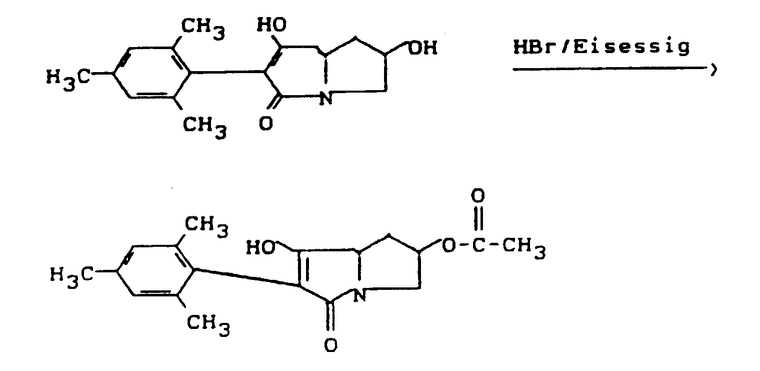

Verwendet man gemäß Verfahren (B) (Variante α) 3-(2,4,6-Trimethylphenyl)-1-aza-7,8-dimethyl-bicyclo-(3,3,0^{1,5})-oktan-2,4-dion und Pivaloylchlorid als Ausgangsstoff, so kann der Verlauf des erfindungsgemäßen Verfahrens durch folgendes Reaktionsschema wiedergegeben werden.

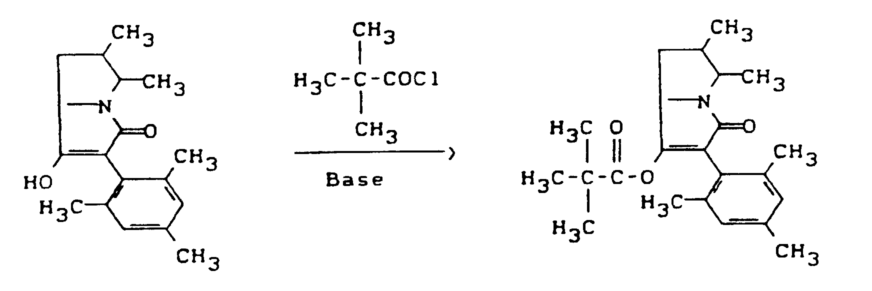

Verwendet man gemäß Verfahren B (Variante β) 8-(2,4,5-Trimethylphenyl)-1-aza-3-methyl-bicyclo-(4,3,0^{1,6})-no nan-7,9-dion und Acetanhydrid als Ausgangsverbindungen, so kann der Verlauf des erfindungsgemäßen Verfahrens durch folgendes Reaktionsschema wiedergegeben werden.

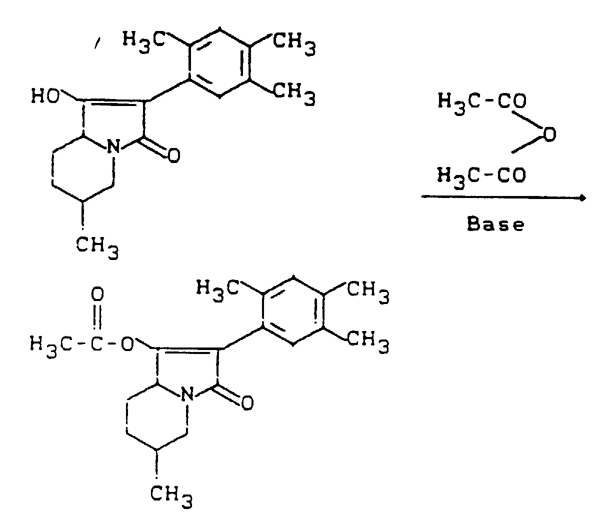

Verwendet man gemäß Verfahren C 8-(2,4-Dichlorphenyl)-1-aza-2-methyl-bicyclo-(4,3,0^{1,6})-nonan-7,9-dion und Chlorameisensäureethoxyethylester als Ausgangsverbindungen, so kann der Verlauf des erfindungsgemäßen Verfahrens durch folgendes Reaktionsschema wiedergegeben werden.

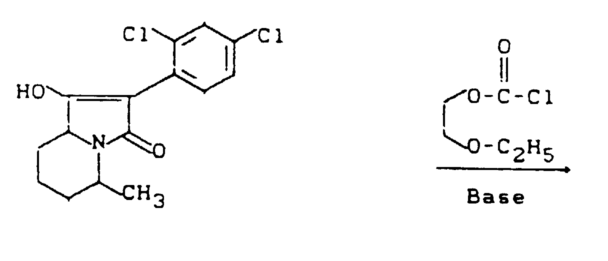

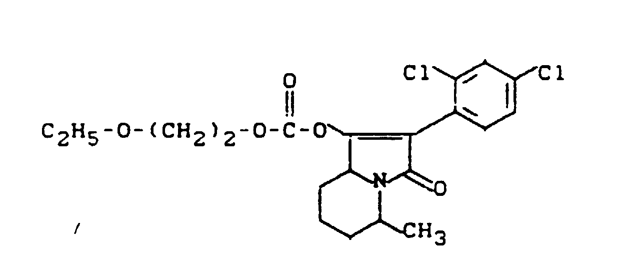

Verwendet man gemäß Verfahren (D_{α}) 3-(2,4,6-Trimethylphenyl)-1-aza-7-methoxy-bicyclo-(3,3,0^{1,5})-octan-2,4-dion und Chlormonothioameisensäuremethylester als Ausgangsprodukte, so kann der Reaktionsverlauf wie folgt wiedergegeben werden:

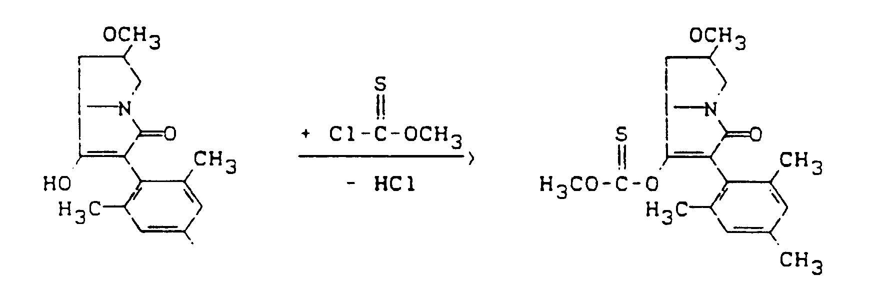

Verwendet man gemäß Verfahren (D_{β}) 3-(2,4,6-Trimethylphenyl)-1-aza-7,8-dimethyl-bicyclo-(3,3-0^{1,5})-octan-2,4-dion, Schwefelkohlenstoff und Methyliodid als Ausgangskomponenten; so kann der Reaktionsverlauf wie folgt wiedergegeben werden:

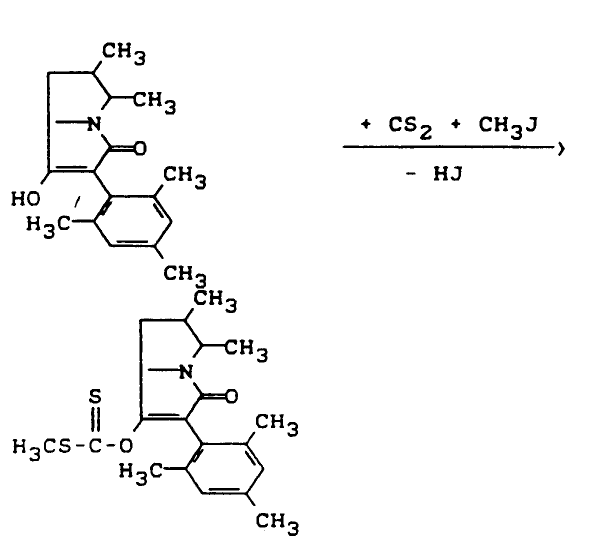

Verwendet man gemäß Verfahren (E) 3-(2,4,6-Trimethylphenyl)-1-aza-7-isopropyl-bicyclo-(3,3,0^{1,5})-nonan-2,4-dion und Methansulfonsäurechlorid als Ausgangsprodukt, so kann der Reaktionsverlauf durch folgendes Reaktionsschema wiedergegeben werden:

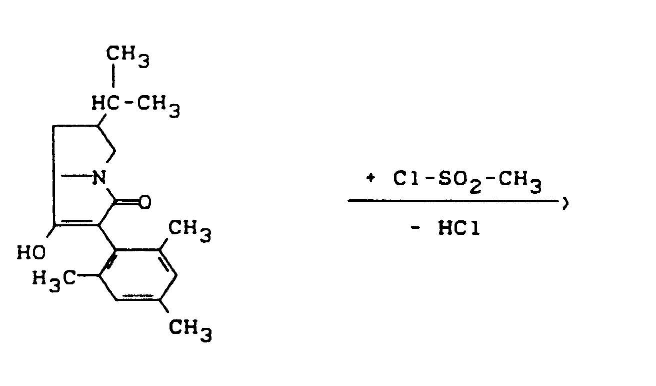

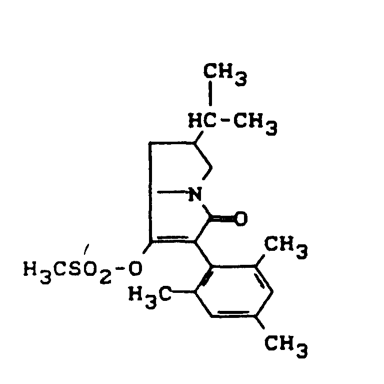

Verwendet man gemäß Verfahren (F) 3-(2,4,6-Trimethylphenyl)-1-aza-6,8-dimethyl-bicyclo-(3,3,0^{1,5})-octan-2,4-dion und Methanthio-phosphonsäurechlorid-(2,2,2-trifluorethylester) als Ausgangsprodukte, so kann der Reaktionsverlauf durch folgendes Reaktionsschema wiedergegeben werden:

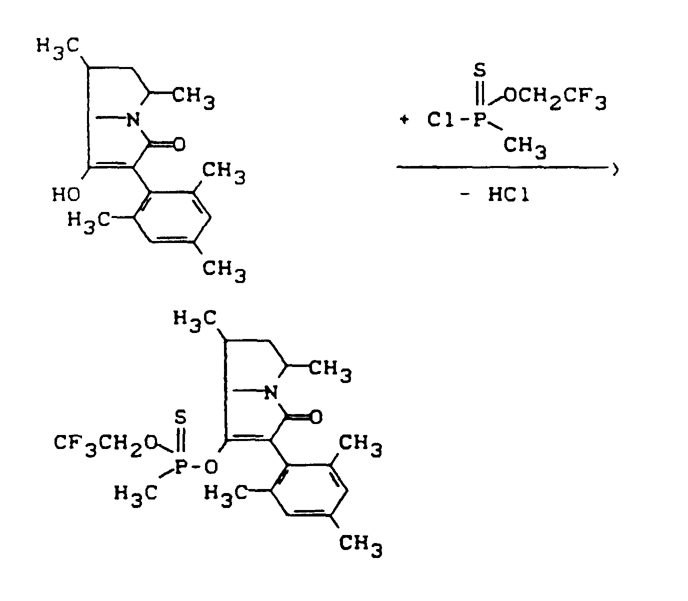

Verwendet man gemäß Verfahren (G) 3-(2,4,6-Trimethylphenyl)-1-aza-7,8-dimethyl-bicyclo-(3,3,0^{1,5})-octan-2,4-dion und NaOH als Komponenten, so kann der Verlauf des erfindungsgemäßen Verfahrens durch folgendes Reaktionsschema wiedergegeben werden:

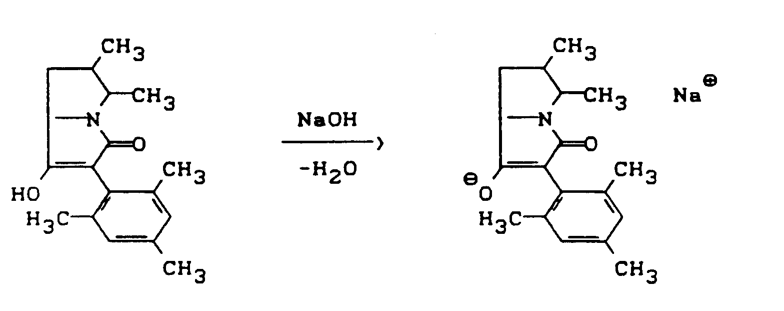

Die zur Durchführung des erfindungsgemäßen Verfahrens (A) als Ausgangsstoffe benötigten N-Acylaminosäureester sind durch die Formel (II) allgemein definiert. In dieser Formel (II) stehen A, B, Q, X, Y, Z, n und R¹⁰ vorzugsweise für diejenigen Reste, die bereits im Zusammenhang mit der Beschreibung der erfindungsgemäßen Stoffe der Formel (I) als bevorzugt für diese Substituenten genannt wurden.

Die Verbindungen der Formel (II) sind teilweise bekannt (vgl. beispielsweise EP-A 355 599, DE-A 3 831 852 und J. Heterocycl. Chem. 25 (1), 49-57). Die noch nicht bekannten Verbindungen lassen sich aber nach im Prinzip bekannten Methoden in einfacher Weise analog herstellen. So erhält man z.B. Acyl-aminosäureester der Formel (II), wenn man
a) Aminosäureester der Formel (XII) 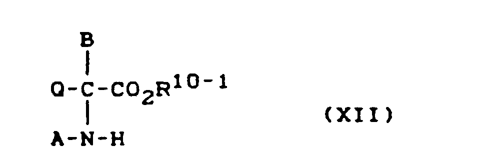 in welcher
   - R¹⁰⁻¹: für Wasserstoff (XIIa) oder Alkyl (XIIb) steht
   und
   - A, B und Q: die oben angegebene Bedeutung haben,
   mit Phenylessigsäurehalogeniden der Formel (XIII) 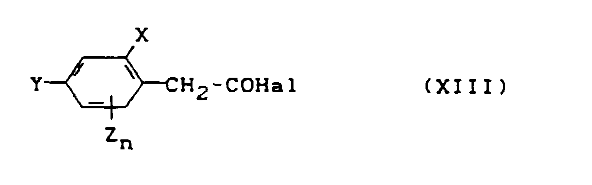 in welcher
   - X, Y, Z und n: die oben angegebene Bedeutung haben und
   - Hal: für Chlor oder Brom steht,
   acycliert (Chem. Reviews 52 237-416 (1953));
   oder
b) wenn man Acylaminosäuren der Formel (IIa), 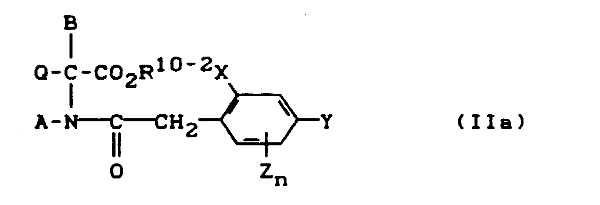 in welcher
   - A, B, Q, X, Y, Z und n: die oben angegebene Bedeutung haben,
   und
   - R¹⁰⁻²: für Wasserstoff steht,
   verestert (Chem. Ind. (London) 1568 (1968)).

Verbindungen der Formel XII, in welcher
- A, B und R¹⁰⁻¹: die oben angegebene Bedeutung haben, sind bekannt oder nach literaturbekannten Verfahren erhältlich (Henning, R., Urbach, H., Tetrahedron Lett. 24, 5339-5342 (1983); EP-A 52 870; US A-4 291 163; EP A-173 199; Urbach, H., Henning, R., Tetrahedron Lett. 26, 1839-1842 (1985).

Die Phenylessigsäurehalogenide der Formel (XIII) sind allgemein bekannte Verbindungen der organischen Chemie. Die zur Herstellung der Acyl-aminosäureester der Formel (II) benötigten Acylaminosäuren sind durch die Formel (IIa) allgemein definiert. In dieser Formel (IIa) stehen A, B, Q, X, Y, Z und n vorzugsweise für diejenigen Reste, die bereits im Zusammenhang mit der Beschreibung der erfindungsgemäßen Stoffe der Formel (I) als bevorzugt für diese Substituenten genannt wurden. R¹⁰⁻² steht für Wasserstoff.

Die Verbindungen der Formel (IIa) sind teilweise bekannt (vgl. beispielsweise EP-OS 355 599 und J. Am. Chem. Soc. 110 (4), 1180-6). Die noch nicht bekannten Verbindungen lassen sich aber nach im Prinzip bekannten Methoden in einfacher Weise analog herstellen.

Die Verbindungen der Formel (IIa) sind beispielsweise aus den Phenylessigsäurehalogeniden der Formel (XIII) und Aminosäuren der Formel (XIIa) nach Schotten-Baumann (Organikum 9. Auflage 446 (1970) VEB Deutscher Verlag der Wissenschaften, Berlin) erhältlich.

Beispielhaft aber nicht begrenzend seien außer den bei den Herstellungsbeispielen genannten Zwischenprodukte die folgenden Verbindungen der Formel (II) genannt:
N-(2,4-Dichlorphenylacetyl)-5-methyl-pyrrolidin-2-carbonsäureethylester, N-(2,6-Dichlorphenylacetyl)-5-methyl-pyrrolidin-2-carbonsäureethylester, N-(2,4,6-Trichlorphenylacetyl-5-methyl-pyrrolidin-2-carbonsäureethylester, N-(2,4-Dimethylphenylacetyl)-5-methyl-pyrrolidin-2-carbonsäureethylester, N-(2,6-Dimethylphenylacetyl)-5-methyl-pyrrolidin-2-carbonsäureethylester, N-(2,4,6-Trimethylphenylacetyl)-5-methyl-pyrrolidin-2-carbonsäureethylester, N-(2,4-Dichlorphenylacetyl)-4-methyl-pyrrolidin-2-carbonsäureethylester, N-(2,6-Dichlorphenylacetyl)-4-methyl-pyrrolidin-2-carbonsäureethylester, N-(2,4,6-Trichlorphenylacetyl)-4-methyl-pyrrolidin-2-carbonsäureethylester, N-(2,4-Dimethylphenylacetyl)-4-methyl-pyrrolidin-2-carbonsäureethylester, N-(2,6-Dimethylphenylacetyl)-4-methyl-pyrrolidin-2-carbonsäureethylester, N-(2,4,6-Trimethylphenylacetyl)-4-methyl-pyrrolidin-2-carbonsäureethylester, N-(2,4-Dichlorphenylacetyl)-3-methyl-pyrrolidin-2-carbonsäureethylester, N-(2,6-Dichlorphenylacetyl)-3-methyl-pyrrolidin-2-carbonsäureethylester, N-(2,4,6-Trichlorphenylacetyl)-3-methyl-pyrrolidin-2-carbonsäureethylester, N-(2,4-Dimethylphenylacetyl)-3-methyl-pyrrolidin-2-carbonsäureethylester, N-(2,6-Dimethylphenylacetyl)-3-methyl-pyrrolidin-2-carbonsäureethylester, N-(2,4,6-Trimethylphenylacetyl)-3-methyl-pyrrolidin-2-carbonsäureethylester, N-(2,4-Dichlorphenylacetyl)-2-methyl-pyrrolidin-2-carbonsäureethylester, N-(2,6-Dichlorphenylacetyl)-2-methyl-pyrrolidin-2-carbonsäureethylester, N-(2,4,6-Trichlorphenylacetyl)-2-methyl-pyrrolidin-2-carbonsäureethylester, N-(2,4-Dimethylphenylacetyl)-2-methyl-pyrrolidin-2-carbonsäureethylester, N-(2,6-Dimethylphenylacetyl)-2-methyl-pyrrolidin-2-carbonsäureethylester, N-(2,4,6-Trimethylphenylaetyl)-2-methyl-pyrrolidin-2-carbonsäureesthylester, N-(2,4-Dichlorphenylacetyl)-4,5-dimethyl-pyrrolidin-2-carbonsäureethylester, N-(2,6-Dichlorphenylacetyl)-4,5-dimethyl-pyrrolidin-2-carbonsäureethylester, N-(2,4,6-Trichlorphenylacetyl)-4,5-dimethyl-pyrrolidin-2-carbonsäureethylester, N-(2,4-Dimethylphenylacetyl)-4,5-dimethyl-pyrrolidin-2-carbonsäureethylester, N-(2,6-Dimethylphenylacetyl)-4,5-dimethylpyrrolidin-2-carbonsäureethylester; N-(2,4,6-Trimethylphenylacetyl)-4,5-dimethyl-pyrrolidin-2-carbonsäureethylester, N-(2,4-Dichlorphenylacetyl)-3,5-dimethylpyrrolidin-2-carbonsäureethylester, N-(2,6-Dichlorphenylacetyl)-3,5-dimethyl-pyrrolidin-2-carbonsäureethylester, N-(2,4,6-Trichlorphenylacetyl)-3,5-dimethyl-pyrrolidin-2-carbonsäureethylester, N-(2,4-Dimethylphenylacetyl)-3,5-dimethyl-pyrrolidin-2-carbonsäureethylester, N-(2,6-Dimethylphenylacetyl)-3,5-dimethyl-pyrrolidin-2-carbonsäureethylester, N-(2,4,6-Trimethylphenylacetyl)-3,5-dimethyl-pyrrolidin-2-carbonsäureethylester; N-(2,4-Dichlorphenylacetyl)-3,4,5-trimethyl-pyrrolidin-2-carbonsäureethylester, N-(2,6-Dichlorphenylacetyl)-3,4,5-trimethyl-pyrrolidin-2-carbonsäureethylester, N-(2,4,6-Trichlorphenylacetyl)-3,4,5-trimethyl-pyrrolidin-2-carbonsäureethylester, N-(2,4-Dimethylphenylacetyl)-3,4,5-trimethyl-pyrrolidin-2-carbonsäureethylester, N-(2,6-Dimethylphenylacetyl)-3,4,5-trimethyl-pyrrolidin-2-carbonsäureethylester, N-(2,4,6-trimethylphenylacetyl)-3,4,5-trimethyl-pyrrolidin-2-carbonsäureethylester, N-(2,4-Dichlorphenylacetyl)-5-cyclohexyl-pyrrolidin-2-carbonsäureethylester, N-(2,6-Dichlorphenylacetyl)-5-cyclohexyl-pyrrolidin-2-carbonsäureethylester, N-(2,4,6-Trichlorphenylacetyl)-5-cyclohexyl-pyrrolidin-2-carbonsäureethylester, N-(2,4-Dimethylphenylacetyl)-5-cyclohexyl-pyrrolidin-2-carbonsäureethylester, N-(2,6-Dimethylphenylacetyl)-5-cyclohexyl-pyrrolidin-2-carbonsäureethylester, N-(2,4,6-Trimethylphenylacetyl)-5-cyclohexyl-pyrrolidin-2-carbonsäureethylester, N-(2,4-Dichlorphenylacetyl)-4-hydroxy-pyrrolidin-2-carbonsäureethylester, N-(2,6-Dichlorphenylacetyl)-4-hydroxy-pyrrolidin-2-carbonsäureethylester, N-(2,4,6-Trichlorphenylacetyl)-4-hydroxy-pyrrolidin-2-carbonsäureethylester, N-(2,4-Dimethylphenylacetyl)-4-hydroxy-pyrrolidin-2-carbonsäureethylester, N-(2,6-Dimethylphenylacetyl)-4-hydroxy-pyrrolidin-2-carbonsäureethylester, N-(2,4,6-Trimethylphenylacetyl)-4-hydroxy-pyrrolidin-2-carbonsäureethylester, N-(2,4-Dichlorphenylacetyl)-4-methoxy-pyrrolidin-2-carbonsäureethylester, N-(2,6-Dichlorphenylacetyl)-4-methoxy-pyrrolidin-2-carbonsäureethylester, N-(2,4,6-Trichlorphenylacetyl)-4-methoxy-pyrrolidin-2-carbonsäureethylester, N-(2,4-Dimethylphenylacetyl)-4-methoxy-pyrrolidin-2-carbonsäureethylester, N-(2,6-Dimethylphenylacetyl)-4-methoxy-pyrrolidin-2-carbonsäureethylester, N-(2,4,6-Trimethylphenylacetyl)-4-methoxy-pyrrolidin-2-carbonsäureethylester, N-(2,4-Dichlorphenylacetyl)-3-methyl-piperidin-2-carbonsäureethylester, N-(2,6-Dichlorphenylacetyl)-3-methyl-piperidin-2-carbonsäureethylester, N-(2,4,6-Trichlorphenylacetyl)-3-methyl-piperidin-2-carbonsäureethylester, N-(2,4-Dimethylphenylacetyl)-3-methyl-piperidin-2-carbonsäureethylester, N-(2,6-Dimethylphenylacetyl)-3-methyl-piperidin-2-carbonsäureethylester, N-(2,4,6-Trimethylphenylacetyl)-3-methyl-piperidin-2-carbonsäureethylester, N-(2,4-Dichlorphenylacetyl)-4-methyl-piperidin-2-carbonsäureethylester, N-(2,6-Dichlorphenylacetyl)-4-methyl-piperidin-2-carbonsäureethylester, N-(2,4,6-Trichlorphenylacetyl)-4-methyl-piperidin-2-carbonsäureethylester, N-(2,4-Dimethylphenylacetyl)-4-methyl-piperidin-2-carbonsäureethylester, N-(2,6-Dimethylphenylacetyl)-4-methyl-piperidin-2-carbonsäureethylester, N-(2,4,6-Trimethylphenylacetyl)-4-methyl-piperidin-2-carbonsäureethylester, N-(2,4-Dichlorphenylacetyl)-5-methyl-piperidin-2-carbonsäureethylester, N-(2,6-Dichlorphenylacetyl)-5-methyl-piperidin-2-carbonsäureethylester, N-(2,4,6-Trichlorphenylacetyl)-5-methyl-piperidin-2-carbonsäureethylester, N-(2,4-Dimethylphenylacetyl)-5-methyl-piperidin-2-carbonsäureethylester, N-(2,6-Dimethylphenylacetyl)-5-methyl-piperidin-2-carbonsäureethylester, N-(2,4,6-Trimethylphenylacetyl)-5-methyl-piperidin-2-carbonsäureethylester, N-(2,4-Dichlorphenylacetyl)-6-methyl-piperidin-2-carbonsäureethylester, N-(2,6-Dichlorphenylacetyl)-6-methylpiperidin-2-carbonsäureethylester, N-(2,4,6-Trichlorphenylacetyl)-6-methyl-piperidin-2-carbonsäureethylester, N-(2,4-Dimethylphenylacetyl)-6-methyl-piperidin-2-carbonsäureethylester, N-(2,6-Dimethylphenylacetyl)-6-methyl-piperidin-2-carbonsäureethylester, N-(2,4,6-Trimethylphenylacetyl)-6-methyl-piperidin-2-carbonsäureethylester, N-(2,4-Dichlorphenylacetyl)-4,6-dimethyl-piperidin-2-carbonsäureethylester, N-(2,6-Dichlorphenylacetyl)-4,6-dimethyl-piperidin-2-carbonsäureethylester, N-(2,4,6-Trichlorphenylacetyl)-4,6-dimethyl-piperidin-2-carbonsäureethylester, N-(2,4-Dimethylphenylacetyl)-4,6-dimethyl-piperidin-2-carbonsäureethylester, N-(2,6-Dimethylphenylacetyl)-4,6-dimethyl-piperidin-2-carbonsäureethylester, N-(2,4,6-Trimethylphenylacetyl)-4,6-dimethyl-piperidin-2-carbonsäureethylester.

Die zur Durchführung der erfindungsgemäßen Verfahren (B), (C), (D), (E), (F) und (G) als Ausgangsstoffe benötigten 3-Aryl-pyrrolidon-2,4-dione bzw. deren Enole sind durch die Formel (Ia) allgemein definiert. In dieser Formel (Ia) stehen A, B, Q, X, Y, Z und n vorzugsweise für diejenigen Reste, die bereits im Zusammenhang mit der Beschreibung der erfindungsgemäßen Stoffe der Formel (I) als bevorzugt für diese Substituenten genannt wurden.

Die Verbindungen der Formel (Ia) sind neu und Gegenstand der vorliegenden Erfindung. Sie sind erhältlich nach Verfahren (A).

Die zur Durchführung der erfindungsgemäßen Verfahren (B), (C), (D), (E), (F) und (G) außerdem als Ausgangsstoffe benötigten Säurehalogenide der Formel (III), Carbonsäureanhydride der Formel (IV), Chlorameisensäureester oder Chlorameisensäurethioester der Formel (V), Chlormonothioameisensäureester oder Chlordithioameisensäureester der Formel (VI), Alkylhalogenide der Formel (VII), Sulfonsäurechloride der Formel (VIII), Phosphorverbindungen der Formel (IX) und Metallhydroxide oder Amine der Formel (X) und (XI) sind allgemein bekannte Verbindungen der organischen bzw. anorganischen Chemie.

Das Verfahren (Aα) ist dadurch gekennzeichnet, daß Verbindungen der Formel (II) in welcher A, X, Y, Z, n und R¹⁰ die oben angegebene Bedeutung haben, in Gegenwart von Basen einer intramolekularen Kondensation unterwirft.

Als Verdünnungsmittel können bei dem erfindungsgemäßen Verfahren (Aα) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Isopropanol, Butanol, iso-Butanol und tert.-Butanol.

Als Basen (Deprotonierungsmittel) können bei der Durchführung des erfindungsgemäßen Verfahrens (Aα) alle üblichen Protonenakzeptoren eingesetzt werden. Vorzugsweise verwendbar sind Alkalimetall- und Erdalkalimetall-oxide, -hydroxide und -carbonate, wie Natriumhydroxid, Kaliumhydroxid, Magnesiumoxid, Calciumoxid, Natriumcarbonat, Kaliumcarbonat und Calciumcarbonat, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 oder TDA 1 eingesetzt werden können. Weiterhin können
- Adogen 464 =: Methyltrialkyl(C₈-C₁₀)ammoniumchlorid
- TDA 1 =: Tris-(methoxyethoxyethyl)-amin

Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natriumethylat und Kalium-tert.-butylat einsetzbar.

Die Reaktionstemperaturen können bei der Durchführung des erfindungsgemäßen Verfahrens (Aα) innerhalb eines größeren Bereiches variiert werden. Im allgemeinen arbeitet man bei Temperaturen zwischen 0°C und 250°C, vorzugsweise zwischen 50°C und 150°C.

Die erfindungsgemäßen Verfahren Aα und Aβ werden im allgemeinen unter Normaldruck durchgeführt.

Bei der Durchführung des erfindungsgemäßen Verfahrens (Aα) setzt man die Reaktionskomponenten der Formeln (II) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Die Umsetzungen der Hydroxylgruppe gemäß dem erfindungsgemäßen Verfahren (A-β) zu den entsprechenden Estern und Ethern, die Swern-Oxidation oder die Pfitzner-Moffart-Oxidation sowie die Umsetzung der daraus entstehenden Ketone mit Aminen, Alkoholen, Diolen, Mercaptanen und Dithiolen erfolgt nach allgemein üblichen und bekannten Methoden (vgl. z.B. Organikum, VEB Deutscher Verlag der Wissenschaften, Berlin 1976 und Herstellungsbeispiele).

Das Verfahren (Bα) ist dadurch gekennzeichnet, daß man Verbindungen der Formel (Ia) mit Carbonsäurehalogeniden der Formel (III) umsetzt.

Als Verdünnungsmittel können bei dem erfindungsgemäßen Verfahren (Bα) bei Verwendung der Säurehalogenide alle gegenüber diesen Verbindungen inerten Solventien eingesetzt werden. Vorzugsweise verwendbar sind Kohlenwasser stoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Verwendet man die entsprechenden Carbonsäurehalogenide so kommen als Säurebindemittel bei der Umsetzung nach dem erfindungsgemäßen Verfahren (Bα) alle üblichen Säureakzeptoren in Betracht. Vorzugsweise verwendbar sind tertiäre Amine, wie Triethylamin, Pyridin, Diazabiyclooctan (DABCO), Diazabicycloundecan (DBU), Diazabicyclononen (DBN), Hüning-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat.

Die Reaktionstemperaturen können auch bei dem erfindungsgemäßen Verfahren (Bα) auch bei der Verwendung von Carbonsäurehalogeniden innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bα) werden die Ausgangsstoffe der Formel (Ia) und das Carbonsäurehalogenid der Formel (III) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Das Verfahren (Bβ) ist dadurch gekennzeichnet, daß man Verbindungen der Formel (Ia) mit Carbonsäurehydriden der Formel (IV) umsetzt.

Verwendet man bei dem erfindungsgemäßen Verfahren (Bβ) als Reaktionskomponente der Formel (IV) Carbonsäureanhydride, so können als Verdünnungsmittel vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäurehydrid gleichzeitig als Verdünnungsmittel fungieren.

Die Reaktionstemperaturen können bei dem erfindungsgemäßen Verfahren (Bα) auch bei der Verwendung von Carbonsäurehalogeniden innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens werden die Ausgangsstoffe der Formel (Ia) und das Carbonsäureanhydrid der Formel (IV) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (Ia) mit Chlorameisensäureestern oder Chlorameisensäurethiolestern der Formel (V) umsetzt.

Verwendet man die entsprechenden Chlorameisensäureester bzw. Chlorameisensäurethiolester so kommen als Säurebindemittel bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calcium-oxid, außerdem Alkali- und Erdalkalimetall-carbonate, wie Natriumcarbonat, Kaliumcarbonat und Calciumcarbonat.

Als Verdünnungsmittel können bei dem erfindungsgemäßen Verfahren (C) bei Verwendung der Chlorameisensäureester bzw. Chlorameisensäurethiolester alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogen kohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan.

Bei Verwendung der Chlorameisensäureester bzw. Chlorameisensäurethioiester als Carbonsäure-Derivate der Formel (V) können die Reaktionstemperaturen bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (Ia) und der entsprechende Chlorameisensäureester bzw. Chlorameisensäurethiolester der Formel (V) im allgemeinen in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Abziehen des Verdünnungsmittels einengt.

Beim Herstellungsverfahren (D_{α}) setzt man pro Mol Ausgangsverbindung der Formel (Ia) ca. 1 Mol Chlormonothioameisensäureester bzw. Chlordithioameisensäureester der Formel (VI) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Alkohole, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindung Ia dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (D_{*β*}) setzt man pro Mol Ausgangsverbindung der Formel (Ia) die äquimolare Menge bzw. einen Überschuß Schwefelkohlenstoff zu. Man arbeitet hierbei vorzugsweise bei Temperaturen von 0 bis 50°C und insbesondere bei 20 bis 30°C.

Oft ist es zweckmäßig zunächst aus der Verbindung der Formel (Ia) durch Zusatz eines Deprotonierungsmittels (wie z.B. Kaliumtertiärbutylat oder Natriumhydrid) das entsprechende Salz herzustellen. Man setzt die Verbindung (Ia) solange mit Schwefelkohlenstoff um, bis die Bildung der Zwischenverbindung abgeschlossen ist, z.B. nach mehrstündigem Rühren bei Raumtemperatur.

Die weitere Umsetzung mit dem Alkylhalogenid der Formel (VII) erfolgt vorzugsweise bei 0 bis 70°C und insbesondere bei 20 bis 50°C. Hierbei wird mindestens die äquimolare Menge Alkylhalogenid eingesetzt.

Man arbeitet bei Normaldruck oder unter erhöhtem Druck, vorzugsweise bei Normaldruck.

Die Aufarbeitung erfolgt wiederum nach üblichen Methoden.

Beim Herstellungsverfahren (E) setzt man pro Mol Ausgangsverbindung der Formel (Ia) ca. 1 Mol Sulfonsäurechlorid (VIII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Nitrile, Alkohole, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung Ia dar, kann auf den weitern Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (E) kann gegebenenfalls unter Phasen-Transfer-Bedingungen gearbeitet werden (W.J. Spillane et. al.; J. Chem. Soc., Perkin Trans I, (3) 677-9 (1982)). In diesem Fall setzt man pro Mol Ausgangsverbindung der Formel a) 0,3 bis 1,5 mol Sulfonsäurechlorid VIII, bevorzugt 0,5 mol bei 0° bis 150°C, vorzugsweise bei 20 bis 70°C um.

Als Phasen-Transfer-Katalysatoren können alle quartären Ammoniumsalze verwendet werden, vorzugsweise Tetraoctylammoniumbromid und Benzyltriethylammoniumchlorid. Als organische Lösungsmittel können in diesem Fall alle unpolaren inerten Lösungsmittel dienen, bevorzugt werden Benzol und Toluol eingesetzt.

Beim Herstellungsverfahren (F) setzt man zum Erhalt von Verbindungen der Struktur (Ie) auf 1 Mol der Verbindung (Ia), 1 bis 2, vorzugsweise 1 bis 1,3 Mol der Phosphorverbindung der Formel (IX) bei Temperaturen zwischen -40°C und 150°C, vorzugsweise zwischen -10 und 110°C um.

Als gegebenenfalls zugesetzte Verdünnungsmittel kommen aller inerten, polaren organischen Lösungsmittel in Frage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Dimethylsulfid eingesetzt.

Als gegebenenfalls zugesetzte Säurebindemittel kommen übliche anorganische oder organische Basen in Frage wie Hydroxide, Carbonate. Beispielhaft seien Natriumhydroxid, Natriumcarbonat, Kaliumcarbonat, Pyridin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Das Verfahren (G) ist dadurch gekennzeichnet, daß man Verbindungen der Formel (Ia) mit Metallhydroxiden (X) oder Aminen (XI) umsetzt.

Als Verdünnungsmittel können bei dem erfindungsgemäßen Verfahren vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (H) wird im allgemeinen unter Normaldruck durchgeführt. Die Reaktionstemperaturen liegen im allgemeinen zwischen -20°C und 100°C, vorzugsweise zwischen 0°C und 50° C.

Bei der Durchführung des erfindungsgemäßen Verfahrens (G) werden die Ausgangsstoffe der Formel (Ia) bzw. (X) oder (XI) im allgemeinen in angenähert äquimolaren Mengen verwendet. Es ist jedoch auch möglich, die eine oder andere Komponente in einem größeren Überschuß (bis zu 2 Mol) einzusetzen. Im allgemeinen geht man so vor, daß man das Reaktionsgemisch durch Abziehen des Verdünnungsmittel einengt.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetis oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarso nemus spp., Bryobia praetiosa, Panonychus spp., Tetranychus spp..

Die erfindungsgemäßen Wirkstoffe zeichnen sich durch eine hohe insektizide und Akarizide Wirksamkeit aus.

Sie lassen sich mit besonders gutem Erfolg zur Bekämpfung von pflanzenschädigenden Insekten, wie beispielsweise gegen die Larven des Meerettichblattkäfers (Phaedon cochleariae) oder gegen die Larven der grünen Reiszikade (Nephotettix cincticeps) zur Bekämpfung von pflanzenschädigenden Milben, wie beispielsweise gegen die gemeine Spinnmilbe oder die Bohnenspinnmilbe (Tetranychus urticae) einsetzen.

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachauflaufverfahren. Sie können beispielsweise in Soja, Helianthus (Sonnenblume) oder Zuckerrüben mit sehr gutem Erfolg zur Bekämpfung von Schadgräsern eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.
Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- undloder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.
Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage. Weiterhin kommen 2,4-Dichlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxybenzonitril (BROMOXYNIL); 2-Chlor-N- [(4-methoxy-6-methyl-1,3,5-triazin-2-yl)-amino]-carbonyl -benzolsulfonamid (CHLORSULFURON); 2-[4-(2,4-Dichlorphenoxy)-phenoxy]-propionsäure, deren Methyl- oder deren Ethylester (DICLOFOPMETHYL); 4-Amino-6-t-butyl-3-ethylthio-1,2,4-triazin-5(4H)-on (ETHIOZIN); 2- 4-[(6-Chlor-2-benzoxazolyl)-oxy]-phenoxy -propansäure, deren Methyl- oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)harnstoff (ISOPROTURON); (2-Methyl-4-chlorphenoxy)essigsäure (MCPA); (4-Chlor-2-methylphenoxy)-propionsäure (MCPP); N-Methyl-2-(1,3-benzthiazol-2-yloxy)acetanilid (MEFENACET); 2- [[((4-Methoxy-6-methyl-1,3,5-triazin-2-yl)-amino)-carbonyl]-amino]-sulfonylbenzoesäure oder deren Methylester (METSULFURON); N-(1-Ethylpropyl)-3,4-dimethyl-2,6-dinitroanilin (PENDIMETHALIN); 0-(6-Chlor-3-phenylpyridazin-4-yl)-S-octylthiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON) in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B, durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

### Herstellungsbeispiele

### Beispiel 1

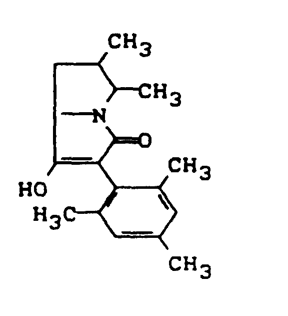

### (Verfahren (A))

Zu einer siedenden Lösung von 2 g (0,067 Mol) NaH in 50 ml absolutem Toluol werden 16 g (0,048 Mol) N-(2,4,6-Trimethylphenylacetyl-4,5-dimethyl-pyrrolidin-2-carbonsäureethylester in 50 ml absolutem Toluol zugetropft und am Rückfluß erhitzt. Das Ende der Reaktion wird durch DC-Kontrolle ermittelt. Anschließend läßt man auf Raumtemperatur abkühlen und gibt unter Kühlung solange absolutes Ethanol zu, bis kein Wasserstoff mehr entweicht. Das Reaktionsgemisch wird unter vermindertem Druck eingedampft, der Rückstand in Wasser aufgenommen und bei Raumtemperatur mit konzentrierter Salzsäure angesäuert. Der Niederschlag wird abgesaugt und im Vakuum bei 70°C über P₂O₅ getrocknet. Die Reinigung erfolgt durch Auskochen mit Chloroform /Methyl-tert-butylether und Ether/n-hexan.

Man erhält 6,2 g (45,3%) der Theorie 3-(2,4,6-Trimethylphenyl)-1-aza-7,8-dimethyl-bicyclo-(3,3,0^{1,5})-octan-2,4-dion mit einem Schmelzpunkt >220°C.

### Beispiel 2

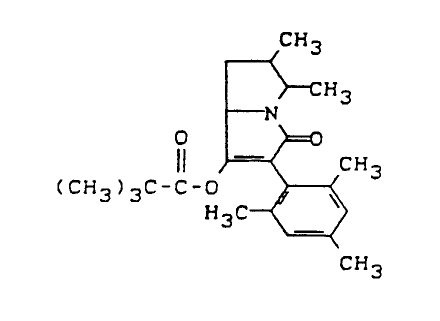

### (Verfahren (Bα))

Eine Mischung aus 2,3 g (0,008 Mol) 3-(2,4,6-Trimethylphenyl)-1-aza-7,8-dimethyl-bicyclo-(3,3,0^{1,5})-octan-2,4-dion und 1,4 ml Ethyldiisopropylamin werden auf 0°C bis 10°C gekühlt und dazu 1,01 ml Pivaloylchlorid in 5 ml Methyl-tert-butylether getropft. Anschließend wird der Ansatz bis zum Reaktionsende bei Raumtemperatur gerührt (DC-Kontrolle). Danach wird der ausgefallene Niederschlag abgesaugt, mit Methyl-tert-butylether nachgewaschen und das Filtrat unter vermindertem Druck eingedampft.

Nach der säulenchromatographischen Reinigung an Kieselgel (Laufmittel Cyclohexan/Essigester 1:1) erhalt man 2,12 g (71,7% der Theorie) 3-(2,4,6-Trimethylphenyl)-4-(tert-butylcarbonyloxy)-1-aza-7,8-dimethyl-bicyclo-(3,3,0^{1,5})-octan-2-on vom Schmelzpunkt 89°C.

### Beispiel 3

Auf analoge Weise zu Beispiel 2 erhält man 3-(2,4,6-Trimethylphenyl)-4-acetyloxy-1-aza-7,8-dimethyl-bicyclo(3,3,0^{1,5})-octan-2-on als Öl.

### Beispiel 4:

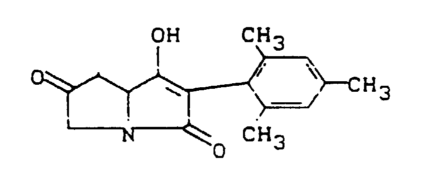

Man tropft zu einer Lösung von 28,0 g (0,22 mol) Oxalylchlorid in 500 ml absoluten Dichlormethan bei - 60°C eine Lösung von 34,4 g (0,44 mol) Dimethylsulfoxid in 100 ml absoluten Dichlormethan zu, rührt 10 min. und tropft dann bei - 60°C eine Lösung von 54,66 g (0,2 mol) 3-(2,4,6-Trimethylphenyl)-1-aza-7-hydroxy-bicyclo-(3,3,0^{1,5})-octan-2,4-dion in 200 ml absoluten Dichlormethan/Dimethylsufoxid (10:1) zu. Man rührt weitere 15 min., gibt dann 101,2 g (1,0 mol) Triethylamin zu, läßt auf Raumtemperatur erwärmen und setzt dem Reaktionsgemisch 1 1 Wasser zu. Die org. Phase wird abgetrennt, getrocknet und bei 180°C am Rotationsverdampfer eingedampft. Die Reinigung erfolgt durch Flash-Chromatographie an Kieselgel mit Methylenchlorid/Methanol 20:1.

Man erhält 50,56 g (93 % d. Th.) 3-(2,4,6-Trimethylphenyl)-1-aza-7-oxy-bicyclo-(3,3,0^{1,5})-octan-2,4-dion mit einem Schmelzpunkt von 240°C.

### Beispiel 5:

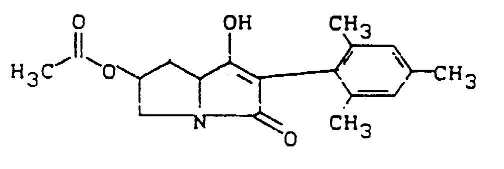

13.67 g (50 mmol) 3-(2,4,6-Trimethylphenyl)-1-aza-7-hydroxy-bicyclo-(3,3,0^{1,5})-octan-2,4-dion werden mit 50 ml einer 33%igen Lösung von Bromwasserstoff in Eisessig 16 Stunden bei Raumtemperatur gerührt. Zur Aufarbeitung gießt man das Reaktionsgemisch in 1 l Eiswasser, saugt den Niederschlag ab und trocknet bei 5°C im Vakuumtrockenschrank.

Man erhält 11,9 g (76 % d. Th) 3-(2,4,6-Trimethylphenyl)-1-aza-7-acetyloxy-bicyclo-(3,3,0^{1,5})-octan-2,4-dion mit einem Schmelzpunkt von 223 °C.

### Beispiel 6:

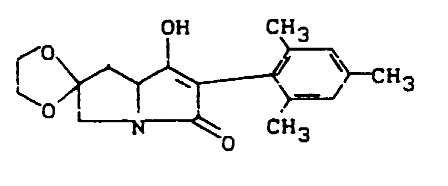

13,55 g (50 mmol) 3-(2,4,6-Trimethylphenyl)-1-aza-7-oxybicyclo-(3,3,0^{1,5})-octan-2,4-dion, 15,53 g (250 mmol) Ethylenglykol und 0,5 g p-Toluolsulfonsäure werden im 250 ml absoluten Toluol 16 h am Wasserabscheider gekocht. Nach dem Abkühlen auf RT wird der Niederschlag abgesaugt, mit Toluol gewaschen und getrocknet.

Man erhält 15,24 g (86 % d. Th.) 3-(2,4,6-Trimethylphenyl)-1-aza-7-(2,5-dioxo-spiropentyl)-bicyclo-(3,3,0^{1,5})-octan-2,4-dion mit einem Schmelzpunkt von 235-236°C.

Analog zu den Herstellungsbeispielen 1 bis 3 und gemäß den allgemeinen Angaben zu den erfindungsgemäßen Verfahren erhält man die in Tabelle 2 aufgeführten substituierten bicyclischen 3-Arylpyrrolidin-2,4-dion-Derivate der Formel (I)

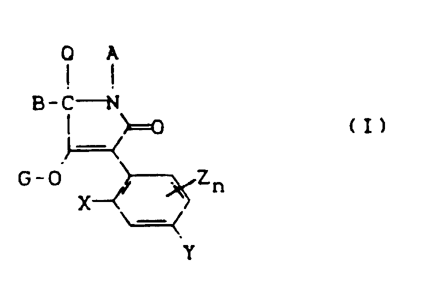

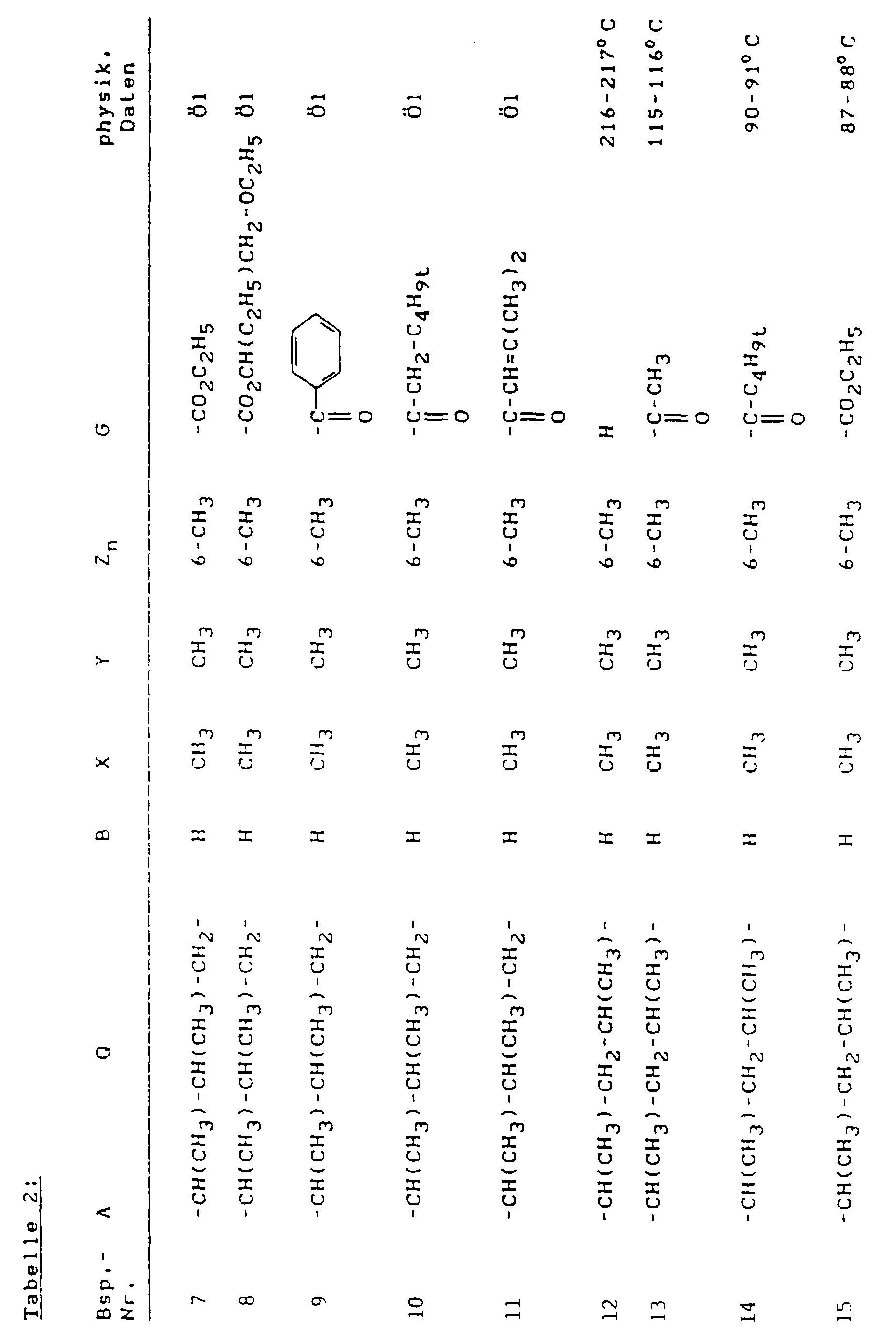

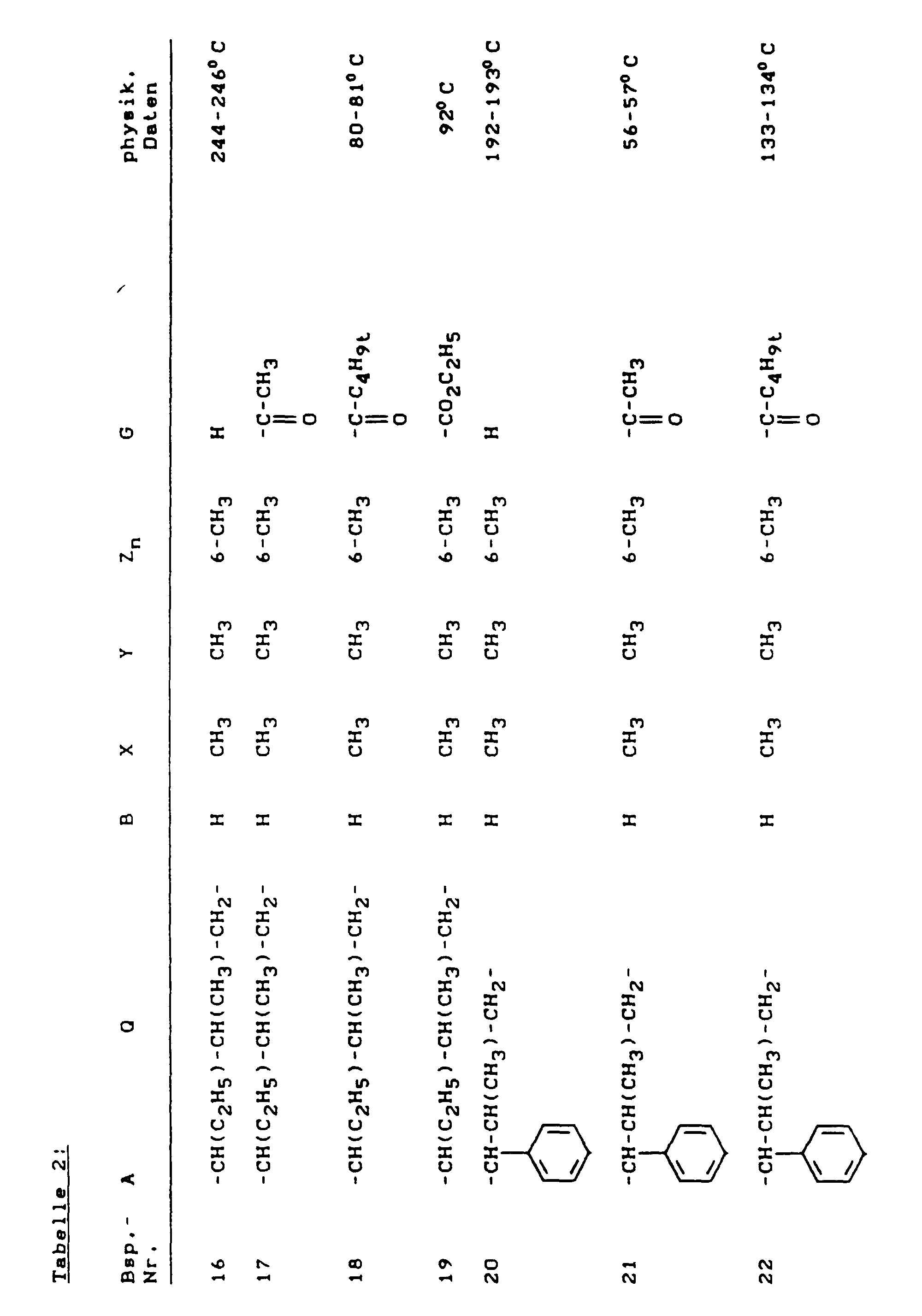

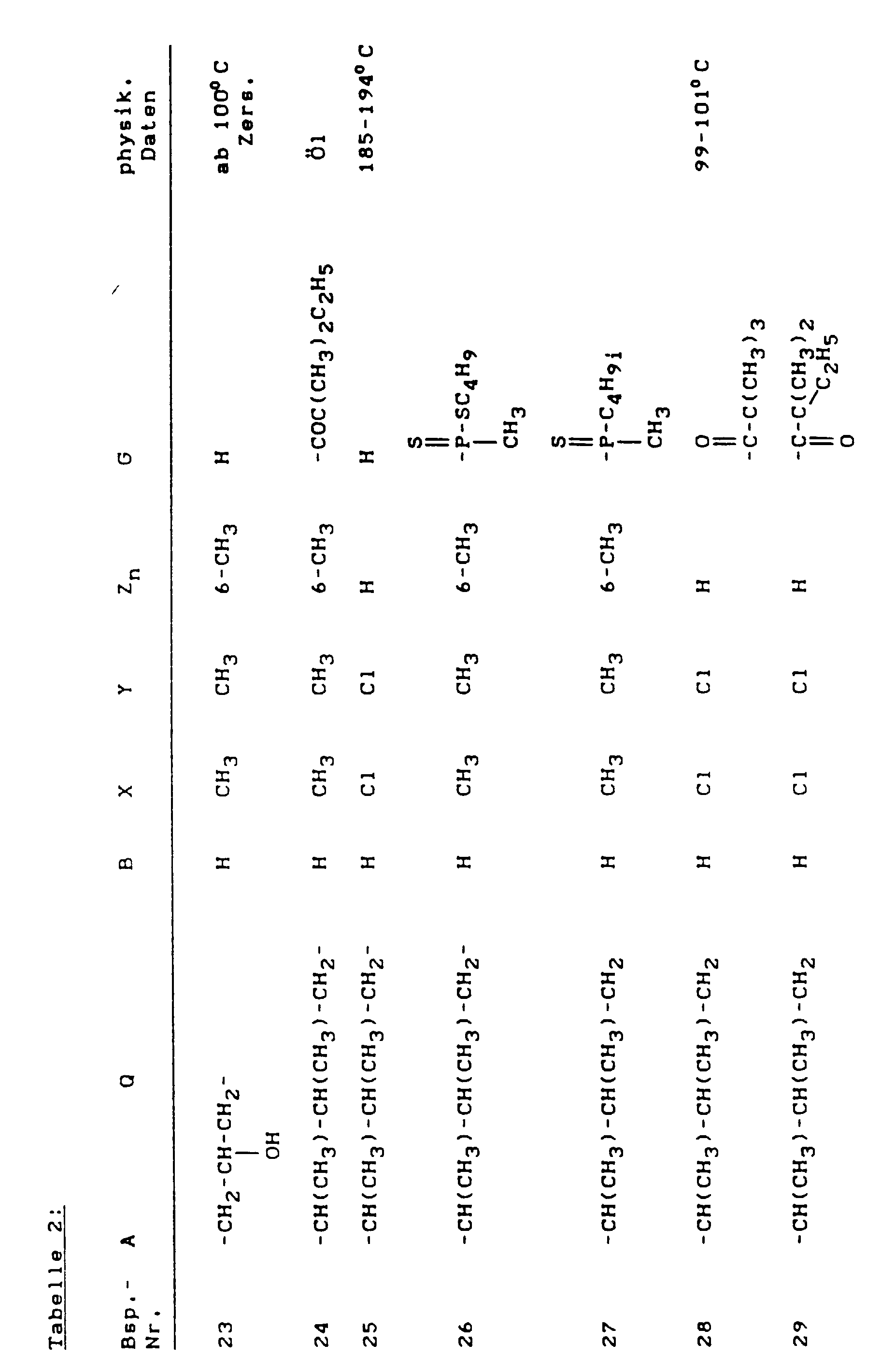

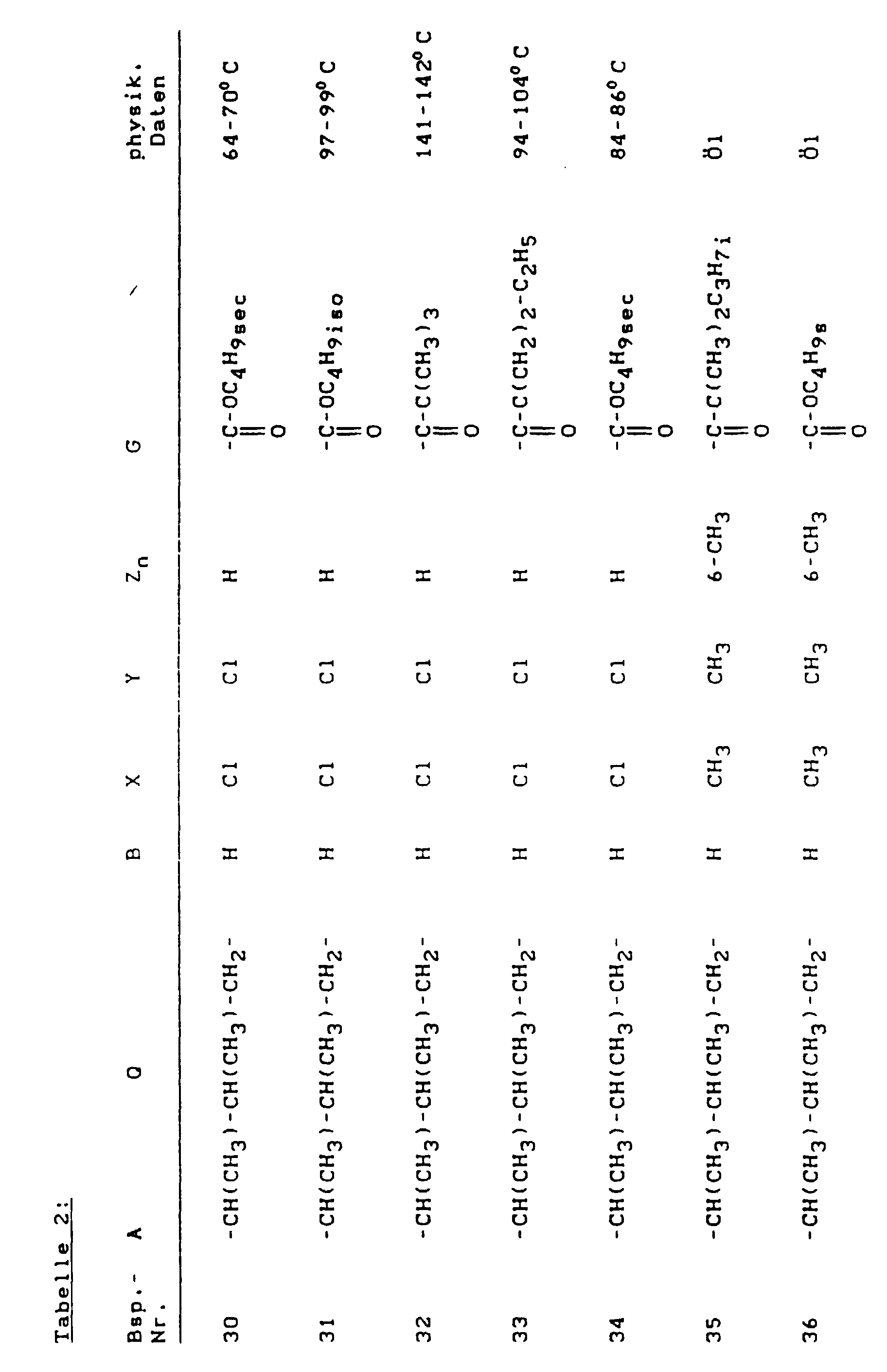

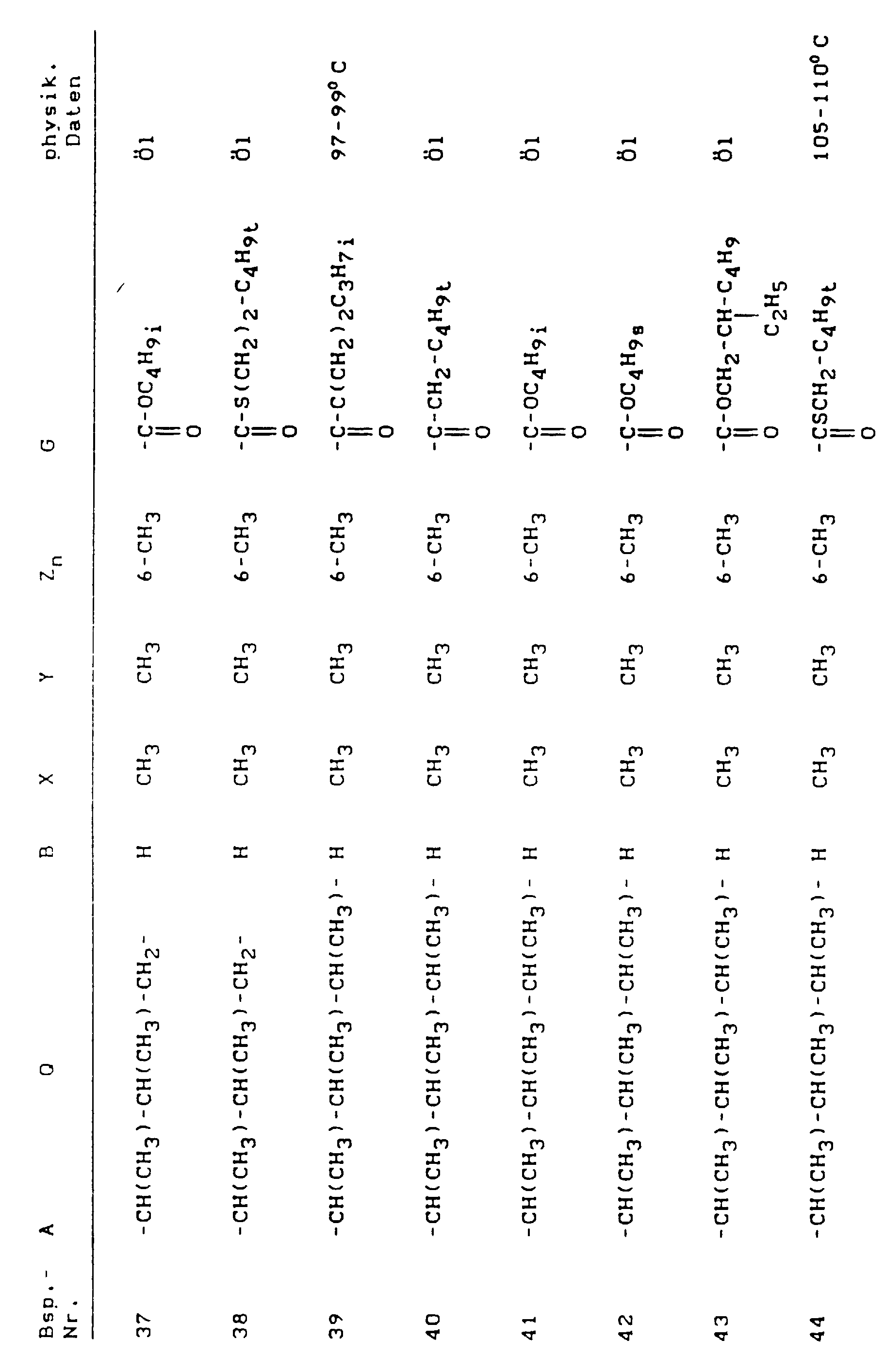

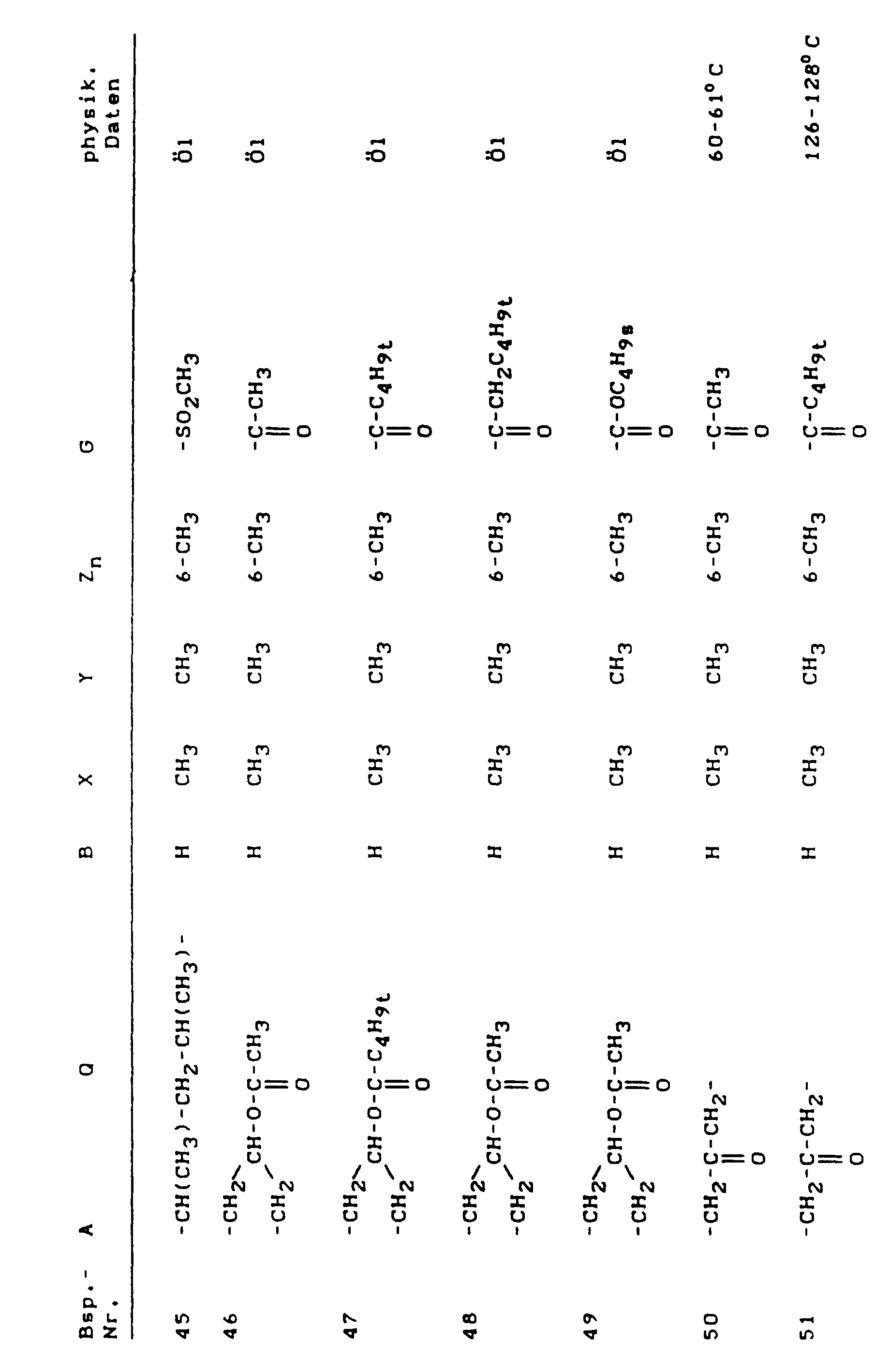

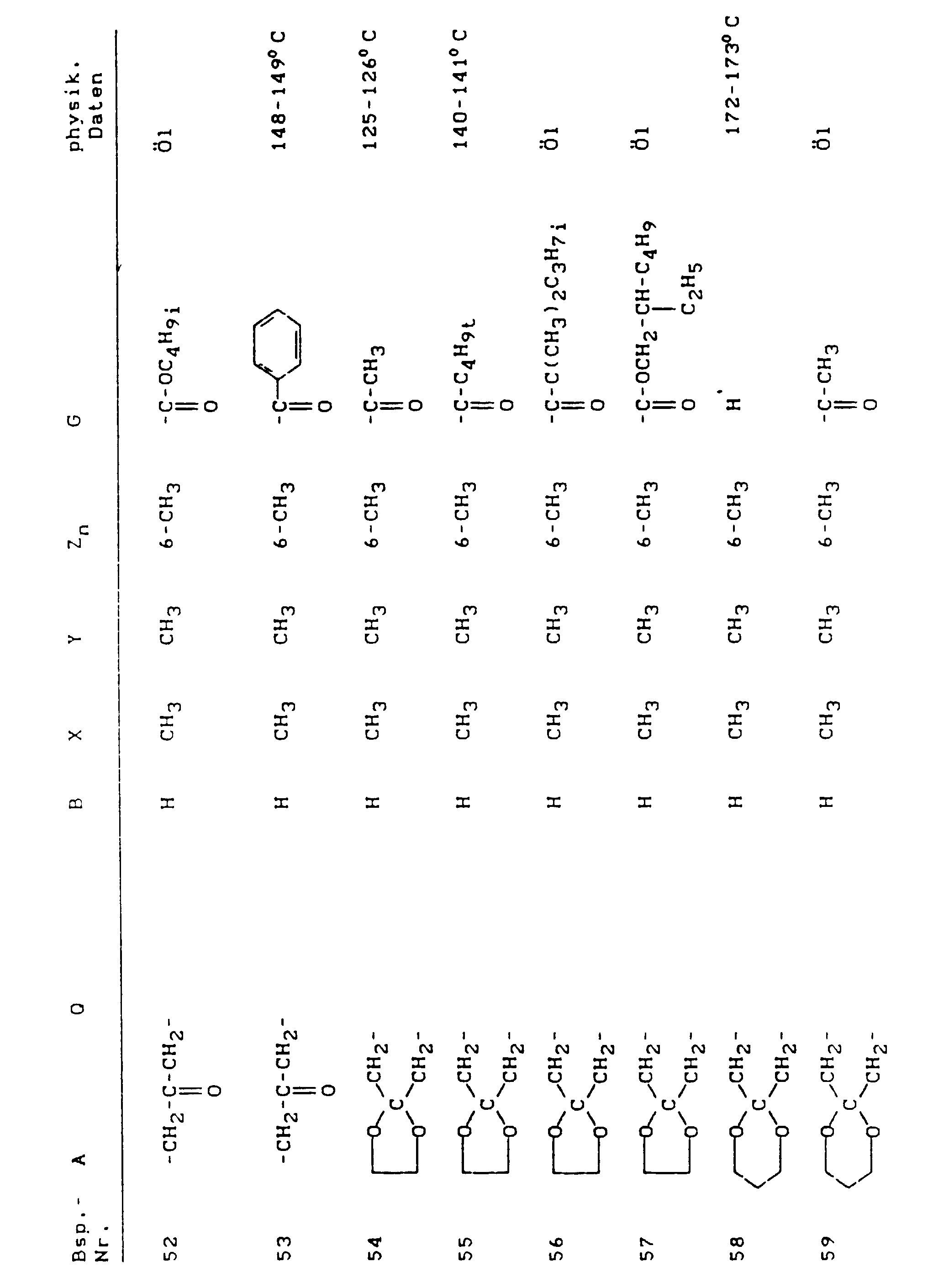

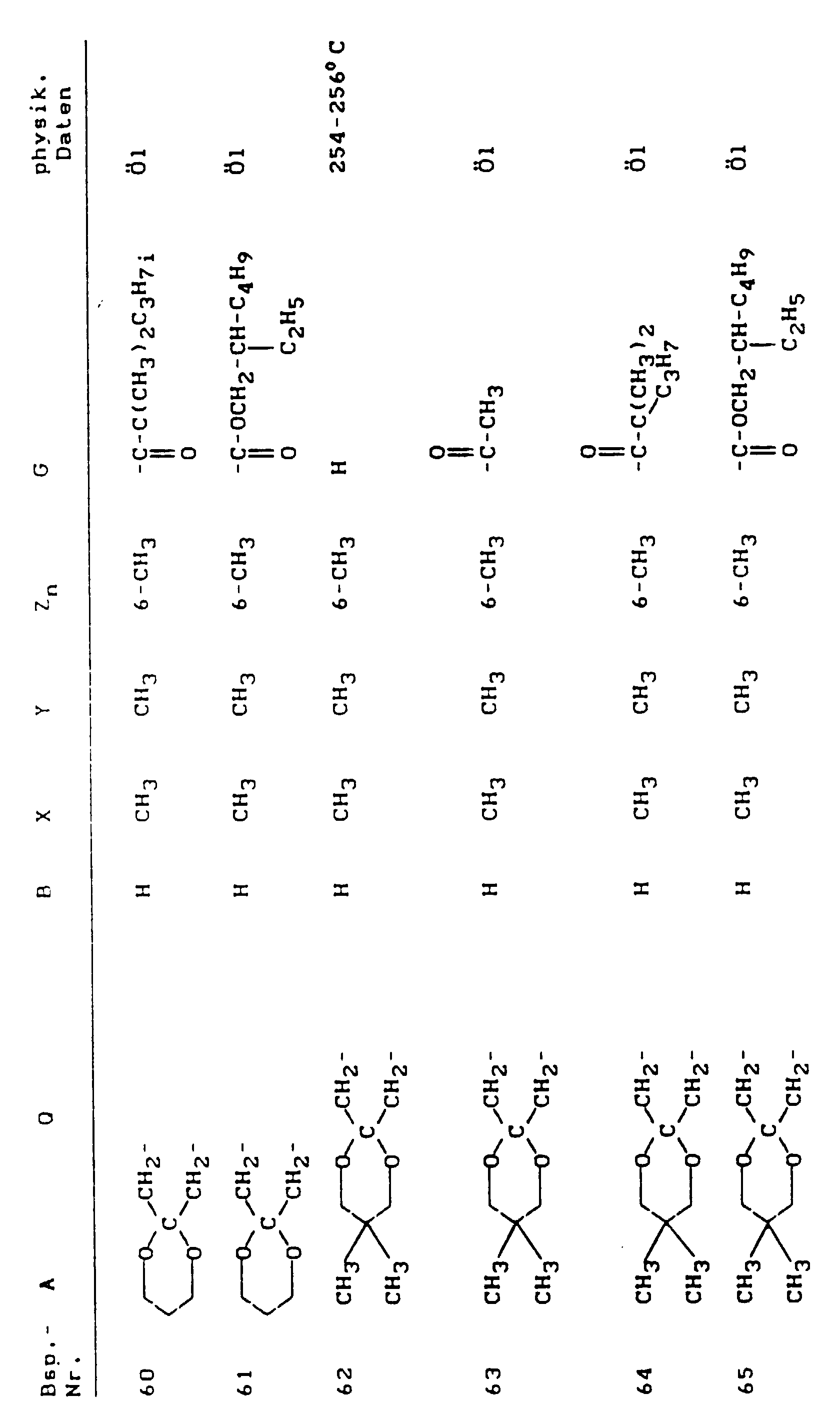

### Herstellung der Zwischenprodukte

### Beispiel (II-1)

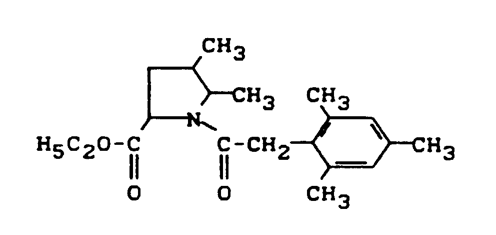

11,7 g (0,068 Mol) 4,5-Dimethyl-pyrrolidin-2-carbonsäureethylester und 9,6 ml Triethylamin werden auf 0°C bis 10°C gekühlt und dazu 13,4 g (0,068 Mol) Mesitylenessigsäurechlorid in 10 ml abs. Tetrahydrofuran getropft. Anschließend wird der Ansatz bis zum Reaktionsende bei Raumtemperatur gerührt (DC-Kontrolle). Danach wird das Reaktionsgemisch in eine Lösung von 300 ml Eiswasser und 100 ml 1N Salzsäure gerührt, mit Dichlormethan extrahiert, getrocknet und das Lösungsmittel unter vermindertem Druck abgezogen.

Man erhält 16 g (71% der Theorie) N-(2,4,6-Trimethylphenylacetyl-4,5-dimethyl-pyrrolidin-2-carbonsäureethylester als Öl.

### Beispiel (II-2)

Analog zu Beispiel (II-1) erhält man N-(2,4,6-Trimethylphenylacetyl-4-hydroxy-pyrrolidin-2-carbonsäuremethylester vom Schmelzpunkt 138-139°C.

### Anwendungsbeispiele:

In den folgenden Anwendungsbeispielen wurden die nachstehend aufgeführten Verbindungen als Vergleichssubstanzen eingesetzt: 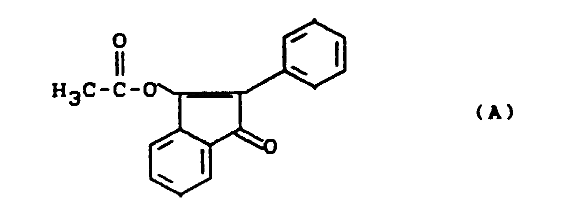 3-(Acetyloxy)-2-phenyl-1H-inden-1-on (bekannt aus US 4 104 043) 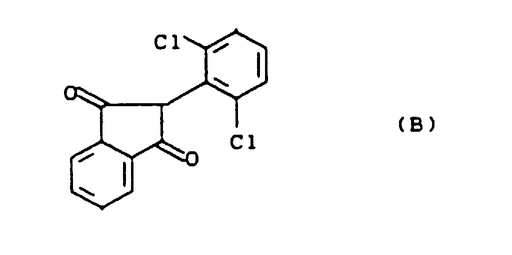 2-(2,6-Dichlorphenyl)-1H-inden-1,3(2H)-dion (bekannt aus US 3 954 998) 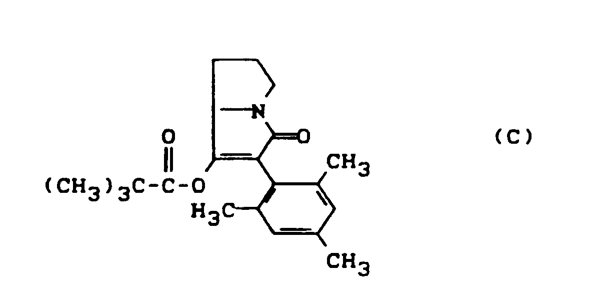 2,2-Dimethyl-2,3,5,7a-tetrahydro-5-oxo-6-(2,4,6-trimethylphenyl)-1H-pyrrolizin-7-yl-propionsäureester (bekannt aus EP-A 355 599) 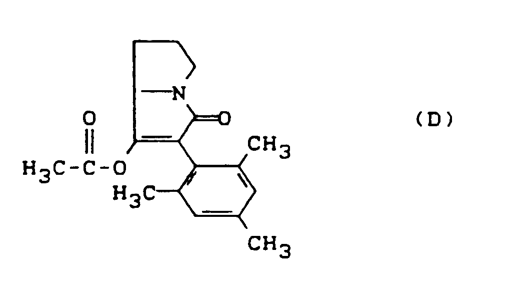 1-(Acetyloxy)-5,6,7,7a-tetrahydro-2-(2,4,6-trimethylphenyl)-3H-pyrrolizin-3-on (bekannt aus EP-A 355 599) 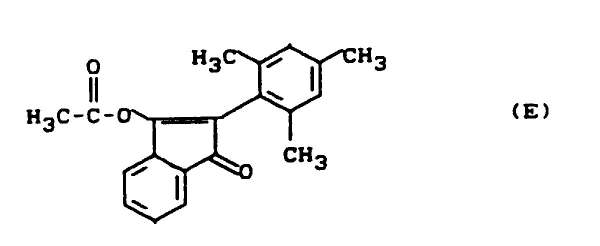 3-(Acetyloxy)-2-(2,4,6-trimethylphenyl)-1H-inden-1-on (bekannt aus US 4 104 043)

### Beispiel A

### Nephotettix-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichstteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigen z.B. die folgenden Verbindungen der Herstellungsbeispiele überlegene Wirksamkeit gegenüber dem Stand der Technik: 1, 2 und 3.

### Beispiel B

### Phaedon-Larven-Test

- Lösungsmittel:: 3 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 1, 2 und 3.

### Beispiel C

### Tetranychus-Test (OP-resistent)

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % be stimmt. Dabei bedeutet 100 %, daß alle Spinnmilben abgetötet wurden; 0 % bedeutet, daß keine Spinnmilben abgetötet wurden.

Bei diesem Test zeigt z.B. die folgende Verbindung der Herstellungsbeispiele überlegene Wirksamkeit gegenüber dem Stand der Technik: 2 und 3.

### Beispiel D

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

| | |
|---|---|
| 0 % = | keine Wirkung (wie unbehandelte Kontrolle) |
| 100 % = | totale Vernichtung |

Eine deutliche Überlegenheit in der Wirksamkeit ebenso wie in der Nutzpflanzenselektivität gegenüber dem Stand der Technik zeigen in diesem Test z.B. die Verbindungen gemäß den Herstellungsbeispielen 1, 2 und 3.

### Beispiel E

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

| | |
|---|---|
| 0 % = | keine Wirkung (wie unbehandelte Kontrolle) |
| 100 % = | totale Vernichtung |

Eine deutliche Überlegenheit in der Wirksamkeit ebenso wie in der Nutzpflanzenselektivität gegenüber dem Stand der Technik zeigt in diesem Test z.B. die Verbindung gemäß Herstellungsbeispiel 1.

## Patentansprüche (Patentansprüche für folgende(n) Vertragsstaat(en): BE, CH, DE, DK, FR, GB, GR, IT, LI, NL)

1. Substituierte bicyclische 3-Aryl-pyrrolidin-2,4-dion-Derivate der Formel (I) 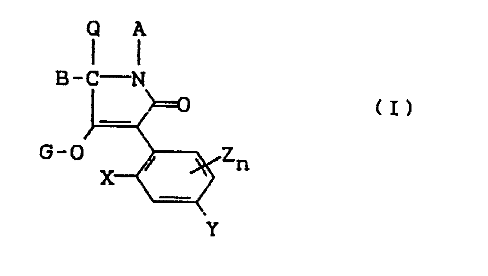 dadurch gekennzeichnet, daß
A und Q gemeinsam für eine jeweils einfach bis dreifach, gleich oder verschieden substituierte Alkandiyl-, Alkendiyl-, Alkendienyl- oder Alkentrienylgruppierung mit 3 bis 6 Kohlenstoffatomen stehen, welche jeweils durch Halogen, Hydroxy, Mercapto, jeweils gegebenenfalls durch Halogen substituiertes Alkyl mit 1 bis 10 Kohlenstoffatomen, Alkylcarbonyloxy mit 1 bis 8 Kohlenstoffatomen, Alkoxy mit 1 bis 6 Kohlenstoffatomen, Alkylthio mit 1 bis 6 Kohlenstoffatomen, Cycloalkyl mit 3 bis 7 Kohlenstoffatomen oder Aryl mit 6 bis 10 Kohlenstoffatomen substituiert ist und welche außerdem gegebenenfalls durch eine der nachstehenden Gruppierungen 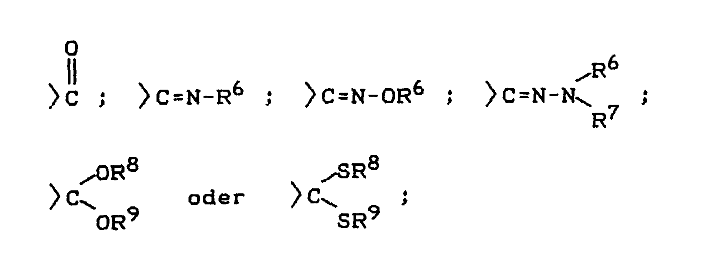 unterbrochen ist,
B für Wasserstoff oder Alkyl mit 1 bis 6 Kohlenstoffatomen steht,
X für C₁-C₆-Alkyl, Halogen oder C₁-C₆-Alkoxy steht,
Y für Wasserstoff, C₁-C₆-Alkyl, Halogen, C₁-C₆-Alkoxy oder C₁-C₃-Halogenalkyl steht,
Z für C₁-C₆-Alkyl, Halogen oder C₁-C₆-Alkoxy steht,
n für eine Zahl 0, 1, 2 oder 3 steht,
G für Wasserstoff (a) oder für die Gruppen 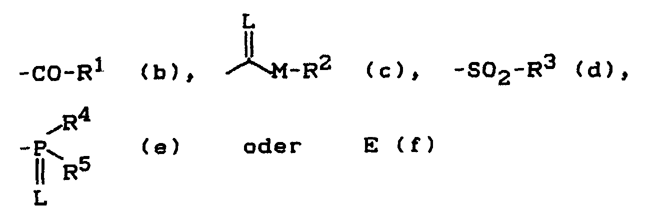
steht,
in welchen
E für ein Metallionäquivalent oder ein Ammoniumion steht,
L und M für Sauerstoff und/oder Schwefel steht,
R¹ für gegebenenfalls durch Halogen substituiertes C₁-C₂₀-Alkyl, C₂-C₂₀-Alkenyl, C₁-C₈-Alkoxy-C₂-C₈-alkyl, C₁-C₈-Alkylthio-C₂-C₈-alkyl, C₁-C₈-Polyalkoxy-C₂-C₈-alkyl oder Cycloalkyl mit 3 bis 8 Ringatomen, das durch Sauerstoff- und/oder Schwefelatome unterbrochen sein kann, steht,
für gegebenenfalls durch Halogen, Nitro, C₁-C₆-Alkyl, C₁-C₆-Alkoxy, C₁-C₆-Halogenalkyl, C₁-C₆-Halogenalkoxy substituiertes Phenyl,
für gegebenenfalls durch Halogen, C₁-C₆-Alkyl, C₁-C₆-Alkoxy, C₁-C₆-Halogenalkyl, C₁-C₆-Halogenalkoxy substituiertes Phenyl-C₁-C₆-alkyl steht,
für gegebenenfalls durch Halogen und/oder C₁-C₆-Alkyl substituiertes Hetaryl steht,
für gegebenenfalls durch Halogen und C₁-C₆-Alkyl substituiertes Phenoxy-C₁-C₆-alkyl steht,
für gegebenenfalls durch Halogen, Amino und C₁-C₆-Alkyl substituiertes Hetaryloxy-C₁-C₆-Alkyl steht,
R² für gegebenenfalls durch Halogen substituiertes C₁-C₂₀-Alkyl, C₂-C₂₀-Alkenyl, C₁-C₈-Alkoxy-C₂-C₈-alkyl, C₁-C₈-Polyalkoxy-C₂-C₈-alkyl steht,
für gegebenenfalls durch Halogen, Nitro, C₁-C₆-Alkyl, C₁-C₆-Alkoxy, C₁-C₆-Halogenalkyl substituiertes Phenyl oder Benzyl steht,
R³, R⁴ und R⁵ unabhängig voneinander für gegebenenfalls durch Halogen substituiertes C₁-C₈-Alkyl, C₁-C₈-Alkoxy, C₁-C₈-Alkylamino, Di-(C₁-C₈)-alkylamino, C₁-C₈-Alkylthio, C₂-C₅-Alkenylthio, C₂-C₅-Alkinylthio, C₃-C₇-Cycloalkylthio, für gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl, C₁-C₄-Halogenalkyl substituiertes Phenyl, Phenoxy oder Phenylthio stehen,
R⁶ und R⁷ unabhängig voneinander für Wasserstoff, gegebenenfalls durch Halogen substituiertes C₁-C₂₀-Alkyl, C₁-C₂₀-Alkoxy, C₂-C₈-Alkenyl, C₁-C₂₀-Alkoxy-C₁-C₂₀-alkyl, für gegebenenfalls durch Halogen, C₁-C₂₀-Halogenalkyl, C₁-C₂₀-Alkyl oder C₁-C₂₀-Alkoxy substituiertes Phenyl, für gegebenenfalls durch Halogen, C₁-C₂₀-Alkyl, C₁-C₂₀-Halogenalkyl oder C₁-C₂₀-Alkoxy substituiertes Benzyl steht oder zusammen für einen gegebenenfalls durch Sauerstoff unterbrochenen C₂-C₆-Alkylenring stehen,
R⁸ und R⁹ unabhängig voneinander für Wasserstoff oder Alkyl mit 1 bis 6 Kohlenstoffatomen stehen oder
R⁸ und R⁹ zusammen für einen Alkandiylrest mit 2 bis 4 Kohlenstoffatomen stehen.

2. Substituierte bicyclische 3-Aryl-pyrrolidin-2,4-dion-Derivate der Formel (I) gemäß Anspruch 1, dadurch gekennzeichnet, daß
A und Q gemeinsam für eine jeweils einfach bis dreifach, gleich oder verschieden substituierte Alkandiyl-, Alkendiyl-, Alkendienylgruppierung mit 3 bis 5 Kohlenstoffatomen stehen, welche jeweils durch Fluor, Chlor, Brom, Hydroxy, Mercapto, jeweils gegebenenfalls durch Halogen substituiertes Alkyl mit 1 bis 8 Kohlenstoffatomen, Alkylcarbonyloxy mit 1 bis 6 Kohlenstoffatomen, Alkoxy mit 1 bis 4 Kohlenstoffatomen, Alkylthio mit 1 bis 4 Kohlenstoffatomen, Cycloalkyl mit 5 bis 7 Kohlenstoffatomen oder Phenyl substituiert ist und welche außerdem gegebenenfalls durch eine der nachstehenden Gruppierungen: 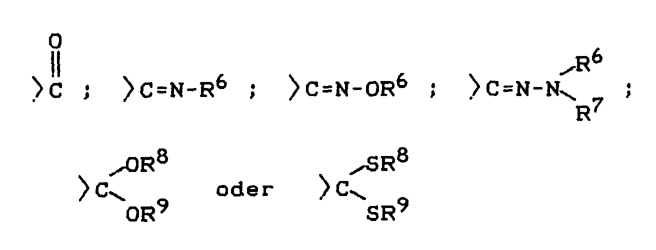 unterbrochen ist,
B für Wasserstoff oder C₁-C₄-Alkyl steht,
X für C₁-C₄-Alkyl, Halogen oder C₁-C₄-Alkoxy steht,
Y für Wasserstoff, C₁-C₄-Alkyl, Halogen, C₁-C₄-Alkoxy oder C₁-C₂-Halogenalkyl steht,
Z für C₁-C₄-Alkyl, Halogen oder C₁-C₄-Alkoxy steht,
n für eine Zahl 0, 1, 2 oder 3 steht,
G für Wasserstoff (a) oder für die Gruppen 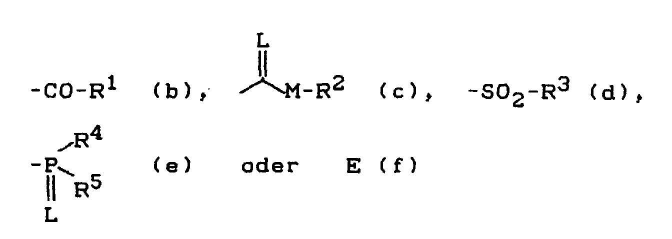
steht,
in welchen
E für ein Metallionäquivalent oder ein Ammoniumion steht,
L und M jeweils für Sauerstoff und/oder Schwefel
R¹ für gegebenenfalls durch Halogen substituiertes C₁-C₁₆-Alkyl, C₂-C₁₆-Alkenyl, C₁-C₆-Alkoxy-C₂-C₆-alkyl, C₁-C₁₆-Alkylthio-C₂-C₆-alkyl, C₁-C₆-Polyalkoxy-C₂-C₆-alkyl oder Cycloalkyl mit 3 bis 7 Ringatomen, das durch 1-2 Sauerstoff-und/oder Schwefelatome unterbrochen sein kann, steht,
für gegebenenfalls durch Halogen, Nitro, C₁-C₄-Alkyl, C₁-C₄-Alkoxy, C₁-C₃-Halogenalkyl, C₁-C₃-Halogenalkoxy substituiertes Phenyl,
für gegebenenfalls durch Halogen, C₁-C₄-Alkyl, C₁-C₄-Alkoxy, C₁-C₃-Halogenalkyl, C₁-C₃-Halogenalkoxy substituiertes Phenyl-C₁-C₄-alkyl steht,
für gegebenenfalls durch Halogen und/oder C₁-C₆-Alkyl substituiertes Hetaryl steht,
für gegebenenfalls durch Halogen und C₁-C₄-Alkyl substituiertes Phenoxy-C₁-C₅-alkyl steht,
für gegebenenfalls durch Halogen, Amino und C₁-C₄-Alkyl substituiertes Hetaryloxy-C₁-C₅-alkyl steht,
R² für gegebenenfalls durch Halogen substituiertes C₁-C₁₆-Alkyl, C₂-C₁₆-Alkenyl, C₁-C₁₆-Alkoxy-C₂-C₆-alkyl, C₁-C₆-Polyalkoxy-C₂-C₆-alkyl steht,
für gegebenenfalls durch Halogen, Nitro, C₁-C₄-Alkyl, C₁-C₃-Alkoxv, C₁-C₃-Halogenalkyl substituiertes Phenyl oder Benzyl steht,
R³, R⁴ und R⁵ unabhängig voneinander für gegebenenfalls durch Halogen substituiertes C₁-C₆-Alkyl, C₁-C₆-Alkoxy, C₁-C₆-Alkylamino, Di-(C₁-C₆)-alkylamino, C₁-C₆-Alkylthio, C₃-C₄-Alkenylthio, C₂-C₄-Alkinylthio, C₃-C₆-Cycloalkylthio, für gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl, C₁-C₃-Halogenalkyl substituiertes Phenyl, Phenoxy oder Phenylthio stehen,
R⁶ und R⁷ unabhängig voneinander für Wasserstoff, gegebenenfalls durch Halogen substituiertes C₁-C₂₀-Alkyl, C₁-C₂₀-Alkoxy, C₂-C₈-Alkenyl, C₁-C₂₀-Alkoxy-C₁-C₂₀-alkyl, für gegebenenfalls durch Halogen, C₁-C₅-Halogenalkyl, C₁-C₅-Alkyl oder C₁-C₅-Alkoxy substituiertes Phenyl, für gegebenenfalls durch Halogen, C₁-C₅-Alkyl, C₁-C₅-Halogenalkyl oder C₁-C₅-Alkoxy substituiertes Benzyl steht,
R⁸ und R⁹ unabhängig voneinander für Wasserstoff oder Alkyl mit 1 bis 4 Kohlenstoffatomen stehen oder
R⁸ und R⁹ zusammen für einen Alkandiylrest mit 2 oder 3 Kohlenstoffatomen stehen.

3. Substituierte bicyclische 3-Aryl-pyrrolidin-2,4-dion-Derivate der Formel (I) gemäß Anspruch 1, dadurch gekennzeichnet, daß
A und Q gemeinsam für eine jeweils einfach bis dreifach, gleich oder verschieden substituierte Alkandiyl-, Alkendiyl- oder Alkendienylgruppierung mit 3 bis 4 Kohlenstoffatomen stehen, welche durch Fluor, Chlor, Brom, Hydroxy, Mercapto, jeweils gegebenenfalls durch Fluor, Chlor, Brom substituiertes Alkyl mit 1 bis 6 Kohlenstoffatomen, Alkylcarbonyloxy mit 1 bis 4 Kohlenstoffatomen, Alkoxy mit 1 oder 2 Kohlenstoffatomen, Alkylthio mit 1 oder 2 Kohlenstoffatomen oder Cycloalkyl mit 5 oder 6 Kohlenstoffatomen substituiert ist und welche außerdem gegebenenfalls durch eine der nachstehenden Gruppierungen: 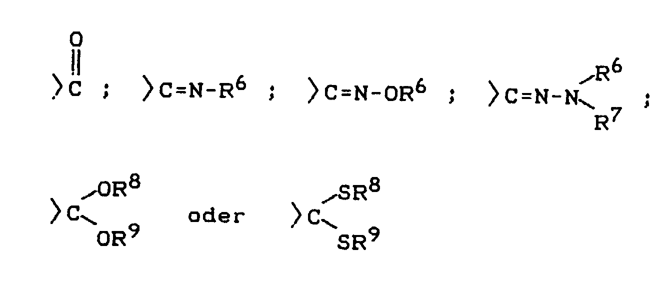 unterbrochen ist,
B für Wasserstoff, Methyl oder Ethyl steht,
X für Methyl, Ethyl, Propyl, 2-Propyl, Fluor, Chlor, Brom, Methoxy oder Ethoxy steht,
Y für Wasserstoff, Methyl, Ethyl, Propyl, i-Propyl, Butyl, i-Butyl, tert.-Butyl, Fluor, Chlor, Brom, Methoxy, Ethoxy oder Trifluormethyl steht,
Z für Methyl, Ethyl, Propyl, i-Propyl, Butyl, i-Butyl, tert.-Butyl, Fluor, Chlor, Brom, Methoxy oder Ethoxy steht,
n für eine Zahl 0, 1, 2 oder 3 steht,
G für Wasserstoff (a) oder für die Gruppen 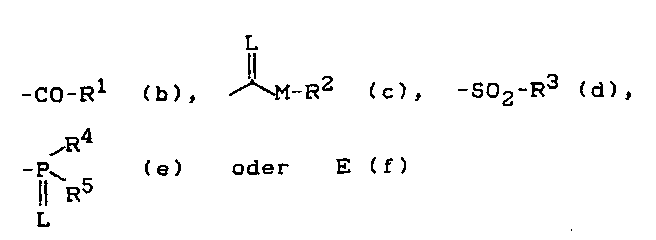
steht,
in welchen
E für ein Metallionäquivalent oder ein Ammoniumion steht,
L und M jeweils für Sauerstoff und/oder Schwefel steht,
R¹ für gegebenenfalls durch Fluor oder Chlor substituiertes C₁-C₁₄-Alkyl, C₂-C₁₄-Alkenyl, C₁-C₄-Alkoxy-C₂-C₆-alkyl, C₁-C₄-Alkylthio-C₂-C₆-alkyl, C₁-C₄-Polyalkoxy-C₂-C₄-alkyl oder Cycloalkyl mit 3 bis 6 Ringatomen, das durch 1 bis 2 Sauerstoff-und/oder Schwefelatome unterbrochen sein kann, steht,
für gegebenenfalls durch Fluor, Chlor, Brom, Methyl, Ethyl, Propyl, i-Propyl, Methoxy, Ethoxy, Trifluormethyl, Trifluormethoxy, Nitro substituiertes Phenyl steht,
für gegebenenfalls durch Fluor, Chlor, Brom, Methyl, Ethyl, Propyl, i-Propyl, Methoxy, Ethoxy, Trifluormethyl, Trifluormethoxy substituiertes Phenyl-C₁-C₃-alkyl steht,
für gegebenenfalls durch Fluor, Chlor, Brom, Methyl, Ethyl substituiertes Pyridyl, Pyrimidyl, Thiazolyl und Pyrazolyl steht,
für gegebenenfalls durch Fluor, Chlor, Methyl, Ethyl substituiertes Phenoxy-C₁-C₄-alkyl steht,
für gegebenenfalls durch Fluor, Chlor, Amino, Methyl, Ethyl substituiertes Pyridyloxy-C₁-C₄-alkyl, Pyrimidyloxy-C₁-C₄-alkyl und Thiazolyloxy-C₁-C₄-alkyl steht,
R² für gegebenenfalls durch Fluor oder Chlor substituiertes C₁-C₁₄-Alkyl, C₂-C₁₄-Alkenyl, C₁-C₄-Alkoxy-C₂-C₆-alkyl, C₁-C₄-Polyalkoxy-C₂-C₆-alkyl steht
oder für gegebenenfalls durch Fluor, Chlor, Nitro, Methyl, Ethyl, Propyl, i-Propyl, Methoxy, Ethoxy, Trifluormethyl substituiertes Phenyl oder Benzyl steht,
R³, R⁴ und R⁵ unabhängig voneinander für gegebenenfalls durch Fluor oder Chlor substituiertes C₁-C₄-Alkyl, C₁-C₄-Alkoxy, C₁-C₄-Alkylamino, Di-(C₁-C₄)-alkylamino, C₁-C₄-Alkylthio, für gegebenenfalls durch Fluor, Chlor, Brom, Nitro, Cyano, C₁-C₂-Alkoxy, C₁-C₄-Fluoralkoxy, C₁-C₂-Chloralkoxy, C₁-C₂-Alkylthio, C₁-C₂-Fluoralkylthio, C₁-C₂-Chloralkylthio, C₁-C₃-Alkyl substituiertes Phenyl, Phenoxy oder Phenylthio stehen,
R⁶ und R⁷ unabhängig voneinander für gegebenenfalls durch Fluor, Chlor, Brom substituiertes C₁-C₁₀-Alkyl, C₁-C₁₀-Alkoxy, C₁-C₁₀-Alkoxy-(C₁-C₁₀)-alkyl, für gegebenenfalls durch Fluor, Chlor, Brom, C₁-C₂₀-Halogenalkyl, C₁-C₂₀-Alkyl oder C₁-C₄-Alkoxy substitiertes Phenyl, für gegebenenfalls durch Fluor, Chlor, Brom, C₁-C₄-Alkyl, C₁-C₄-Halogenalkyl oder C₁-C₄-Alkoxy substituiertes Benzyl steht,
R⁸ und R⁹ unabhängig voneinander für Wasserstoff oder Alkyl mit 1 bis 4 Kohlenstoffatomen stehen oder
R⁸ und R⁹ zusammen für einen Alkandiylrest mit 2 oder 3 Kohlenstoffatomen stehen.

4. Verfahren zur Herstellung von substituierten bicyclischen 3-Aryl-pyrrolidin-2,4-dion-Derivaten der Formel (I) gemäß Anspruch 1,
dadurch gekennzeichnet,
(Aα) daß man zum Erhalt von 3-Aryl-pyrrolidin-2,4-dionen bzw. deren Enolen der Formel (Ia) 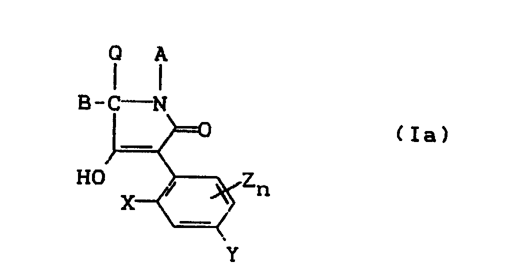 in welcher
A, B, Q, X, Y, Z und n die oben angegebene Bedeutung haben,
N-Acylaminosäureester der Formel (II) 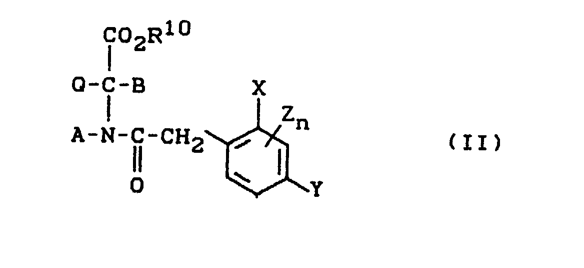 in welcher
A, B, Q, X, Y, Z und n die oben angegebene Bedeutung haben,
und
R¹⁰ für Alkyl steht,
in Gegenwart eines Verdünnungsmittels und in Gegenwart einer Base intramolekular kondensiert
oder
(A-β) daß man die nach Verfahren (Aα) erhaltenen Verbindungen der Formel (Ia-a) 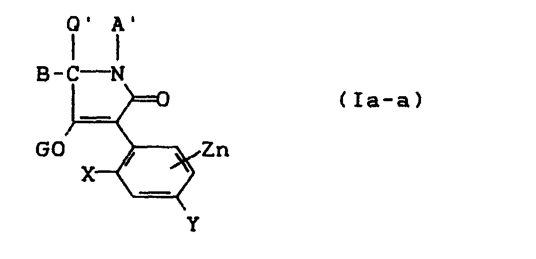 in welcher
B, X, Y, Z, n und G die oben angegebene Bedeutung haben und
A' und Q' gemeinsam für eine durch Hydroxy substituierte Alkandiyl-, Alkendiyl, Alkendienyl- oder Alkentrienyl-Gruppierung stehen
entweder an der Hydroxy-Gruppierung nach allgemein üblichen Verfahren in Ester oder Ether umwandelt oder die Hydroxy-Gruppierung in einer ersten Stufe mit Hilfe üblicher Oxidationsmethoden in die entsprechenden Ketone überführt und diese in einer zweiten Stufe nach ebenfalls üblichen Methoden mit Thienen, Alkoholen, Diolen, Mercaptanen oder Dithiolen weiter umsetzt,
oder
(B) daß man zum Erhalt von Verbindungen der Formel (Ib) 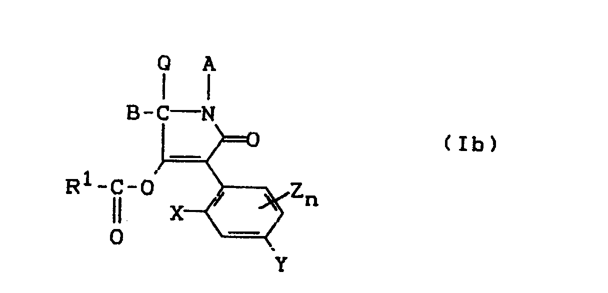 in welcher
A, B, Q, X, Y, Z, R¹ und n die oben angegebene Bedeutung haben,
Verbindungen der Formel (Ia), 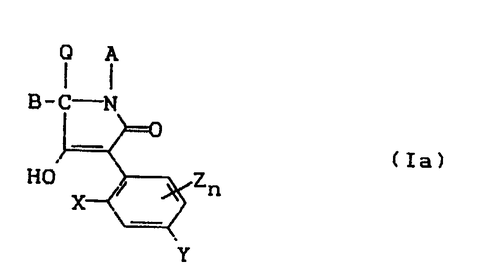 in welcher
A, B, Q, X, Y, Z und n die oben angegebene Bedeutung haben,
α) mit Säurehalogeniden der allgemeinen Formel (III) 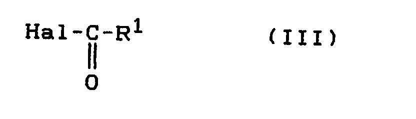 in welcher
R¹ die oben angegebene Bedeutung hat und
Hal für Halogen, insbesondere Chlor und Brom steht,
gegebenenfalls in Gegenwart eines Verdünnungsmittels und gegebenenfalls in Gegenwart eines Säurebindemittels umsetzt
oder
β) mit Carbonsäureanhydriden der allgemeinen Formel (IV)
R¹-CO-O-CO-R¹ (IV)
in welcher
R¹ die oben angegebene Bedeutung hat,
gegebenenfalls in Gegenwart eines Verdünnungsmittels und gegebenenfalls in Gegenwart eines Säurebindemittels,
umsetzt;
oder
(C) daß man zum Erhalt von Verbindungen der Formel (Ic-1) 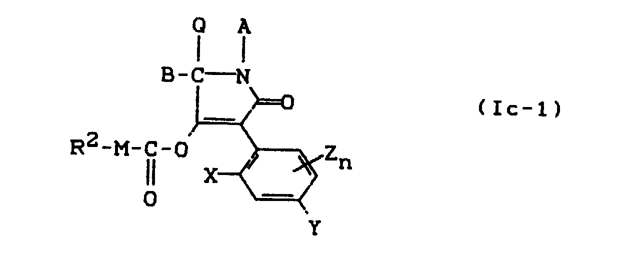 in welcher
A, B, Q, X, Y, Z, R² und n die oben angegebene Bedeutung haben,
und
M für Sauerstoff oder Schwefel steht,
Verbindungen der Formel (Ia) 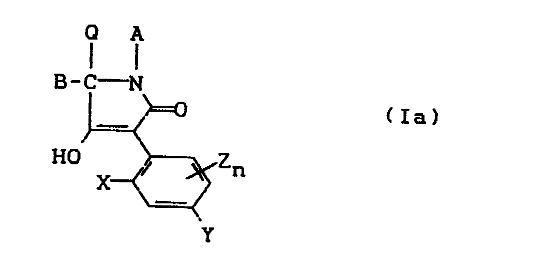 in welcher
A, B, Q, X, Y, Z und n die oben angegebene Bedeutung haben,
mit Chlorameisensäureester oder Chlorameisensäurethiolester der allgemeinen Formel (V)
R²-M-CO-Cl (V)
in welcher
R² und M die oben angegebene Bedeutung haben,
gegebenenfalls in Gegenwart eines Verdünnungsmittels und gegebenenfalls in Gegenwart eines Säurebindemittels umsetzt;
oder
(D) daß man zum Erhalt von Verbindungen der Formel (Ic-2) 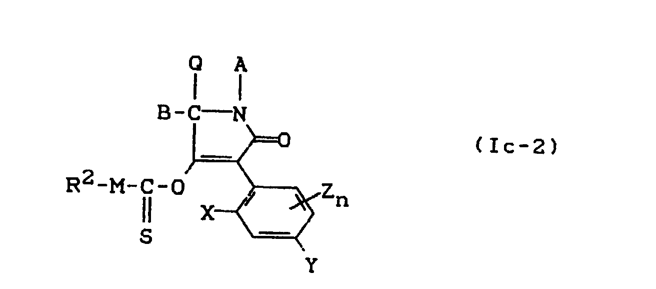 in welcher
A, B, Q, R², X, Y, Z und n die oben angegebene Bedeutung haben,
und
M für Sauerstoff oder Schwefel steht,
Verbindungen der Formel (Ia) 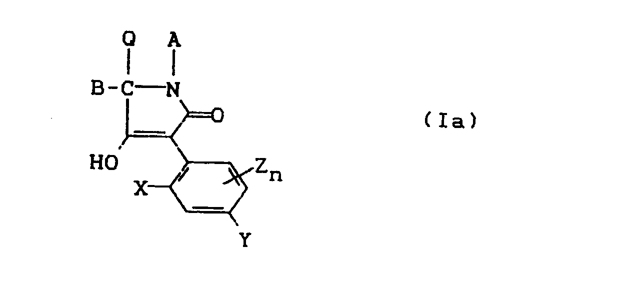 in welcher
A, B, Q, X, Y, Z und n die oben angegebene Bedeutung haben,
α) mit Chlormonothioameisensäureestern oder Chlordithioameisensäureestern der allgemeinen Formel (VI) 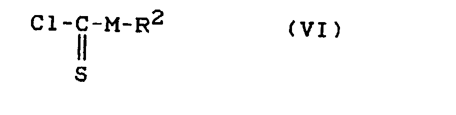 in welcher
M und R² die oben angegebene Bedeutung haben,
gegebenenfalls in Gegenwart eines Verdünnungsmittels und gegebenenfalls in Gegenwart eines Säurebindemittels umsetzt,
oder
β) mit Schwefelkohlenstoff und anschließend mit Alkylhalogeniden der allgemeinen Formel (VII)
R²-Hal (VII)
in welcher
R² die oben angegebene Bedeutung hat
und
Hal für Chlor, Brom, Jod steht,
umsetzt;
oder
(E) daß man zum Erhalt von Verbindungen der Formel (Id) 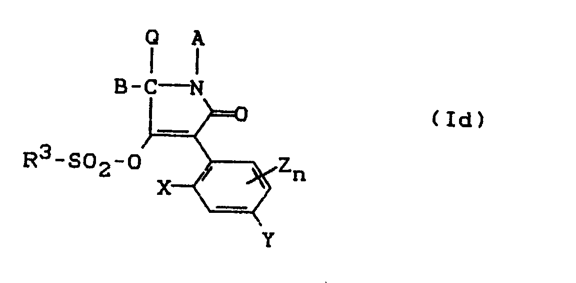 in welcher
A, B, Q, X, Y, Z, R³ und n die oben angegebene Bedeutung haben,
Verbindungen der Formel (Ia) 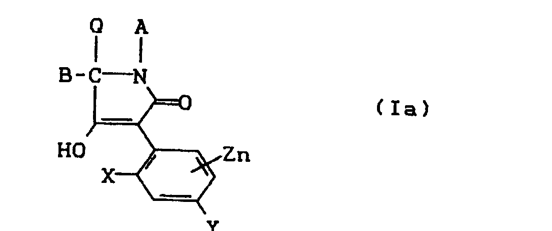 in welcher
A, B, Q, X, Y, Z und n die oben angegebene Bedeutung haben,
mit Sulfonsäurechloriden der allgemeinen Formel (VIII)
R³-SO₂-Cl (VIII)
in welcher
R³ die oben angegebene Bedeutung hat,
gegebenenfalls in Gegenwart eines Verdünnungsmittels und gegebenenfalls in Gegenwart eines Säurebindemittels,
umsetzt;
oder
(F) daß man zum Erhalt von 3-Aryl-pyrrolidin-2,4-dionen der Formel (Ie) 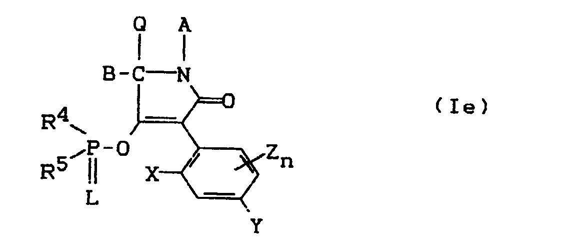 in welcher
A, B, Q, L, X, Y, Z, R⁴, R⁵ und n die oben angegebene Bedeutung haben,
3-Aryl-pyrrolidin-2,4-dione der Formel (Ia) bzw. deren Enole 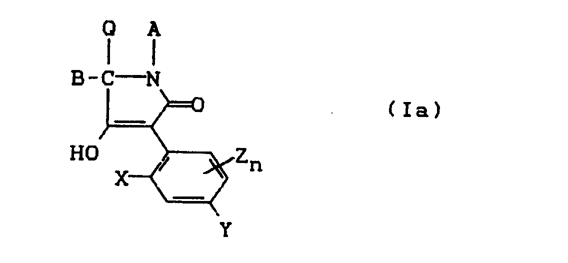 in welcher
A, B, Q, X, Y, Z und n die oben angegebene Bedeutung haben,
mit Phosphorverbindungen der allgemeinen Formel (IX) 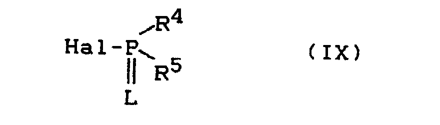 in welcher
L, R⁴ und R⁵ die oben angegebene Bedeutung haben
und
Hal für Halogen, insbesondere Chlor und Brom steht,
umsetzt;
oder
(G) daß man zum Erhalt von Verbindungen der Formel (If) 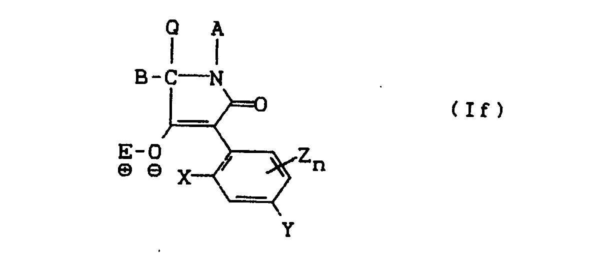 in welcher
A, B, Q, X, Y, Z und n die oben angegebene Bedeutung haben,
und
E für ein Metallionäquivalent oder für ein Ammoniumion steht,
Verbindungen der Formel (Ia) 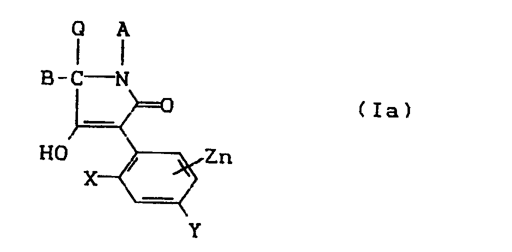 in welcher
A, B, Q, X, Y, Z und n die oben angegebene Bedeutung haben,
mit Metallhydroxiden oder Aminen der allgemeinen Formeln (X) und (XI) 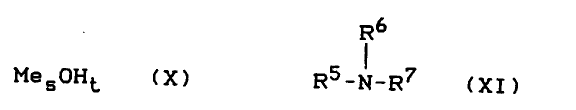 in welchen
Me für ein- oder zweiwertige Metallionen,
s und t für die Zahl 1 und 2 und
R⁵, R⁶ und R⁷ unabhängig voneinander für Wasserstoff und Alkyl
stehen,
gegebenenfalls in Gegenwart eines Verdünnungsmittels, umsetzt.

5. Schädlingsbekämpfungsmittel gekennzeichnet durch einen Gehalt an mindestens einem substituierten bicyclischen 3-Arylpyrrolidin-2,4-dion-Derivat der Formel (I).

6. Verfahren zur Bekämpfung von tierischen Schädlingen, dadurch gekennzeichnet, daß man substituierte bicyclische 3-Arylpyrrolidin-2,4-dion-Derivate der Formel (I) auf tierische Schädlinge und/oder deren Lebensraum einwirken läßt.

7. Verwendung von substituierten bicyclischen 3-Arylpyrrolidin-2,4-dion-Derivaten der Formel (I) zur Bekämpfung von tierischen Schädlingen.

8. Verfahren zur Herstellung von Schädlingsbekämpfungsmitteln, dadurch gekennzeichnet, daß man substituierte bicyclische 3-Arylpyrrolidin-2,4-dion-Derivate der Formel (I), mit Streckmitteln und/oder oberflächenaktiven Mitteln vermischt.

9. Herbizide Mittel, gekennzeichnet durch einen Gehalt an mindestens einem substituierten bicyclischen 3-Arylpyrrolidin-2,4-dion-Derivat der Formel (I).

10. Verfahren zur Bekämpfung von Unkräutern, dadurch gekennzeichnet, daß man substituierte bicyclische 3-Arylpyrrolidin-2,4-dion-Derivate der Formel (I) auf Unkräuter und/oder deren Lebensraum einwirken läßt.

11. Verwendung von substituierten bicyclischen 3-Arylpyrrolidin-2,4-dion-Derivaten der Formel (I) zur Bekämpfung von Unkräutern.

12. Verfahren zur Herstellung von herbiziden Mitteln, dadurch gekennzeichnet, daß man substituierte bicyclische 3-Arylpyrrolidin-2,4-dion-Derivate der Formel (I) mit Streckmitteln und/oder oberflächenaktiven Mitteln vermischt.

## Patentansprüche (Patentansprüche für folgende(n) Vertragsstaat(en): ES)

1. Verfahren zur Herstellung von substituierten bicyclischen 3-Aryl-pyrrolidin-2,4-dion-Derivaten der Formel (I) 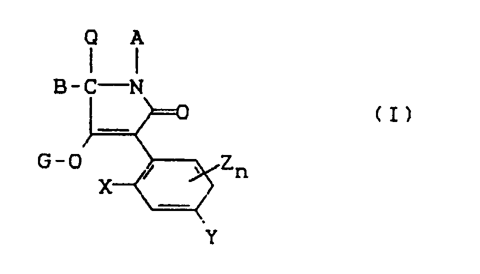 in welcher
A und Q gemeinsam für eine jeweils einfach bis dreifach, gleich oder verschieden substituierte Alkandiyl-, Alkendiyl-, Alkendienyl- oder Alkentrienylgruppierung mit 3 bis 6 Kohlenstoffatomen stehen, welche jeweils durch Halogen, Hydroxy, Mercapto, jeweils gegebenenfalls durch Halogen substituiertes Alkyl mit 1 bis 10 Kohlenstoffatomen, Alkylcarbonyloxy mit 1 bis 8 Kohlenstoffatomen, Alkoxy mit 1 bis 6 Kohlenstoffatomen, Alkylthio mit 1 bis 6 Kohlenstoffatomen, Cycloalkyl mit 3 bis 7 Kohlenstoffatomen oder Aryl mit 6 bis 10 Kohlenstoffatomen substituiert ist und welche außerdem gegebenenfalls durch eine der nachstehenden Gruppierungen 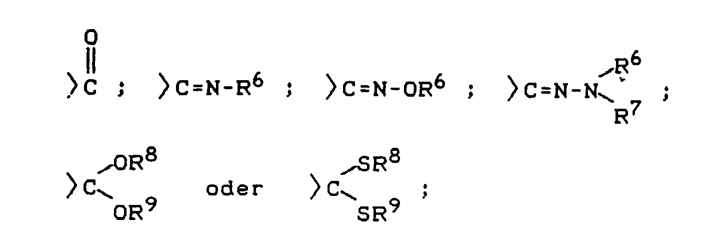 unterbrochen ist,
B für Wasserstoff oder Alkyl mit 1 bis 6 Kohlenstoffatomen steht,
X für C₁-C₆-Alkyl, Halogen oder C₁-C₆-Alkoxy steht,
Y für Wasserstoff, C₁-C₆-Alkyl, Halogen, C₁-C₆-Alkoxy oder C₁-C₃-Halogenalkyl steht,
Z für C₁-C₆-Alkyl, Halogen oder C₁-C₆-Alkoxy steht,
n für eine Zahl 0, 1, 2 oder 3 steht.
G für Wasserstoff (a) oder für die Gruppen 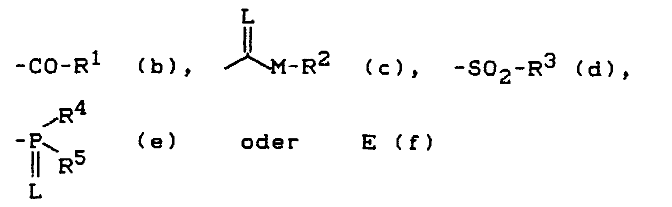 steht,
in welchen
E für ein Metallionäquivalent oder ein Ammoniumion steht,
L und M für Sauerstoff und/oder Schwefel steht,
R¹ für gegebenenfalls durch Halogen substituiertes C₁-C₂₀-Alkyl, C₂-C₂₀-Alkenyl, C₁-C₈-Alkoxy-C₂-C₈-alkyl, C₁-C₆-Alkylthio-C₂-C₈-alkyl, C₁-C₈-Polyalkoxy-C₂-C₆-alkyl oder Cycloalkyl mit 3 bis 8 Ringatomen, das durch Sauerstoff-und/oder Schwefelatome unterbrochen sein kann, steht,
für gegebenenfalls durch Halogen, Nitro, C₁-C₂₀-Alkyl, C₂-C₂₀-Alkenyl, C₁-C₆-Halogenalkyl, C₁-C₆-Halogenalkoxy substituiertes Phenyl,
für gegebenenfalls durch Halogen, C₁-C₆-Alkyl, C₁-C₆-Alkoxy, C₁-C₆-Halogenalkyl, C₁-C₆-Halogenalkoxy substituiertes Phenyl-C₁-C₆-alkyl steht,
für gegebenenfalls durch Halogen und/oder C₁-C₆-Alkyl substituiertes Hetaryl steht,
für gegebenenfalls durch Halogen und C₁-C₆-Alkyl substituiertes Phenoxy-C₁-C₆-alkyl steht,
für gegebenenfalls durch Halogen, Amino und C₁-C₆-Alkyl substituiertes Hetaryloxy-C₁-C₆-Alkyl steht,
R² für gegebenenfalls durch Halogen substituiertes C₁-C₂₀-Alkyl, C₂-C₂₀-Alkenyl, C₁-C₈-Alkoxy-C₂-C₈-alkyl, C₁-C₈-Polyalkoxy-C₂-C₈-alkyl steht,
für gegebenenfalls durch Halogen, Nitro, C₁-C₂₀-Alkyl, C₁-C₆-Alkoxy, C₁-C₆-Halogenalkyl substituiertes Phenyl oder Benzyl steht,
R³, R⁴ und R⁵ unabhängig voneinander für gegebenenfalls durch Halogen substituiertes C₁-C₈-Alkyl, C₁-C₈-Alkoxy, C₁-C₈-Alkylamino, Di-(C₁-C₈)-alkylamino, C₁-C₈-Alkylthio, C₂-C₅-Alkenylthio, C₂-C₅-Alkinylthio, C₃-C₇-Cycloalkylthio, für gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl, C₁-C₄-Halogenalkyl substituiertes Phenyl, Phenoxy oder Phenylthio stehen,
R⁶ und R⁷ unabhängig voneinander für Wasserstoff, gegebenenfalls durch Halogen substituiertes C₁-C₂₀-Alkyl, C₁-C₂₀-Alkoxy, C₂-C₈-Alkenyl, C₁-C₂₀-Alkoxy-C₁-C₂₀-alkyl, für gegebenenfalls durch Halogen, C₁-C₂₀-Halogenalkyl, C₁-C₂₀-Alkyl oder C₁-C₂₀-Alkoxy substituiertes Phenyl, für gegebenenfalls durch Halogen, C₁-C₂₀-Alkyl, C₁-C₂₀-Halogenalkyl oder C₁-C₂₀-Alkoxy substituiertes Benzyl steht oder zusammen für einen gegebenenfalls durch Sauerstoff unterbrochenen C₂-C₆-Alkylenring stehen,
R⁸ und R⁹ unabhängig voneinander für Wasserstoff oder Alkyl mit 1 bis 6 Kohlenstoffatomen stehen oder
R⁸ und R⁹ zusammen für einen Alkandiylrest mit 2 bis 4 Kohlenstoffatomen stehen,
dadurch gekennzeichnet,
(Aα) daß man zum Erhalt von 3-Aryl-pyrrolidin-2,4-dionen bzw. deren Enolen der Formel (Ia) 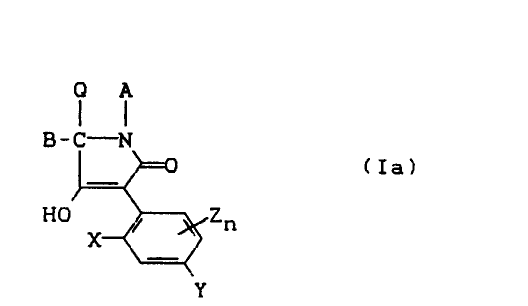 in welcher
A, B, Q, X, Y, Z und n die oben angegebene Bedeutung haben,
N-Acylaminosäureester der Formel (II) 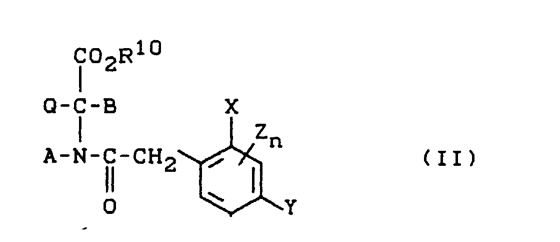 in welcher
A, B, Q, X, Y, Z und n die oben angegebene Bedeutung haben,
und
R¹⁰ für Alkyl steht,
in Gegenwart eines Verdünnungsmittels und in Gegenwart einer Base intramolekular kondensiert
oder
(A-β) daß man die nach Verfahren (Aα) erhaltenen Verbindungen der Formel (Ia-a) 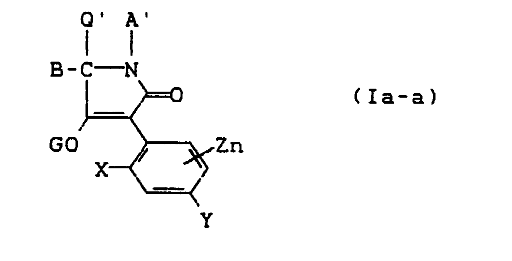 in welcher
B, X, Y, Z, n und G die oben angegebene Bedeutung haben und
A' und Q' gemeinsam für eine durch Hydroxy substituierte Alkandiyl-, Alkendiyl, Alkendienyl- oder Alkentrienyl-Gruppierung stehen
entweder an der Hydroxy-Gruppierung nach allgemein üblichen Verfahren in Ester oder Ether umwandelt oder die Hydroxy-Gruppierung in einer ersten Stufe mit Hilfe üblicher Oxidationsmethoden in die entsprechenden Ketone überführt und diese in einer zweiten Stufe nach ebenfalls üblichen Methoden mit Thienen, Alkoholen, Diolen, Mercaptanen oder Dithiolen weiter umsetzt,
oder
(B) daß man zum Erhalt von Verbindungen der Formel (Ib) 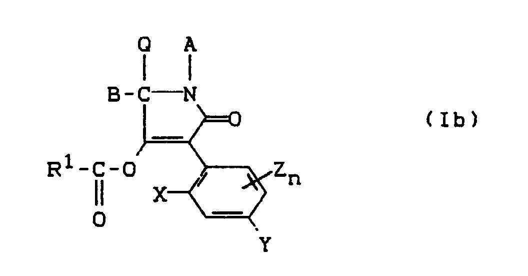 in welcher
A, B, Q, X, Y, Z, R¹ und n die oben angegebene Bedeutung haben,
Verbindungen der Formel (Ia), 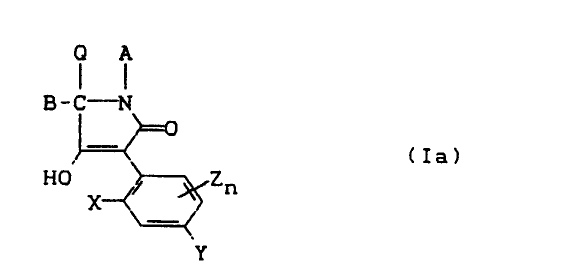 in welcher
A, B, Q, X, Y, Z und n die oben angegebene Bedeutung haben,
α) mit Säurehalogeniden der allgemeinen Formel (III) 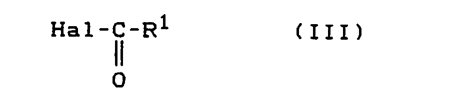 in welcher
R¹ die oben angegebene Bedeutung hat und
Hal für Halogen, insbesondere Chlor und Brom steht,
gegebenenfalls in Gegenwart eines Verdünnungsmittels und gegebenenfalls in Gegenwart eines Säurebindemittels umsetzt
oder
β) mit Carbonsäureanhydriden der allgemeinen Formel (IV)
R¹-CO-O-CO-R¹ (IV)
in welcher
R¹ die oben angegebene Bedeutung hat,
gegebenenfalls in Gegenwart eines Verdünnungsmittels und gegebenenfalls in Gegenwart eines Säurebindemittels.
umsetzt:
oder
(C) daß man zum Erhalt von Verbindungen der Formel (Ic-1) 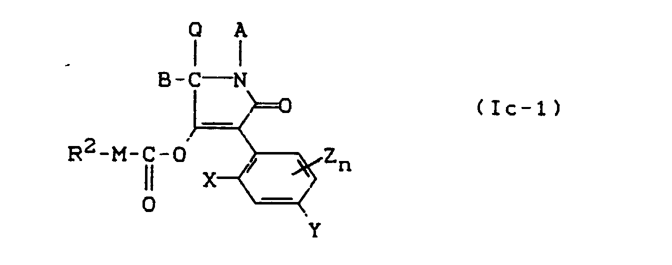 in welcher
A, B, Q, X, Y, Z, R² und n die oben angegebene Bedeutung haben,
und
M für Sauerstoff oder Schwefel steht,
Verbindungen der Formel (Ia) 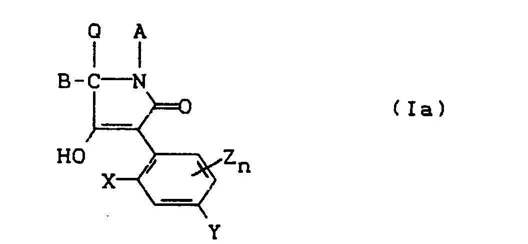 in welcher
A, B, Q, X, Y, Z und n die oben angegebene Bedeutung haben,
mit Chlorameisensäureester oder Chlorameisensäurethiolester der allgemeinen Formel (V)
R²-M-CO-Cl (V)
in welcher
R² und M die oben angegebene Bedeutung haben,
gegebenenfalls in Gegenwart eines Verdünnungsmittels und gegebenenfalls in Gegenwart eines Säurebindemittels umsetzt:
oder
(D) daß man zum Erhalt von Verbindungen der Formel (Ic-2) 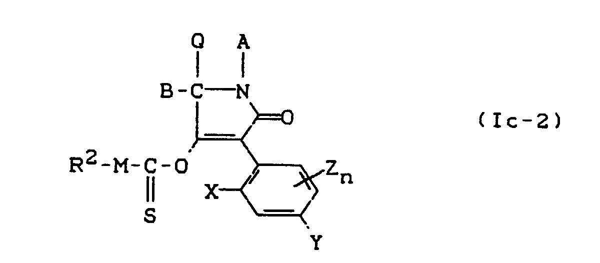 in welcher
A, B, Q, R², X, Y, Z und n die oben angegebene Bedeutung haben,
und
M für Sauerstoff oder Schwefel steht,
Verbindungen der Formel (Ia) 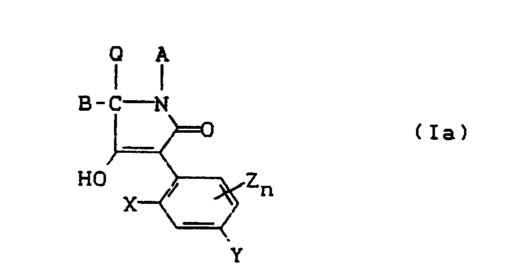 in welcher
A, B, Q, X, Y, Z und n die oben angegebene Bedeutung haben,
α) mit Chlormonothioameisensäureestern oder Chlordithioameisensäureestern der allgemeinen Formel (VI) 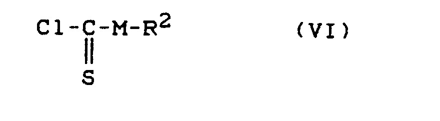 in welcher
M und R² die oben angegebene Bedeutung haben,
gegebenenfalls in Gegenwart eines Verdünnungsmittels und gegebenenfalls in Gegenwart eines Säurebindemittels umsetzt,
oder
β) mit Schwefelkohlenstoff und anschließend mit Alkylhalogeniden der allgemeinen Formel (VII)
R²-Hal (VII)
in welcher
R² die oben angegebene Bedeutung hat
und
Hal für Chlor, Brom, Jod steht,
umsetzt:
oder
(E) daß man zum Erhalt von Verbindungen der Formel (Id) 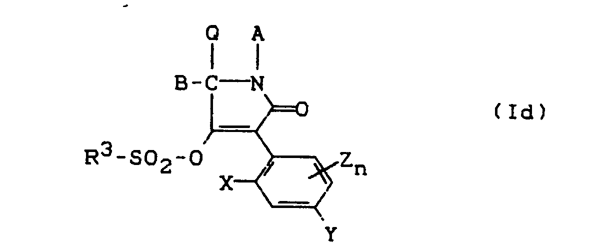 in welcher
A, B, Q, X, Y, Z, R³ und n die oben angegebene Bedeutung haben,
Verbindungen der Formel (Ia) 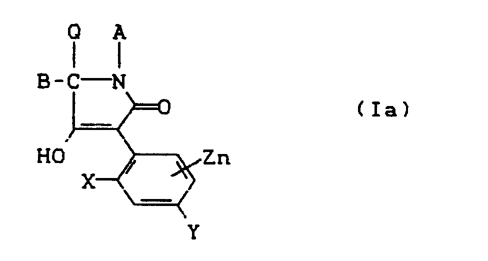 in welcher
A, B, Q, X, Y, Z und n die oben angegebene Bedeutung haben,
mit Sulfonsäurechloriden der allgemeinen Formel (VIII)
R³-SO₂-Cl (VIII)
in welcher
R³ die oben angegebene Bedeutung hat,
gegebenenfalls in Gegenwart eines Verdünnungsmittels und gegebenenfalls in Gegenwart eines Säurebindemittels,
umsetzt:
oder
(F) daß man zum Erhalt von 3-Aryl-pyrrolidin-2,4-dionen der Formel (Ie) 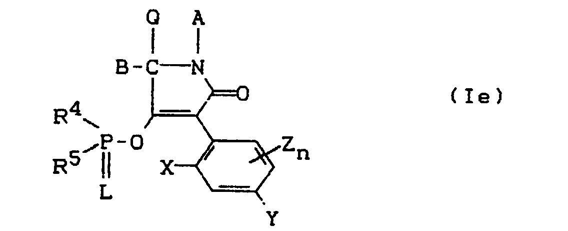 in welcher
A, B, Q, L, X, Y, Z, R⁴, R⁵ und n die oben angegebene Bedeutung haben,
3-Aryl-pyrrolidin-2,4-dione der Formel (Ia) bzw. deren Enole 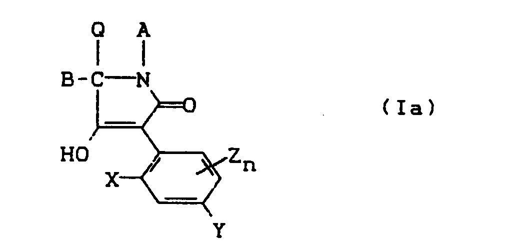 in welcher
A, B, Q, X, Y, Z und n die oben angegebene Bedeutung haben,
mit Phosphorverbindungen der allgemeinen Formel (IX) 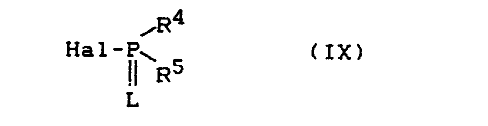 in welcher
L, R⁴ und R⁵ die oben angegebene Bedeutung haben
und
Hal für Halogen, insbesondere Chlor und Brom steht,
umsetzt:
oder
(G) daß man zum Erhalt von Verbindungen der Formel (If) 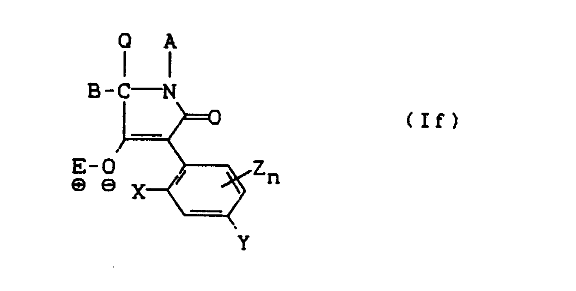 in welcher
A, B, Q, X, Y, Z und n die oben angegebene Bedeutung haben,
und
E für ein Metallionäquivalent oder für ein Ammoniumion steht,
Verbindungen der Formel (Ia) 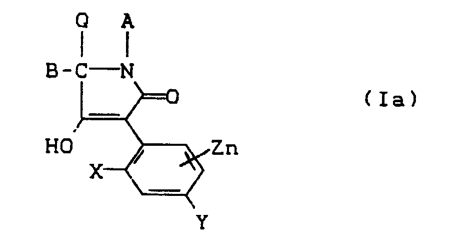 in welcher
A, B, Q, X, Y, Z und n die oben angegebene Bedeutung haben,
mit Metallhydroxiden oder Aminen der allgemeinen Formeln (X) und (XI) 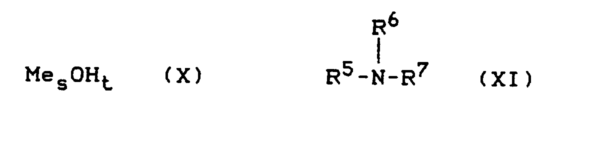 in welchen
Me für ein- oder zweiwertige Metallionen,
s und t für die Zahl 1 und 2 und
R⁵, R⁶ und R⁷ unabhängig voneinander für Wasserstoff und Alkyl
stehen,
gegebenenfalls in Gegenwart eines Verdünnungsmittels, umsetzt.

2. Schädlingsbekämpfungsmittel gekennzeichnet durch einen Gehalt an mindestens einem substituierten bicyclischen 3-Arylpyrrolidin-2,4-dion-Derivat der Formel (I).

3. Verfahren zur Bekämpfung von tierischen Schädlingen, dadurch gekennzeichnet, daß man substituierte bicyclische 3-Arylpyrrolidin-2,4-dion-Derivate der Formel (I) auf tierische Schädlinge und/oder deren Lebensraum einwirken läßt.

4. Verwendung von substituierten bicyclischen 3-Arylpyrrolidin-2,4-dion-Derivaten der Formel (I) zur Bekämpfung von tierischen Schädlingen.

5. Verfahren zur Herstellung von Schädlingsbekämpfungsmitteln, dadurch gekennzeichnet, daß man substituierte bicyclische 3-Arylpyrrolidin-2,4-dion-Derivate der Formel (I); mit Streckmitteln und/oder oberflächenaktiven Mitteln vermischt.

6. Herbizide Mittel, gekennzeichnet durch einen Gehalt an mindestens einem substituierten bicyclischen 3-Arylpyrrolidin-2,4-dion-Derivat der Formel (I).

7. Verfahren zur Bekämpfung von Unkräutern, dadurch gekennzeichnet, daß man substituierte bicyclische 3-Arylpyrrolidin-2,4-dion-Derivate der Formel (I) auf Unkräuter und/oder deren Lebensraum einwirken läßt.

8. Verwendung von substituierten bicyclischen 3-Arylpyrrolidin-2,4-dion-Derivaten der Formel (I) zur Bekämpfung von Unkräutern.

9. Verfahren zur Herstellung von herbiziden Mitteln, dadurch gekennzeichnet, daß man substituierte bicyclische 3-Arylpyrrolidin-2,4-dion-Derivate der Formel (I) mit Streckmitteln und/oder oberflächenaktiven Mitteln vermischt.

## Claims (Claims for the following Contracting State(s): BE, CH, DE, DK, FR, GB, GR, IT, LI, NL)

1. Substituted bicyclic 3-aryl-pyrrolidine-2,4-dione derivatives of the formula (I) 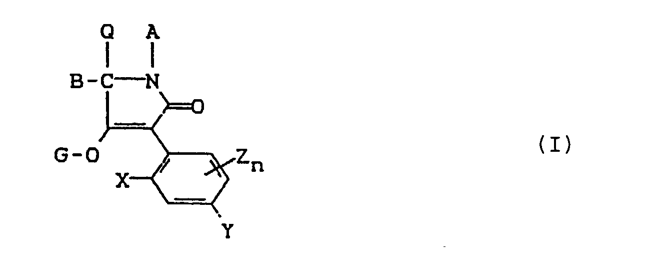
A and Q together represent an alkanediyl, alkenediyl, alkenedienyl or alkenetrienyl group having 3 to 6 carbon atoms, each of these groups being monosubstituted to trisubstituted by identical or different substituents, each of these groups being substituted by halogen, hydroxyl, mercapto, in each case optionally halogen-substituted alkyl having 1 to 10 carbon atoms, alkylcarbonyloxy having 1 to 8 carbon atoms, alkoxy having 1 to 6 carbon atoms, alkylthio having 1 to 6 carbon atoms, cycloalkyl having 3 to 7 carbon atoms or aryl having 6 to 10 carbon atoms, and each of these groups additionally being interrupted, if appropriate, by one of the groups below: 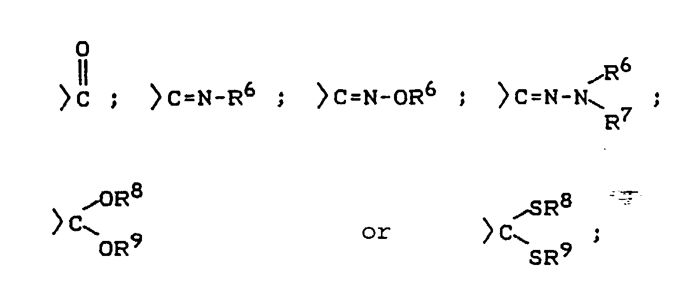
B represents hydrogen or alkyl having 1 to 6 carbon atoms,
X represents C₁-C₆-alkyl, halogen or C₁-C₆-alkoxy,
Y represents hydrogen, C₁-C₆-alkyl, halogen, C₁-C₆-alkoxy or C₁-C₃-halogenoalkyl,
Z represents C₁-C₆-alkyl, halogen or C₁-C₆-alkoxy,
n represents a number 0, 1, 2 or 3,
G represents hydrogen (a) or the groups 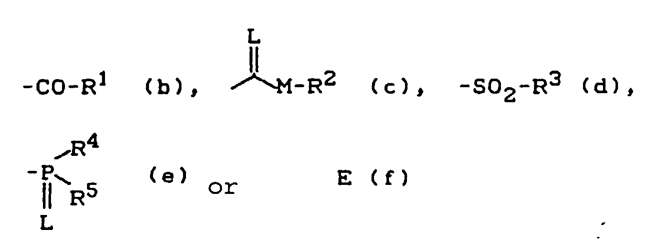
in which
E represents a metal ion equivalent or an ammonium ion,
L and M represent oxygen and/or sulphur,
R¹ represents optionally halogen-substituted C₁-C₂₀-alkyl, C₂-C₂₀-alkenyl, C₁-C₈-alkoxy-C₂-C₈-alkyl, C₁-C₈-alkylthio-C₂-C₈-alkyl, C₁-C₈-polyalkoxy-C₂-C₈-alkyl or cycloalkyl which have 3 to 8 ring atoms and which can be interrupted by oxygen and/or sulphur atoms,
or represents phenyl which is optionally substituted by halogen, nitro, C₁-C₆-alkyl, C₁-C₆-alkoxy, C₁-C₆-halogenoalkyl or C₁-C₆-halogenoalkoxy,
or represents phenyl-C₁-C₆-alkyl which is optionally substituted by halogen, C₁-C₆-alkyl, C₁-C₆-alkoxy, C₁-C₆-halogenoalkyl or C₁-C₆-halogenoalkoxy,
or represents hetaryl which is optionally substituted by halogen and/or C₁-C₆-alkyl,
or represents phenoxy-C₁-C₆-alkyl which is optionally substituted by halogen and C₁-C₆-alkyl,
or represents hetaryloxy-C₁-C₆-alkyl which is optionally substituted by halogen, amino and C₁-C₆-alkyl,
R² represents optionally halogen-substituted C₁-C₂₀-alkyl, C₂-C₂₀-alkenyl, C₁-C₈-alkoxy-C₂-C₈-alkyl or C₁-C₈-polyalkoxy-C₂-C₈-alkyl,
or represents phenyl or benzyl which are optionally substituted by halogen, nitro, C₁-C₆-alkyl, C₁-C₆-alkoxy or C₁-C₆-halogenoalkyl,
R³, R⁴ and R⁵ independently of one another represent optionally halogen-substituted C₁-C₈-alkyl, C₁-C₈-alkoxy, C₁-C₈-alkylamino, di-(C₁-C₈)-alkylamino, C₁-C₈-alkylthio, C₂-C₅-al kenylthio, C₂-C₅-alkinylthio or C₃-C₇-cycloalkylthio, or represent phenyl, phenoxy or phenylthio which are optionally substituted by halogen, nitro, cyano, C₁-C₄-alkoxy, C₁-C₄-halogenoalkoxy, C₁-C₄-alkylthio, C₁-C₄-halogenoalkylthio, C₁-C₄- alkyl or C₁-C₄-halogenoalkyl,
R⁶ and R⁷ independently of one another represent hydrogen, optionally halogen-substituted C₁-C₂₀-alkyl, C₁-C₂₀-alkoxy, C₂-C₈-alkenyl or C₁-C₂₀-alkoxy-C₁-C₂₀-alkyl, or represent phenyl which is optionally substituted by halogen, C₁-C₂₀-halogenoalkyl, C₁-C₂₀-alkyl or C₁-C₂₀-alkoxy, or represent benzyl which is optionally substituted by halogen, C₁-C₂₀-alkyl, C₁-C₂₀-halogenoalkyl or C₁-C₂₀- alkoxy, or together represent a C₂-C₆- alkylene ring which is optionally interrupted by oxygen,
R⁸ and R⁹ independently of one another represent hydrogen or alkyl having 1 to 6 carbon atoms, or
R⁸ and R⁹ together represent an alkanediyl radical having 2 to 4 carbon atoms.

2. Substituted bicyclic 3-aryl-pyrrolidine-2,4-dione derivatives of the formula (I) according to Claim 1, characterised in that
A and Q together represent an alkanediyl, alkenediyl or alkenedienyl group having 3 to 5 carbon atoms, each of these groups being monosubstituted to trisubstituted by identical or different substituents, each of these groups being substituted by fluorine, chlorine, bromine, hydroxyl, mercapto, in each case optionally halogen-substituted alkyl having 1 to 8 carbon atoms, alkylcarbonyloxy having 1 to 6 carbon atoms, alkoxy having 1 to 4 carbon atoms, alkylthio having 1 to 4 carbon atoms, cycloalkyl having 5 to 7 carbon atoms or phenyl, and each of these groups additionally being interrupted, if appropriate, by one of the groups below 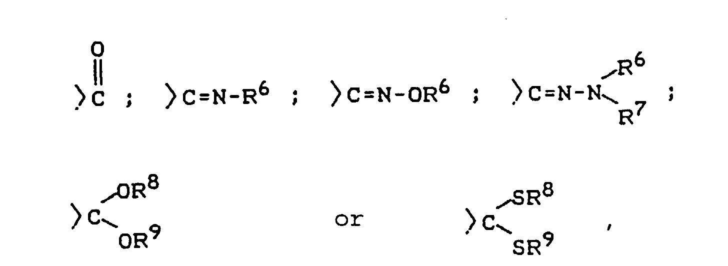
B represents hydrogen or C₁-C₄-alkyl,
X represents C₁-C₄-alkyl, halogen or C₁-C₄-alkoxy,
Y represents hydrogen, C₁-C₄-alkyl, halogen, C₁-C₄-alkoxy or C₁-C₂-halogenoalkyl,
Z represents C₁-C₄-alkyl, halogen or C₁-C₄-alkoxy,
n represents a number 0, 1, 2 or 3,
G represents hydrogen a) or the groups 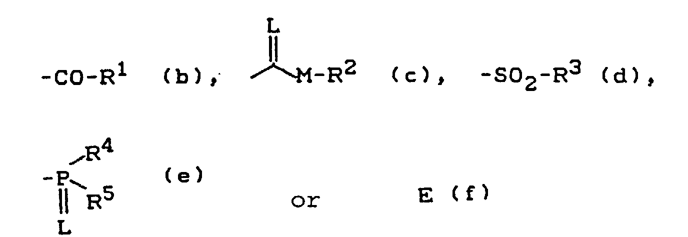
in which
E represents a metal ion equivalent or an ammonium ion,
L and M in each case represent oxygen and/or sulphur
R¹ represents optionally halogen-substituted C₁-C₁₆-alkyl, C₂-C₁₆-alkenyl, C₁-C₆-alkoxy-C₂-C₆-alkyl, C₁-C₁₆-alkylthio-C₂-C₆-alkyl, C₁-C₆-polyalkoxy-C₂-C₆-alkyl or cycloalkyl which have 3 to 7 ring atoms and which can be interrupted by 1-2 oxygen and/or sulphur atoms,
or represents phenyl which is optionally substituted by halogen, nitro, C₁-C₄-alkyl, C₁-C₄-alkoxy, C₁-C₃-halogenoalkyl or C₁-C₃-halogenoalkoxy,
or represents phenyl-C₁-C₄-alkyl which is optionally substituted by halogen, C₁-C₄-alkyl, C₁-C₄-alkoxy, C₁-C₃-halogenoalkyl or C₁-C₃-halogenoalkoxy,
or represents hetaryl which is optionally substituted by halogen and/or C₁-C₆-alkyl,
or represents phenoxy-C₁-C₅-alkyl which is optionally substituted by halogen- and C₁-C₄-alkyl,
or represents hetaryloxy-C₁-C₅-alkyl which is optionally substituted by halogen, amino and C₁-C₄-alkyl,
R² represents optionally halogen-substituted C₁-C₁₆-alkyl, C₂-C₁₆-alkenyl, C₁-C₁₆-alkoxy-C₂-C₆-alkyl or C₁-C₆-polyalkoxy-C₂-C₆-alkyl,
or represents phenyl or benzyl which are optionally substituted by halogen, nitro, C₁-C₄-alkyl, C₁-C₃-alkoxy or C₁-C₃-halogenoalkyl,
R³, R⁴ and R⁵ independently of one another represent optionally halogen-substituted C₁-C₆-alkyl, C₁-C₆-alkoxy, C₁-C₆-alkylamino, di-(C₁-C₆) -alkylamino, C₁-C₆-alkylthio, C₃-C₄-alkenylthio, C₂-C₄-alkinylthio or C₃-C₆-cycloalkylthio, or represent phenyl, phenoxy or phenylthio which are optionally substituted by fluorine, chlorine, bromine, nitro, cyano, C₁-C₃-alkoxy, C₁-C₃-halogenoalkoxy, C₁-C₃-alkylthio, C₁-C₃-halogenoalkylthio, C₁-C₃-alkyl or C₁-C₃-halogenoalkyl,
R⁶ and R⁷ independently of one another represent hydrogen, optionally halogen-substituted C₁-C₂₀-alkyl, C₁-C₂₀-alkoxy, C₂-C₈-alkenyl or C₁-C₂₀-alkoxy-C₁-C₂₀-alkyl, or represent phenyl which is optionally substituted by halogen, C₁-C₅-halogenoalkyl, C₁-C₅-alkyl or C₁-C₅-alkoxy, or represent benzyl which is optionally substituted by halogen, C₁-C₅-alkyl, C₁-C₅-halogenoalkyl or C₁-C₅-alkoxy,
R⁸ and R⁹ independently of one another represent hydrogen or alkyl having 1 to 4 carbon atoms, or
R⁸ and R⁹ together represent an alkanediyl radical having 2 or 3 carbon atoms.

3. Substituted bicyclic 3-aryl-pyrrolidine-2,4-dione derivatives of the formula (I) according to Claim 1, characterised in that
A and Q together represent an alkanediyl, alkenediyl or alkenedienyl group having 3 to 4 carbon atoms, each of these groups being monosubstituted to trisubstituted by identical or different substituents, each of these groups being substituted by fluorine, chlorine, bromine, hydroxyl, mercapto, in each case optionally fluorine-, chlorine- or bromine-substituted alkyl having 1 to 6 carbon atoms, alkylcarbonyloxy having 1 to 4 carbon atoms, alkoxy having 1 or 2 carbon atoms, alkylthio having 1 or 2 carbon atoms or cycloalkyl having 5 or 6 carbon atoms, and each of these groups additionally being interrupted, if appropriate, by one of the groups below: 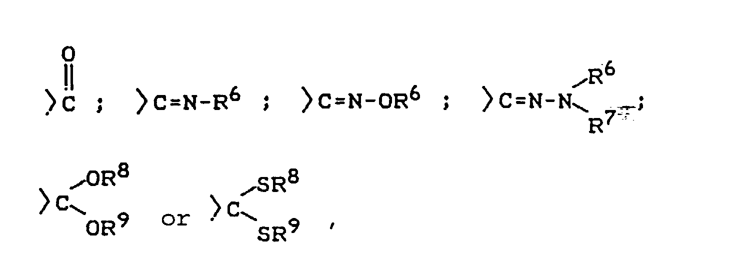
B represents hydrogen, methyl or ethyl,
X represents methyl, ethyl, propyl, 2-propyl, fluorine, chlorine, bromine, methoxy or ethoxy,
Y represents hydrogen, methyl, ethyl, propyl, i-propyl, butyl, i-butyl, tert.-butyl, fluorine, chlorine, bromine, methoxy, ethoxy or trifluoromethyl,
Z represents methyl, ethyl, propyl, i-propyl, butyl, i-butyl, tert.-butyl, fluorine, chlorine, bromine, methoxy or ethoxy,
n represents a number 0, 1, 2 or 3,
G represents hydrogen (a) or the groups 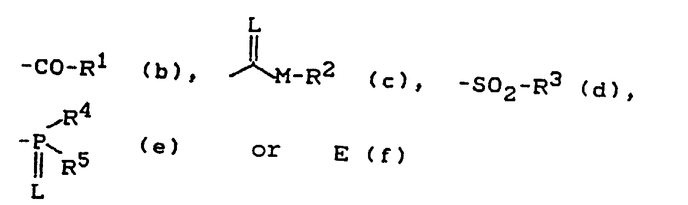
in which
E represents a metal ion equivalent or an ammonium ion,
L and M in each case represent oxygen and/or sulphur,
R¹ represents optionally fluorine- or chlorine-substituted C₁-C₁₄-alkyl, C₂-C₁₄-alkenyl, C₁-C₄-alkoxy-C₂-C₆-alkyl, C₁-C₄-alkylthio-C₂-C₆-alkyl, C₁-C₄-polyalkoxy-C₂-C₄-alkyl or cycloalkyl which has 3 to 6 ring atoms and which can be interrupted by 1 to 2 oxygen and/or sulphur atoms,
or represents phenyl which is optionally substituted by fluorine, chlorine, bromine, methyl, ethyl, propyl, i-propyl, methoxy, ethoxy, trifluoromethyl, trifluoromethoxy or nitro,
or represents phenyl-C₁-C₃-alkyl which is optionally substituted by fluorine, chlorine, bromine, methyl, ethyl, propyl, i-propyl, methoxy, ethoxy, trifluoromethyl or trifluoromethoxy,
or represents pyridyl, pyrimidyl, thiazolyl and pyrazolyl which are optionally substituted by fluorine, chlorine, bromine, methyl or ethyl,
or represents phenoxy-C₁-C₄-alkyl which is optionally substituted by fluorine, chlorine, methyl or ethyl,
or represents pyridyloxy-C₁-C₄-alkyl, pyrimidyloxy-C₁-C₄-alkyl and thiazolyloxyC₁-C₄-alkyl which are optionally substituted by fluorine, chlorine, amino, methyl or ethyl,
R² represents C₁-C₁₄-alkyl, C₂-C₁₄-alkenyl, C₁-C₄-alkoxy-C₂-C₆-alkyl or C₁-C₄-polyalkoxy-C₂-C₆-alkyl which are optionally substituted by fluorine or chlorine,
or represents phenyl or benzyl which are optionally substituted by fluorine, chlorine, nitro, methyl, ethyl, propyl, i-propyl, methoxy, ethoxy, trifluoromethyl,
R³, R⁴ and R⁵ independently of one another represent C₁-C₄-alkyl, C₁-C₄-alkoxy, C₁-C₄-alkylamino, di-(C₁-C₄)-alkylamino or C₁-C₄-alkylthio which are optionally substituted by fluorine or chlorine, or represent phenyl, phenoxy or phenylthio which are optionally substituted by fluorine, chlorine, bromine, nitro, cyano, C₁-C₂-alkoxy, C₁-C₄-fluoroalkoxy, C₁-C₂-chloroalkoxy, C₁-C₂-alkylthio, C₁-C₂-fluoroalkylthio, C₁-C₂-chloroalkylthio or C₁-C₃-alkyl,
R⁶ and R⁷ independently of one another represent optionally fluorine-, chlorine- or bromine-substituted C₁-C₁₀-alkyl, C₁-C₁₀-alkoxy or C₁-C₁₀-alkoxy- (C₁-C₁₀)-alkyl, or represent phenyl which is optionally substituted by fluorine, chlorine, bromine, C₁-C₂₀-halogenoalkyl, C₁-C₂₀-alkyl or C₁-C₄-alkoxy, or represent benzyl which is optionally substituted by fluorine, chlorine, bromine, C₁-C₄-alkyl, C₁-C₄-halogenoalkyl or C₁-C₄-alkoxy,
R⁸ and R⁹ independently of one another represent hydrogen or alkyl having 1 to 4 carbon atoms, or
R⁸ and R⁹ together represent an alkanediyl radical having 2 or 3 carbon atoms.

4. Process for the preparation of substituted bicyclic 3-aryl-pyrrolidine-2,4-dione derivatives of the formula (I) according to Claim 1,
(Aα) to obtain 3-aryl-pyrrolidine-2,4-diones or enols thereof of the formula (Ia) 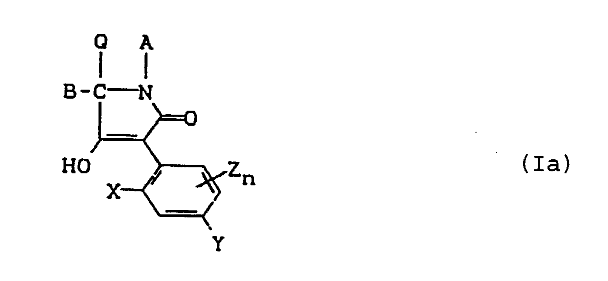 in which
A, B, Q, X, Y, Z and n have the abovementioned meaning,
N-acyl amino acid esters of the formula (II) 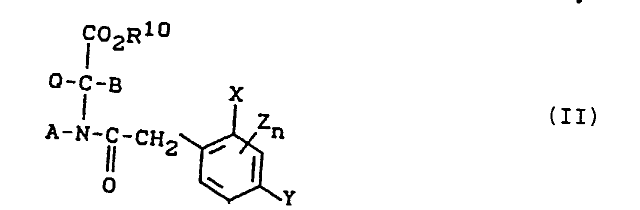 in which
A, B, Q, X, Y, Z and n have the abovementioned meaning
and
R¹⁰ represents alkyl
are subjected to an intramolecular condensation reaction in the presence of a diluent and in the presence of a base
or
(A-β) the compounds of the formula (Ia-a) which are obtained by process (Aα) 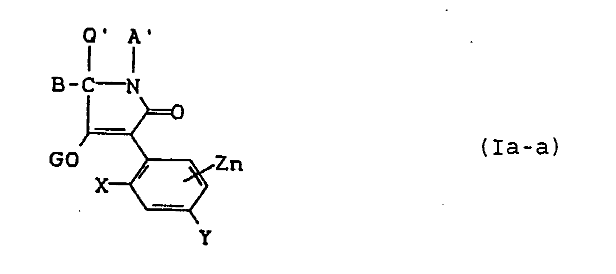 in which
B, X, Y, Z, n and G have the abovementioned meaning and
A¹ and Q¹ together represent a hydroxyl-substituted alkanediyl, alkenediyl, alkenedienyl or alkenetrenyl group
are either converted on the hydroxyl group by generally customary methods to give esters or ethers or in a first step, the hydroxyl group is converted with the aid of customary oxidation methods to give the corresponding ketones and the latter are, in a second step, reacted further with thienes, alcohols, diols, mercaptans or dithiols, also by customary methods,
or
(B) to obtain compounds of the formula (Ib) 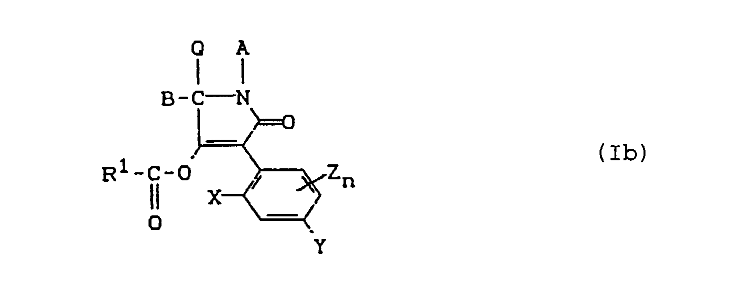 in which
A, B, Q, X, Y, Z, R¹ and n have the abovementioned meaning,
compounds of the formula (Ia) 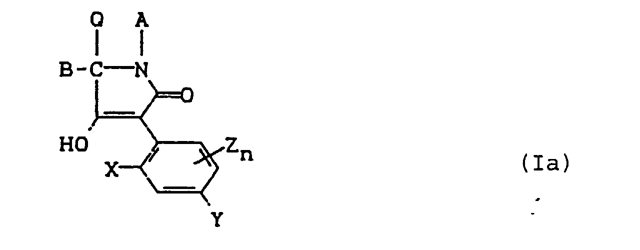 in which
A, B, Q, X, Y, Z and n have the abovementioned meaning
α) are reacted with acid halides of the general formula (III) 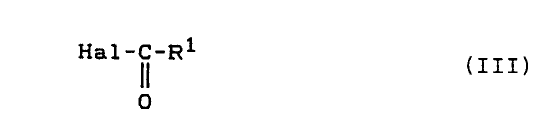 in which
R¹ has the abovementioned meaning and
Hal represents halogen, in particular chlorine and bromine,
if appropriate in the presence of a diluent and if appropriate in the presence of an acid-binding agent,
or
β) are reacted with carboxylic anhydrides of the general formula (IV)
R¹-CO-O-CO-R¹ (IV)
in which
R¹ has the abovementioned meaning,
if appropriate in the presence of a diluent and
if appropriate in the presence of an acid-binding agent;
or
(C) to obtain compounds of the formula (Ic-l) 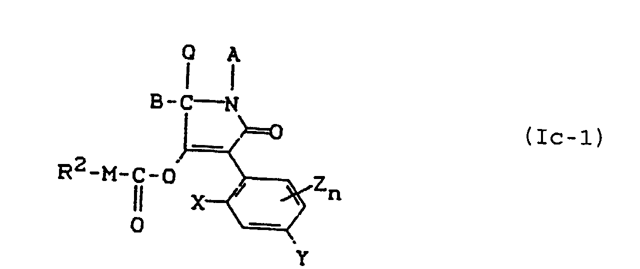 in which
A, B, Q, X, Y, Z, R² and n have the abovementioned meaning
and
M represents oxygen or sulphur,
compounds of the formula (Ia) 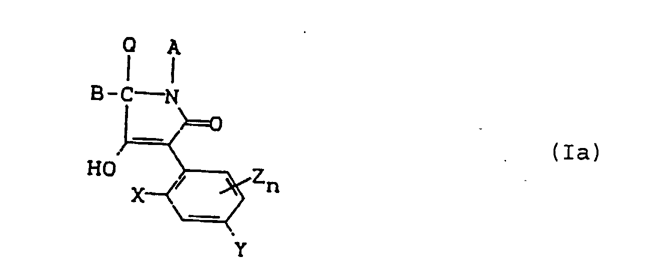 in which
A, B, Q, X, Y, Z and n have the abovementioned meaning
are reacted with chloroformic ester or chloroformic thioester of the general formula (V)
R²-M-CO-Cl (V)
in which
R² and M have the abovementioned meaning,
if appropriate in the presence of a diluent and if appropriate in the presence of an acid-binding agent;
or
(D) to obtain compounds of the formula (Ic-2) 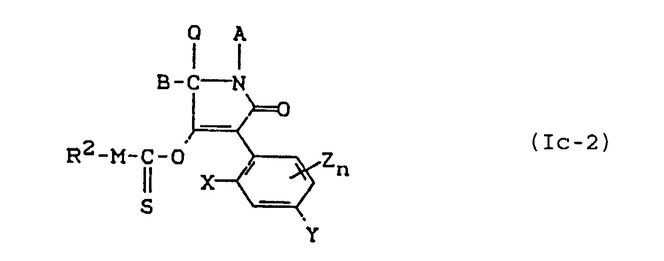 in which
A, B, Q, R², X, Y, Z and n have the abovementioned meaning
and
M represents oxygen or sulphur,
compounds of the formula (Ia) 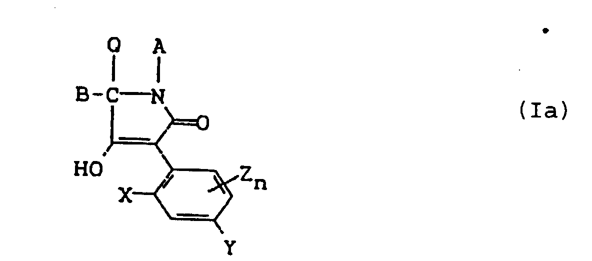 in which
A, B, Q, X, Y, Z and n have the abovementioned meaning
α) are reacted with chloromonothioformic esters or chlorodithioformic esters of the general formula (VI) 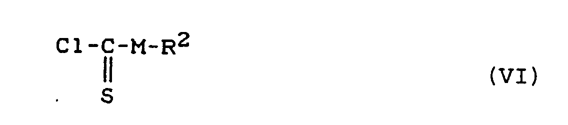 in which
M and R² have the abovementioned meaning,
if appropriate in the presence of a diluent and if appropriate in the presence of an acid-binding agent,
or
β) are reacted with carbon disulphide and subsequently with alkyl halides of the general formula (VII)
R²-Hal (VII)
in which
R² has the abovementioned meaning
and
Hal represents chlorine, bromine or iodine;
or
(E) to obtain compounds of the formula (Id) 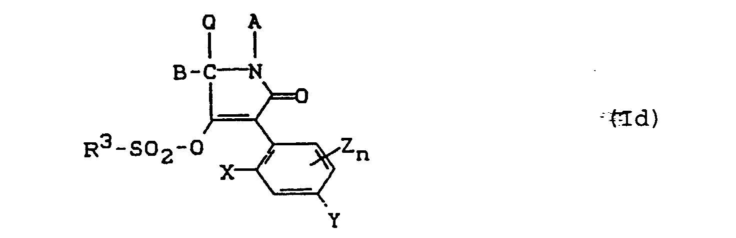 in which
A, B, Q, X, Y, Z, R³ and n have the abovementioned meaning,
compounds of the formula (Ia) 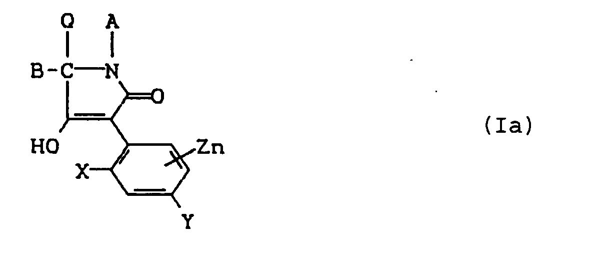 in which
A, B, Q, X, Y, Z and n have the abovementioned meaning
are reacted with sulphonyl chlorides of the general formula (VIII)
R³-SO₂-Cl (VIII)
in which
R³ has the abovementioned meaning,
if appropriate in the presence of a diluent and if appropriate in the presence of an acid-binding agent;
or
(F) to obtain 3-aryl-pyrrolidine-2,4-diones of the formula (Ie) 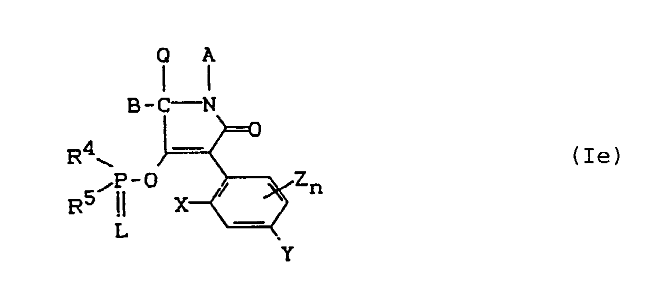 in which
A, B, Q, L, X, Y, Z, R⁴, R⁵ and n have the abovementioned meaning,
3-aryl-pyrrolidine-2,4-diones of the formula (Ia) or enols thereof 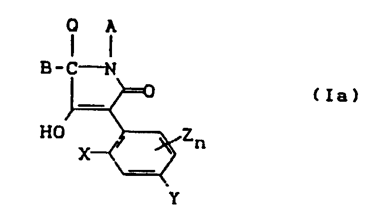 in which
A, B, Q, X, Y, Z and n have the abovementioned meaning
are reacted with phosphorus compounds of the general formula (IX) 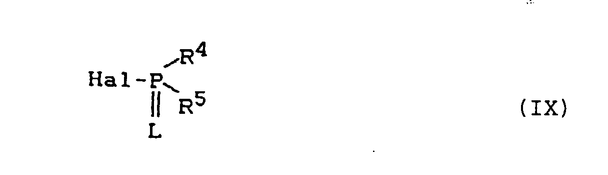 in which
L, R⁴ and R⁵ have the abovementioned meaning
and
Hal represents halogen, in particular chlorine and bromine;
or
(G) to obtain compounds of the formula (If) 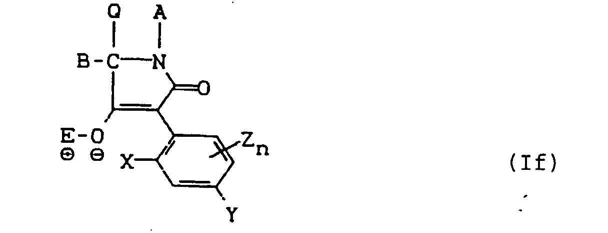 in which
A, B, Q, X, Y, Z and n have the abovementioned meaning
and
E represents a metal ion equivalent or an ammonium ion,
compounds of the formula (Ia) 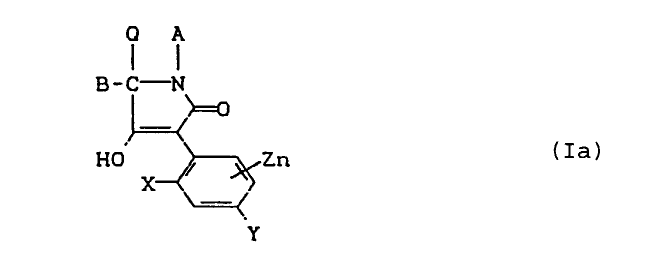 in which
A, B, Q, X, Y, Z and n have the abovementioned meaning
are reacted with metal hydroxides or amines of the general formulae (X) and (XI) 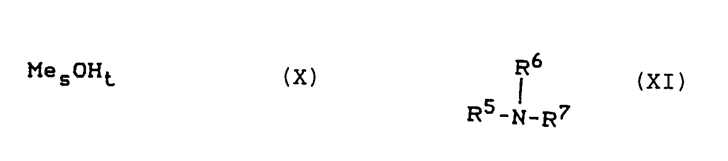 in which
Me represents mono- or divalent metal ions,
s and t represent the numbers 1 and 2 and
R⁵, R⁶ and R⁷ independently of one another represent hydrogen and alkyl,
if appropriate in the presence of a diluent.

5. Pesticides, characterised in that they contain at least one substituted bicyclic 3-arylpyrrolidine-2,4-dione derivative of the formula (I).

6. Method of combating animal pests, characterised in that substituted bicyclic 3-arylpyrrolidine-2,4-dione derivatives of the formula (I) are allowed to act on animal pests and/or their environment.

7. Use of substituted bicyclic 3-arylpyrrolidine-2,4-dione derivatives of the formula (I) for combating animal pests.

8. Process for the preparation of pesticides, characterised in that substituted bicyclic 3-arylpyrrolidine-2,4-dione derivatives of the formula (I) are mixed with extenders and/or surface-active agents.

9. Herbicidal agents, characterised in that they contain at least one substituted bicyclic 3-arylpyrrolidine-2,4-dione derivative of the formula (I).

10. Method of combating weeds, characterised in that substituted bicyclic 3-arylpyrrolidine-2,4-dione derivatives of the formula (I) are allowed to act on weeds and/or their environment.

11. Use of substituted bicyclic 3-arylpyrrolidine-2,4-dione derivatives of the formula (I) for combating weeds.

12. Process for the preparation of herbicidal agents, characterised in that substituted bicyclic 3-arylpyrrolidine-2,4-dione derivatives of the formula (I) are mixed with extenders and/or surface-active agents.

## Claims (Claims for the following Contracting State(s): ES)

1. Process for the preparation of substituted bicyclic 3-aryl-pyrrolidine-2,4-dione derivatives of the formula (I) 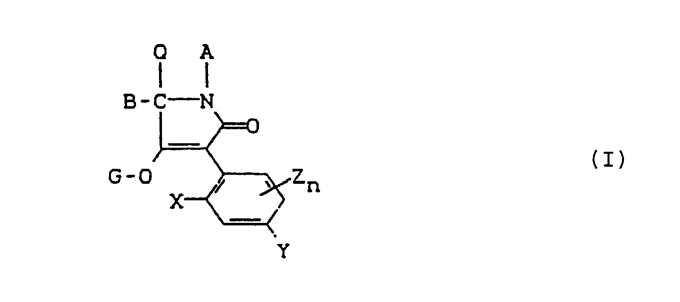 in which
A and Q together represent an alkanediyl, alkenediyl, alkenedienyl or alkenetrienyl group having 3 to 6 carbon atoms, each of these groups being monosubstituted to trisubstituted by identical or different substituents, each of these groups being substituted by halogen, hydroxyl, mercapto, in each case optionally halogen-substituted alkyl having 1 to 10 carbon atoms, alkylcarbonyloxy having 1 to 8 carbon atoms, alkoxy having 1 to 6 carbon atoms, alkylthio having 1 to 6 carbon atoms, cycloalkyl having 3 to 7 carbon atoms or aryl having 6 to 10 carbon atoms, and each of these groups additionally being interrupted, if appropriate, by one of the groups below: 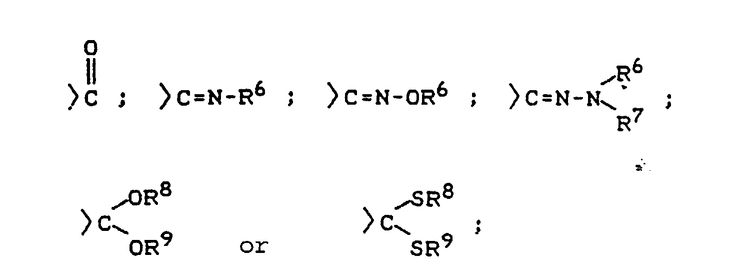
B represents hydrogen or alkyl having 1 to 6 carbon atoms,
X represents C₁-C₆-alkyl, halogen or C₁-C₆-alkoxy,
Y represents hydrogen, C₁-C₆-alkyl, halogen, C₁-C₆-alkoxy or C₁-C₃-halogenoalkyl,
Z represents C₁-C₆-alkyl, halogen or C₁-C₆-alkoxy,
n represents a number 0, 1, 2 or 3,
G represents hydrogen (a) or the groups 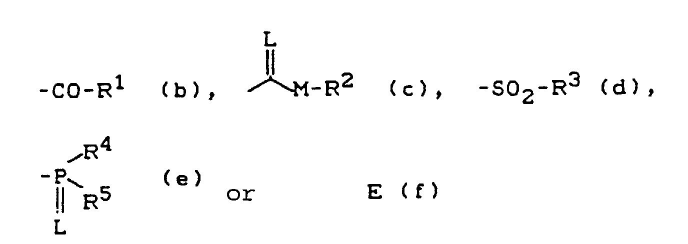
in which
E represents a metal ion equivalent or an ammonium ion,
L and M represent oxygen and/or sulphur,
R¹ represents optionally halogen-substituted C₁-C₂₀-alkyl, C₂-C₂₀-alkenyl, C₁-C₈-alkoxy-C₂-C₈-alkyl, C₁-C₈-alkylthio-C₂-C₈-alkyl, C₁-C₈-polyalkoxy-C₂-C₈-alkyl or cycloalkyl which have 3 to 8 ring atoms and which can be interrupted by oxygen and/or sulphur atoms,
or represents phenyl which is optionally substituted by halogen, nitro, C₁-C₆-alkyl, C₁-C₆-alkoxy, C₁-C₆-halogenoalkyl or C₁-C₆-halogenoalkoxy,
or represents phenyl-C₁-C₆-alkyl which is optionally substituted by halogen, C₁-C₆-alkyl, C₁-C₆-alkoxy, C₁-C₆-halogenoalkyl or C₁-C₆-halogenoalkoxy,
or represents hetaryl which is optionally substituted by halogen and/or C₁-C₆-alkyl,
or represents phenoxy-C₁-C₆-alkyl which is optionally substituted by halogen and C₁-C₆-alkyl,
or represents hetaryloxy-C₁-C₆-alkyl which is optionally substituted by halogen, amino and C₁-C₆-alkyl,
R² represents optionally halogen-substituted C₁-C₂₀-alkyl, C₂-C₂₀-alkenyl, C₁-C₈-alkoxy-C₂-C₈-alkyl or C₁-C₈-polyalkoxy-C₂-C₈-alkyl,
or represents phenyl or benzyl which are optionally substituted by halogen, nitro, C₁-C₆-alkyl, C₁-C₆-alkoxy or C₁-C₆-halogenoalkyl,
R³, R⁴ and R⁵ independently of one another represent optionally halogen-substituted C₁-C₈-alkyl, C₁-C₈-alkoxy, C₁-C₈-alkylamino, di-(C₁-C₈)-alkylamino, C₁-C₈-alkylthio, C₂-C₅-al kenylthio, C₂-C₅-alkinylthio or C₃-C₇- cycloalkylthio, or represent phenyl, phenoxy or phenylthio which are optionally substituted by halogen, nitro, cyano, C₁-C₄-alkoxy, C₁-C₄-halogenoalkoxy, C₁-C₄- alkylthio, C₁-C₄-halogenoalkyl-thio, C₁-C₄- alkyl or C₁-C₄-halogenoalkyl,
R⁶ and R⁷ independently of one another represent hydrogen, optionally halogen-substituted C₁-C₂₀-alkyl, C₁-C₂₀-alkoxy, C₂-C₈-alkenyl or C₁-C₂₀-alkoxy-C₁-C₂₀-alkyl, or represent phenyl which is optionally substituted by halogen, C₁-C₂₀-halogenoalkyl, C₁-C₂₀-alkyl or C₁-C₂₀-alkoxy, or represent benzyl which is optionally substituted by halogen, C₁-C₂₀-alkyl, C₁-C₂₀-halogenoalkyl or C₁-C₂₀- alkoxy, or together represent a C₂-C₆- alkylene ring which is optionally interrupted by oxygen,
R⁸ and R⁹ independently of one another represent hydrogen or alkyl having 1 to 6 carbon atoms, or
R⁸ and R⁹ together represent an alkanediyl radical having 2 to 4 carbon atoms,
characterized in that
(Aα) to obtain 3-aryl-pyrrolidine-2,4-diones or enols thereof of the formula (Ia) 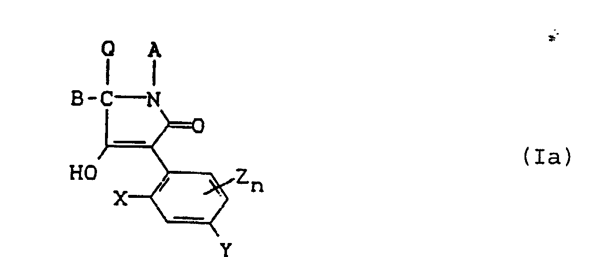 in which
A, B, Q, X, Y, Z and n have the abovementioned meaning,
N-acyl amino acid esters of the formula (II) 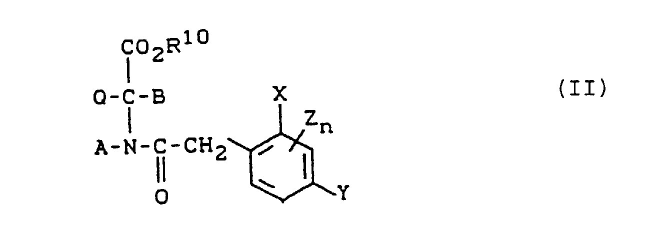 in which
A, B, Q, X, Y, Z and n have the abovementioned meaning
and
R¹⁰ represents alkyl,
are subjected to an intramolecular condensation reaction in the presence of a diluent and in the presence of a base
or
(A-β) the compounds of the formula (Ia-a) which are obtained by process (Aα) 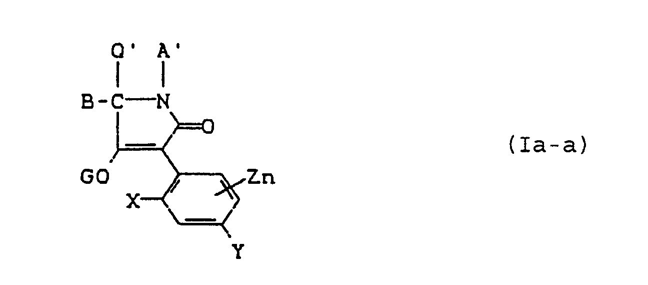 in which
B, X, Y, Z, n and G have the abovementioned meaning and
A¹ and Q¹ together represent a hydroxyl-substituted alkanediyl, alkenediyl, alkenedienyl or alkenetrenyl group
are either converted on the hydroxyl group by generally customary methods to give esters or ethers or in a first step, the hydroxyl group is converted with the aid of customary oxidation methods to give the corresponding ketones and the latter are, in a second step, reacted further with thienes, alcohols, diols, mercaptans or dithiols, also by customary methods,
or
(B) to obtain compounds of the formula (Ib) 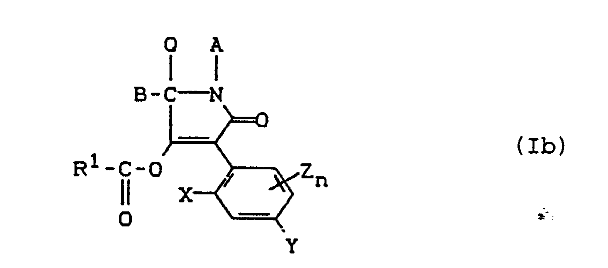 in which
A, B, Q, X, Y, Z, R¹ and n have the abovementioned meaning,
compounds of the formula (Ia) 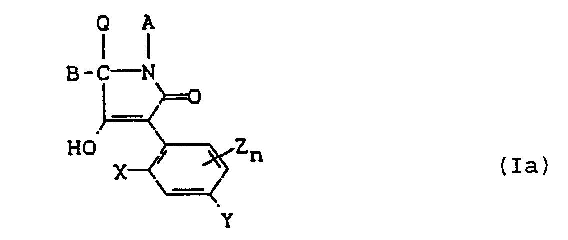 in which
A, B, Q, X, Y, Z and n have the abovementioned meaning
α) are reacted with acid halides of the general formula (III) 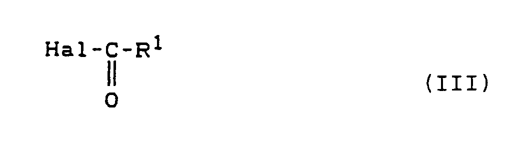 in which
R¹ has the abovementioned meaning and
Hal represents halogen, in particular chlorine and bromine,
if appropriate in the presence of a diluent and if appropriate in the presence of an acid-binding agent,
or
β) are reacted with carboxylic anhydrides of the general formula (IV)
R¹-CO-O-CO-R¹ (IV)
in which
R¹ has the abovementioned meaning,
if appropriate in the presence of a diluent and if appropriate in the presence of an acid-binding agent;
or
(C) to obtain compounds of the formula (Ic-l) 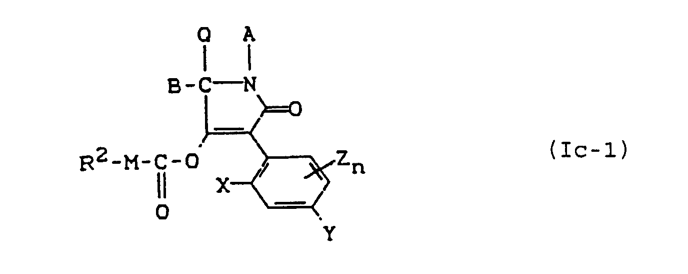 in which
A, B, Q, X, Y, Z, R² and n have the abovementioned meaning
and
M represents oxygen or sulphur,
compounds of the formula (Ia) 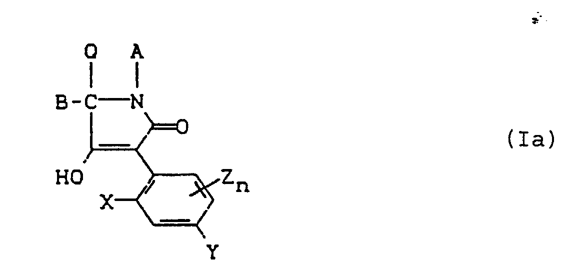 in which
A, B, Q, X, Y, Z and n have the abovementioned meaning
are reacted with chloroformic ester or chloroformic thioester of the general formula (V)
R²-M-CO-Cl (V)
in which
R² and M have the abovementioned meaning,
if appropriate in the presence of a diluent and if appropriate in the presence of an acid-binding agent;
or
(D) to obtain compounds of the formula (Ic-2) 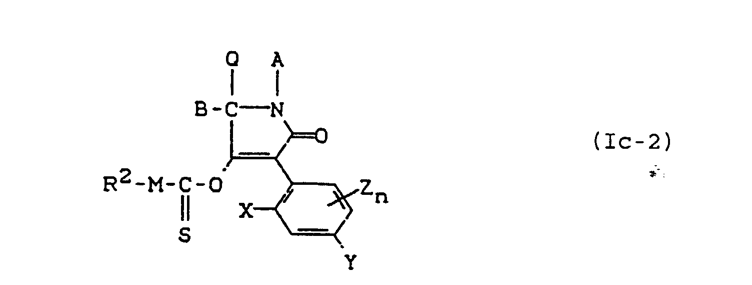 in which
A, B, Q, R², X, Y, Z and n have the abovementioned meaning
and
M represents oxygen or sulphur,
compounds of the formula (Ia) 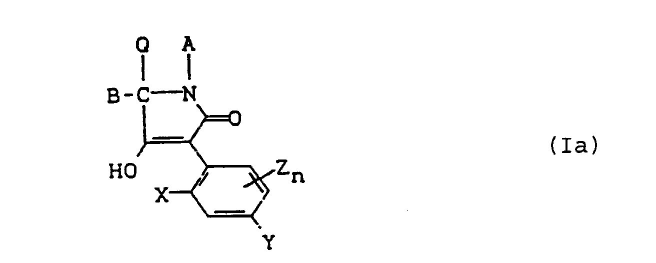 in which
A, B, Q, X, Y, Z and n have the abovementioned meaning
α) are reacted with chloromonothioformic esters or chlorodithioformic esters of the general formula (VI) 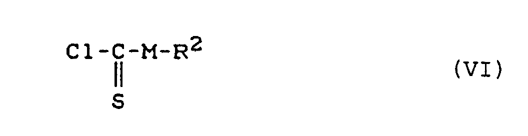 in which
M and R² have the abovementioned meaning,
if appropriate in the presence of a diluent and if appropriate in the presence of an acid-binding agent,
or
β) are reacted with carbon disulphide and subsequently with alkyl halides of the general formula (VII)
R²-Hal (VII)
in which
R² has the abovementioned meaning
and
Hal represents chlorine, bromine or iodine;
or
(E) to obtain compounds of the formula (Id) 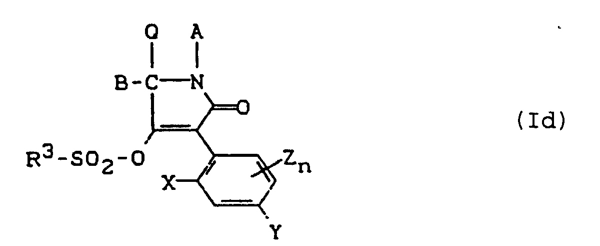 in which
A, B, Q, X, Y, Z, R³ and n have the abovementioned meaning,
compounds of the formula (Ia) 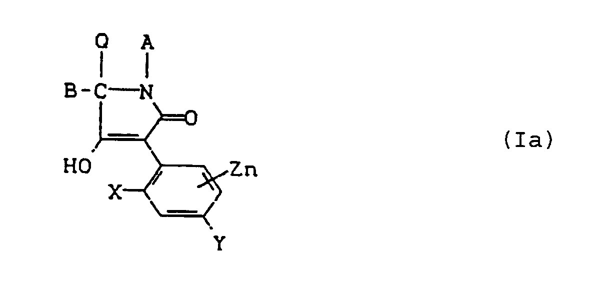 in which
A, B, Q, X, Y, Z and n have the abovementioned meaning
are reacted with sulphonyl chlorides of the general formula (VIII)
R³-SO₂-Cl (VIII)
in which
R³ has the abovementioned meaning,
if appropriate in the presence of a diluent and if appropriate in the presence of an acid-binding agent;
or
(F) to obtain 3-aryl-pyrrolidine-2,4-diones of the formula (Ie) 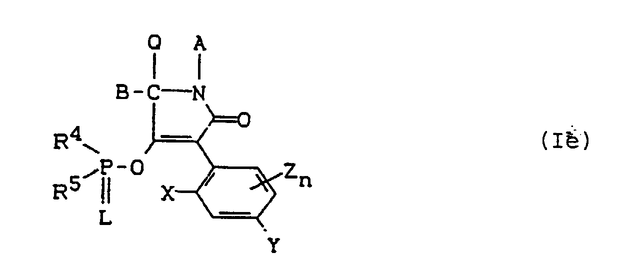 in which
A, B, Q, L, X, Y, Z, R⁴, R⁵ and n have the abovementioned meaning,
3-aryl-pyrrolidine-2,4-diones of the formula (Ia) or enols thereof 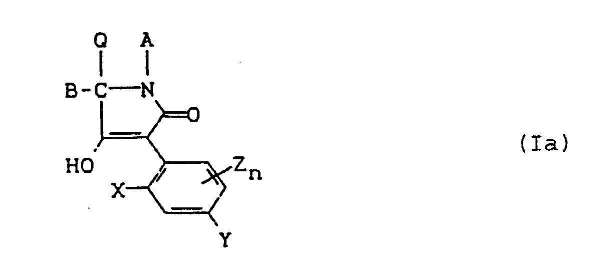 in which
A, B, Q, X, Y, Z and n have the abovementioned meaning
are reacted with phosphorus compounds of the general formula (IX) 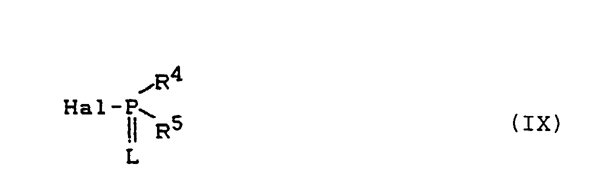 in which
L, R⁴ and R⁵ have the abovementioned meaning
and
Hal represents halogen, in particular chlorine and bromine;
or
(G) to obtain compounds of the formula (If) 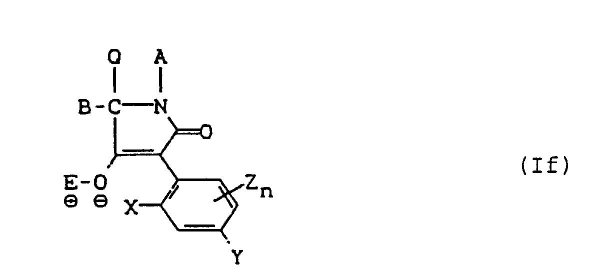 in which
A, B, Q, X, Y, Z and n have the abovementioned meaning
and
E represents a metal ion equivalent or an ammonium ion,
compounds of the formula (Ia) 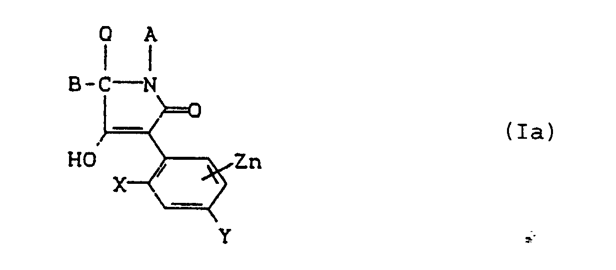 in which
A, B, Q, X, Y, Z and n have the abovementioned meaning
are reacted with metal hydroxides or amines of the general formulae (X) and (XI) 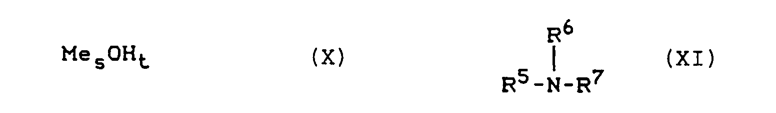 in which
Me represents mono- or divalent metal ions,
s and t represent the numbers 1 and 2 and
R⁵, R⁶ and R⁷ independently of one another represent hydrogen and alkyl,
if appropriate in the presence of a diluent.

2. Pesticides, characterised in that they contain at least one substituted bicyclic 3-arylpyrrolidine-2,4-dione derivative of the formula (I).

3. Method of combating animal pests, characterised in that substituted bicyclic 3-arylpyrrolidine-2,4-dione derivatives of the formula (I) are allowed to act on animal pests and/or their environment.

4. Use of substituted bicyclic 3-arylpyrrolidine-2,4-dione derivatives of the formula (I) for combating animal pests.

5. Process for the preparation of pesticides, characterised in that substituted bicyclic 3-arylpyrrolidine-2,4-dione derivatives of the formula (I) are mixed with extenders and/or surface-active agents.

6. Herbicidal agents, characterised in that they contain at least one substituted bicyclic 3-arylpyrrolidine-2,4-dione derivative of the formula (I).

7. Method of combating weeds, characterised in that substituted bicyclic 3-arylpyrrolidine-2,4-dione derivatives of the formula (I) are allowed to act on weeds and/or their environment.

8. Use of substituted bicyclic 3-arylpyrrolidine-2,4-dione derivatives of the formula (I) for combating weeds.

9. Process for the preparation of herbicidal agents, characterised in that substituted bicyclic 3-arylpyrrolidine-2,4-dione derivatives of the formula (I) are mixed with extenders and/or surface-active agents.

## Revendications (Revendications pour l'(les) Etat(s) contractant(s) suivant(s): BE, CH, DE, DK, FR, GB, GR, IT, LI, NL)

1. Dérivés bicycliques substitués de 3-arylpyrrolidine-2,4-diones de formule (I) 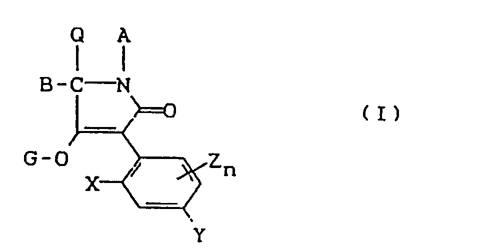 caractérisés en ce que
A et Q forment en commun un groupement alcanediyle, alcènediyle, alcènediényle ou alcénetriényle de 3 à 6 atomes de carbone, qui est substitué dans chaque cas une à trois fois identiques ou différentes par un halogène, un groupe hydroxy, mercapto, un groupe alkyle ayant 1 à 10 atomes de carbone, alkylcarbonyloxy ayant 1 à 8 atomes de carbone dans la partie alkyle, alkoxy ayant 1 à 6 atomes de carbone, alkylthio ayant 1 à 6 atomes de carbone, cycloalkyle ayant 3 à 7 atomes de carbone ou aryle ayant 6 à 10 atomes de carbone substitué dans chaque cas éventuellement par un halogène, et qui est en outre interrompu, le cas échéant par l'un des groupements suivants : 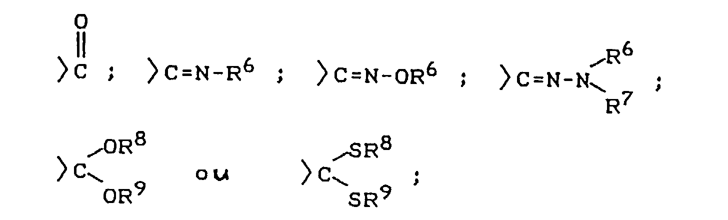
B représente l'hydrogène ou un groupe alkyle ayant 1 à 6 atomes de carbone,
X est un groupe alkyle en C₁ à C₆, un halogène ou un groupe alkoxy en C₁ à C₆,
Y est l'hydrogène, un groupe alkyle en C₁ à C₆, un halogène, un groupe alkoxy en C₁ à C₆ ou un groupe halogénalkyle en C₁ à C₃,
Z est un groupe alkyle en C₁ à C₆, un halogène ou un groupe alkoxy en C₁ à C₆,
n représente le nombre 0, 1, 2 ou 3,
G est l'hydrogène (a) ou les groupes 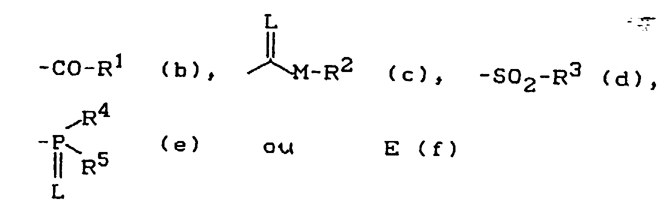
dans lesquels
E représente un équivalent d'ion métallique ou un ion ammonium,
L et M représentent l'oxygène et/ou le soufre,
R¹ est un groupe, éventuellement substitué par un halogène, alkyle en C₁ à C₂₀, alcényle en C₂ à C₂₀, (alkoxy en C₁ à C₈)-(alkyle en C₂ à C₈), (alkylthio en C₁ à C₈)-(alkyle en C₂ à C₈), poly(alkoxy en C₁ à C₈)-(alkyle en C₂ à C₈) ou cycloalkyle à noyau de 3 à 8 atomes de carbone, qui peut être interrompu par des atomes d'oxygène et/ou de soufre,
un groupe phényle éventuellement substitué par un radical halogéno, nitro, alkyle en C₁ à C₆, alkoxy en C₁ à C₆, halogénalkyle en C₁ à C₆, halogénalkoxy en C₁ à C₆,
un groupe phényl-(alkyle en C₁ à C₆) éventuellement substitué par un radical halogéno, alkyle en C₁ à C₆, alkoxy en C₁ à C₆, halogénalkyle en C₁ à C₆, halogénalkoxy en C₁ à C₆,
un groupe hétaryle éventuellement substitué par un radical halogéno et/ou alkyle en C₁ à C₆,
un groupe phénoxy-(alkyle en C₁ à C₆) éventuellement substitué par un radical halogéno et un radical alkyle en C₁ à C₆,
un groupe hétaryloxy-(alkyle en C₁ à C₆) éventuellement substitué par un radical halogéno, amino et alkyle en C₁ à C₆,
R² est un groupe, éventuellement substitué par un halogène, alkyle en C₁ à C₂₀, alcényle en C₂ à C₂₀, (alkoxy en C₁ à C₈)-(alkyle en C₂ à C₈), poly(alkoxy en C₁ à C₈)-(alkyle en C₂ à C₈),
un groupe phényle ou benzyle éventuellement substitué par un radical halogéno, nitro, alkyle en C₁ à C₆, alkoxy en C₁ à C₆, halogénalkyle en C₁ à C₆,
R³, R⁴ et R⁵ représentent, indépendamment les uns des autres, un groupe, éventuellement substitué par un halogène, alkyle en C₁ à C₈, alkoxy en C₁ à C₈, alkylamino en C₁ à C₈, di-(alkyle en C₁ à C₈)-amino, alkylthio en C₁ à C₈, alcénylthio en C₂ à C₅, alcynylthio en C₂ à C₅, cycloalkylthio en C₃ à C₇, un groupe phényle, phénoxy ou phénylthio éventuellement substitué par un radical halogéno, nitro, cyano, alkoxy en C₁ à C₄, halogénalkoxy en C₁ à C₄, alkylthio en C₁ à C₄, halogénalkylthio en C₁ à C₄, alkyle en C₁ à C₄, halogénalkyle en C₁ à C₄,
R⁶ et R⁷ représentent, indépendamment l'un de l'autre, l'hydrogène, un groupe, éventuellement substitué par un halogène, alkyle en C₁ à C₂₀, alkoxy en C₁ à C₂₀, alcényle en C₂ à C₈, (alkoxy en C₁ à C₂₀)-(alkyle en C₁ à C₂₀), un groupe phényle éventuellement substitué par un radical halogéno, halogénalkyle en C₁ à C₂₀, alkyle en C₁ à C₂₀ ou alkoxy en C₁ à C₂₀, un groupe benzyle éventuellement substitué par un radical halogéno, alkyle en C₁ à C₂₀, halogénalkyle en C₁ à C₂₀ ou alkoxy en C₁ à C₂₀, ou forment ensemble un noyau alkylène en C₂ à C₆ éventuellement interrompu par de l'oxygène,
R⁸ et R⁹ représentent, indépendamment l'un de l'autre, l'hydrogène ou un groupe alkyle ayant 1 à 6 atomes de carbone, ou bien
R⁸ et R⁹ forment ensemble un reste alcanediyle ayant 2 à 4 atomes de carbone.

2. Dérivés bicycliques substitués de 3-arylpyrrolidine-2,4-diones de formule (I) suivant la revendication 1, caractérisés en ce que
A et Q forment ensemble un groupement alcanediyle, alcènediyle, alcènediényle de 3 à 5 atomes de carbone qui est substitué dans chaque cas une à trois fois identiques ou différentes par du fluor, du chlore, du brome, un radical hydroxy, mercapto, un radical alkyle ayant 1 à 8 atomes de carbone, alkylcarbonyloxy ayant 1 à 6 atomes de carbone dans la partie alkyle, alkoxy ayant 1 à 4 atomes de carbone, alkylthio ayant 1 à 4 atomes de carbone, cycloalkyle ayant 5 à 7 atomes de carbone ou phényle substitué dans chaque cas éventuellement par un halogène, et qui est en outre interrompu, le cas échéant par l'un des groupements suivants : 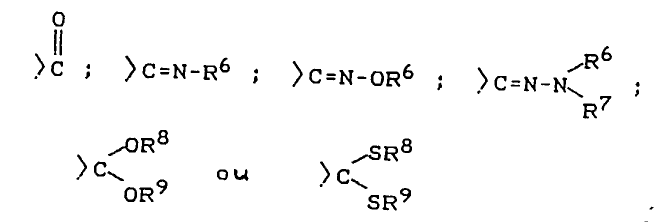
B représente l'hydrogène ou un groupe alkyle en C₁ à C₄,
X représente un groupe alkyle en C₁ à C₄, un halogène ou un groupe alkoxy en C₁ à C₄,
Y est l'hydrogène, un groupe alkyle en C₁ à C₄, un halogène, un groupe alkoxy en C₁ à C₄ ou halogénalkyle en C₁ ou C₂,
Z est un groupe alkyle en C₁ à C₄, un halogène ou un groupe alkoxy en C₁ à C₄,
n représente le nombre 0, 1, 2 ou 3,
G représente l'hydrogène (a) ou les groupes 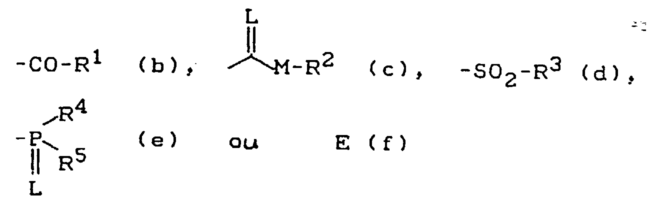
dans lesquels
E est un équivalent d'ion métallique ou un ion ammonium,
L et M représentent chacun l'oxygène et/ou le soufre,
R¹ est un groupe, éventuellement substitué par un halogène, alkyle en C₁ à C₁₆, alcényle en C₂ à C₁₆, (alkoxy en C₁ à C₆)-(alkyle en C₂ à C₆), (alkylthio en C₁ à C₁₆)-(alkyle en C₂ à C₆), poly(alkoxy en C₁ à C₆)-(alkyle en C₂ à C₆) ou cycloalkyle à noyau de 3 à 7 atomes de carbone, qui peut être interrompu par 1-2 atomes d'oxygène et/ou de soufre,
un groupe phényle éventuellement substitué par un radical halogéno, nitro, alkyle en C₁ à C₄, alkoxy en C₁ à C₄, halogénalkyle en C₁ à C₃, halogénalkoxy en C₁ à C₃,
un groupe phényl-(alkyle en C₁ à C₄) éventuellement substitué par un radical halogéno, alkyle en C₁ à C₄, alkoxy en C₁ à C₄, halogénalkyle en C₁ à C₃, halogénalkoxy en C₁ à C₃,
un groupe hétaryle éventuellement substitué par un radical halogéno et/ou alkyle en C₁ à C₆,
un groupe phénoxy-(alkyle en C₁ à C₅) éventuellement substitué par un radical halogéno et un radical alkyle en C₁ à C₄,
un groupe hétaryloxy-(alkyle en C₁ à C₅) éventuellement substitué par un radical halogéno, amino et alkyle en C₁ à C₄,
R² est un groupe, éventuellement substitué par un halogène, alkyle en C₁ à C₁₆, alcényle en C₂ à C₁₆, (alkoxy en C₁ à C₁₆)-(alkyle en C₂ à C₆), poly(alkoxy en C₁ à C₆)-(alkyle en C₂ à C₆),
un groupe phényle ou benzyle éventuellement substitué par un radical halogéno, nitro, alkyle en C₁ à C₄, alkoxy en C₁ à C₃, halogénalkyle en C₁ à C₃,
R³, R⁴ et R⁵ représentent, indépendamment les uns des autres, un groupe, éventuellemenet substitué par un halogène, alkyle en C₁ à C₆, alkoxy en C₁ à C₆, alkylamino en C₁ à C₆, di-(alkyle en C₁ à C₆)-amino, alkylthio en C₁ à C₆, alcénylthio en C₃ ou C₄, alcynylthio en C₂ à C₄, cycloalkylthio en C₃ à C₆, un groupe phényle, phénoxy ou phénylthio éventuellement substitué par du fluor, du chlore, du brome, un radical nitro, cyano, alkoxy en C₁ à C₃, halogénalkoxy en C₁ à C₃, alkylthio en C₁ à C₃, halogénalkylthio en C₁ à C₃, alkyle en C₁ à C₃, halogénalkyle en C₁ à C₃,
R⁶ et R⁷ représentent, indépendamment l'un de l'autre, l'hydrogène, un groupe, éventuellement substitué par un halogène, alkyle en C₁ à C₂₀, alkoxy en C₁ à C₂₀, alcényle en C₂ à C₈, (alkoxy en C₁ à C₂₀)-(alkyle en C₁ à C₂₀), un groupe phényle éventuellement substitué par un radical halogéno, halogénalkyle en C₁ à C₅, alkyle en C₁ à C₅ ou alkoxy en C₁ à C₅, un groupe benzyle éventuellement substitué par un radical halogéno, alkyle en C₁ à C₅, halogénalkyle en C₁ à C₅ ou alkoxy en C₁ à C₅,
R⁸ et R⁹ représentent, indépendamment l'un de l'autre, l'hydrogène ou un groupe alkyle ayant 1 à 4 atomes de carbone, ou bien
R⁸ et R⁹ forment conjointement un reste alcanediyle ayant 2 ou 3 atomes de carbone.

3. Dérivés bicycliques substitués de 3-arylpyrrolidine-2,4-diones de formule (I) suivant la revendication 1, caractérisés en ce que
A et Q forment ensemble un groupement alcanediyle, alcènediyle ou alcènediényle de 3 ou 4 atomes de carbone, qui est substitué dans chaque cas une à trois fois identiques ou différentes par un radical fluoro, chloro, bromo, hydroxy, mercapto, un radical alkyle ayant 1 à 6 atomes de carbone, alkylcarbonyloxy ayant 1 à 4 atomes de carbone dans la partie alkyle, alkoxy ayant 1 ou 2 atomes de carbone, alkylthio ayant 1 ou 2 atomes de carbone ou cycloalkyle ayant 5 ou 6 atomes de carbone éventuellement substitué dans chaque cas par du fluor, du chlore ou du brome, et qui est en outre interrompu, le cas échéant par l'un des groupements suivants : 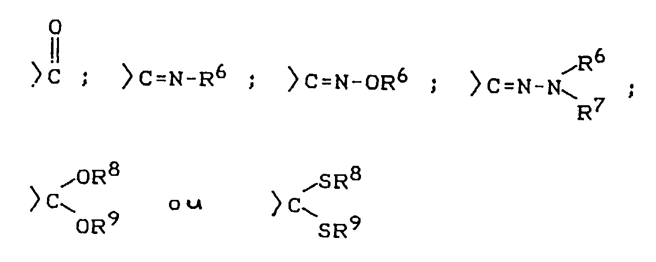
B représente l'hydrogène, un groupe méthyle ou éthyle,
X est un groupe méthyle, éthyle, propyle, 2-propyle, du fluor, du chlore, du brome, un groupe méthoxy ou éthoxy,
Y représente l'hydrogène, un groupe méthyle, éthyle, propyle, isopropyle, butyle, isobutyle, tertio-butyle, du fluor, du chlore, du brome, un groupe méthoxy, éthoxy ou trifluorométhyle,
Z représente un groupe méthyle, éthyle, propyle, isopropyle, butyle, isobutyle, tertio-butyle, du fluor, du chlore, du brome, un groupe méthoxy ou éthoxy,
n représente le nombre 0, 1, 2 ou 3,
G représente l'hydrogène (a) ou les groupes 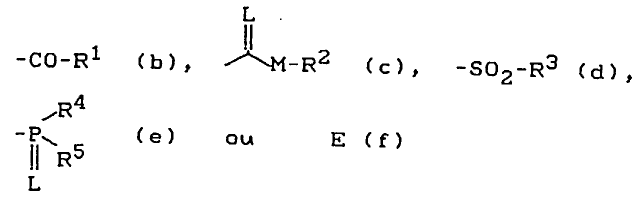
dans lesquels
E est un équivalent d'ion métallique ou un ion ammonium,
L et M représentent chacun l'oxygène et/ou le soufre,
R¹ est un groupe, éventuellement substitué par du fluor ou du chlore, alkyle en C₁ à C₁₄, alcényle en C₂ à C₁₄, (alkoxy en C₁ à C₄)-(alkyle en C₂ à C₆), (alkylthio en C₁ à C₄)-(alkyle en C₂ à C₆), poly(alkoxy en C₁ à C₄)-(alkyle en C₂ à C₄) ou cycloalkyle à noyau de 3 à 6 atomes de carbone, qui peut être interrompu par 1 ou 2 atomes d'oxygène et/ou de soufre,
un groupe phényle éventuellement substitué par du fluor, du chlore, du brome, un radical méthyle, éthyle, propyle, isopropyle, méthoxy, éthoxy, trifluorométhyle, trifluorométhoxy, nitro,
un groupe phényl-(alkyle en C₁ à C₃) éventuellement substitué par du fluor, du chlore, du brome, un radical méthyle, éthyle, propyle, isopropyle, méthoxy, éthoxy, trifluorométhyle, trifluorométhoxy,
des groupes pyridyle, pyrimidyle, thiazolyle et pyrazolyle éventuellement substitués par du fluor, du chlore, du brome, un radical méthyle, éthyle,
un groupe phénoxy-(alkyle en C₁ à C₄) éventuellement substitué par du fluor, du chlore, un radical méthyle, éthyle,
des groupes pyridyloxy-(alkyle en C₁ à C₄), pyrimidyloxy-(alkyle en C₁ à C₄) et thiazolyloxy-(alkyle en C₁ à C₄) éventuellement substitués par un radical fluoro, chloro, amino, méthyle, éthyle,
R² est un groupe, éventuellement substitué par du fluor ou du chlore, alkyle en C₁ à C₁₄, alcényle en C₂ à C₁₄, (alkoxy en C₁ à C₄) -(alkyle en C₂ à C₆), poly(alkoxy en C₁ à C₄)-(alkyle en C₂ à C₆),
ou un groupe phényle ou benzyle éventuellement substitué par du fluor, du chlore, un radical nitro, méthyle, éthyle, propyle, isopropyle, méthoxy, éthoxy, trifluorométhyle,
R³, R⁴ et R⁵ représentent, indépendamment les uns des autres, un groupe, éventuellemenet substitué par du fluor ou du chlore, alkyle en C₁ à C₄, alkoxy en C₁ à C₄, alkylamino en C₁ à C₄, di-(alkyle en C₁ à C₄)-amino, alkylthio en C₁ à C₄, un groupe phényle, phénoxy ou phénylthio éventuellement substitué par du fluor, du chlore, du brome, un radical nitro, cyano, alkoxy en C₁ ou C₂, fluoralkoxy en C₁ à C₄, chloralkoxy en C₁ ou C₂, alkylthio en C₁ ou C₂, fluoralkylthio en C₁ ou C₂, chloralkylthio en C₁ ou C₂, alkyle en C₁ à C₃,
R⁶ et R⁷ représentent, indépendamment l'un de l'autre, un groupe, éventuellement substitué par du fluor, du chlore ou du brome, alkyle en C₁ à C₁₀, alkoxy en C₁ à C₁₀, (alkoxy en C₁ à C₁₀)-(alkyle en C₁ à C₁₀), un groupe phényle éventuellement substitué par du fluor, du chlore, du brome, un radical halogénalkyle en C₁ à C₂₀, alkyle en C₁ à C₂₀ ou alkoxy en C₁ à C₄, un groupe benzyle éventuellement substitué par du fluor, du chlore, du brome, un radical alkyle en C₁ à C₄, halogénalkyle en C₁ à C₄ ou alkoxy en C₁ à C₄,
R⁸ et R⁹ représentent, indépendamment l'un de l'autre, l'hydrogène ou un groupe alkyle ayant 1 à 4 atomes de carbone, ou bien
R⁸ et R⁹ forment ensemble un reste alcanediyle ayant 2 ou 3 atomes de carbone.

4. Procédé de production de dérivés bicycliques substitués de 3-aryl-pyrrolidine-2,4-diones de formule (I) suivant la revendication 1,
caractérisé en ce que
(Aα) pour obtenir des 3-aryl-pyrrolidine-2,4-diones et leurs énols de formule (Ia) 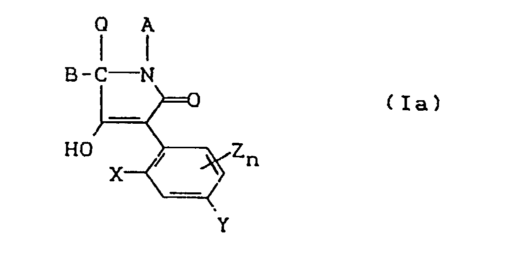 dans laquelle
A, B, Q, X, Y, Z et n ont la définition indiquée ci-dessus,
on effectue la condensation intramoléculaire d'un ester de N-acylamino-acide de formule (II) 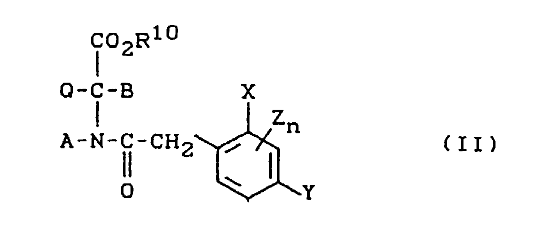 dans laquelle
A, B, Q, X, Y, Z et n ont la définition indiquée ci-dessus, et
R¹⁰ est un groupe alkyle,
en présence d'un diluant et en présence d'une base,
ou bien
(A-β) on transforme en esters ou éthers les composés, obtenus selon le procédé (Aα), de formule (Ia-a) 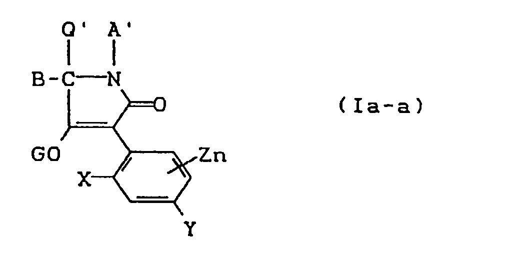 dans laquelle
B, X, Y, Z, n et G ont la définition indiquée ci-dessus et
A' et Q' forment ensemble un groupement alcanediyle, alcènediyle, alcènediényle ou alcènetriényle substitué par un radical hydroxy
au niveau du groupement hydroxy selon des procédés généraux classiques, ou bien on transforme dans une première étape le groupement hydroxy en les cétones correspondantes à l'aide de modes opératoires classiques d'oxydation et on fait réagir ces cétones dans une seconde étape selon des modes opératoires également classiques avec des thiènes, des alcools, des diols, des mercaptans ou des dithiols,
ou bien
(B) pour obtenir des composés de formule (Ib) 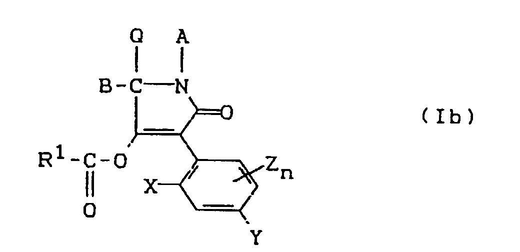 dans laquelle
A, B, Q, X, Y, Z, R¹ et n ont la définition indiquée ci-dessus,
des composés de formule (Ia), 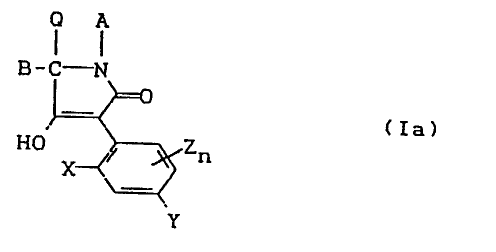 dans laquelle
A, B, Q, X, Y, Z et n ont la définition indiquée ci-dessus,
sont amenés à réagir
α) avec des halogénures d'acides de formule générale (III) 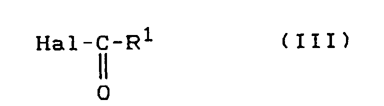 dans laquelle
R¹ a la définition indiquée ci-dessus, et
Hal représente un halogène, en particulier le chlore et le brome,
le cas échéant en présence d'un diluant et en la présence éventuelle d'un accepteur d'acide
ou bien
β) avec des anhydrides d'acides carboxyliques de formule générale (IV)
R¹-CO-O-CO-R¹ (IV)
dans laquelle
R¹ a la définition indiquée ci-dessus,
le cas échéant en présence d'un diluant et en la présence éventuelle d'un accepteur d'acide,
ou bien
(C) pour obtenir des composés de formule (Ic-l) 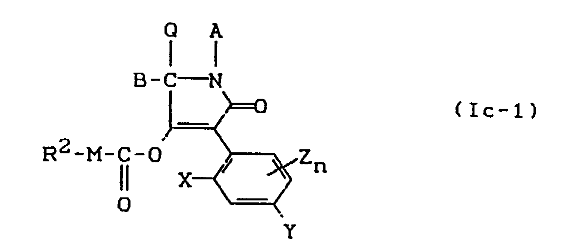 dans laquelle
A, B, Q, X, Y, Z, R² et n ont la définition indiquée ci-dessus,
et
M est l'oxygène ou le soufre,
on fait réagir des composés de formule (Ia) 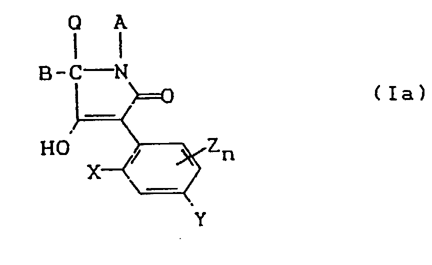 dans laquelle
A, B, Q, X, Y, Z et n ont la définition indiquée ci-dessus,
avec un ester d'acide chloroformique ou un thioester d'acide chloroformique de formule générale (V)
R²-M-CO-Cl (V)
dans laquelle
R² et M ont la définition indiquée ci-dessus,
le cas échéant en présence d'un diluant et en la présence éventuelle d'un accepteur d'acide ;
ou bien
(D) pour obtenir des composés de formule (Ic-2) 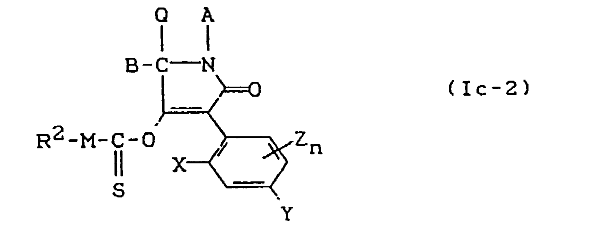 dans laquelle
A, B, Q, R², X, Y, Z et n ont la définition indiquée ci-dessus,
et
M représente l'oxygène ou le soufre,
on fait réagir des composés de formule (Ia) 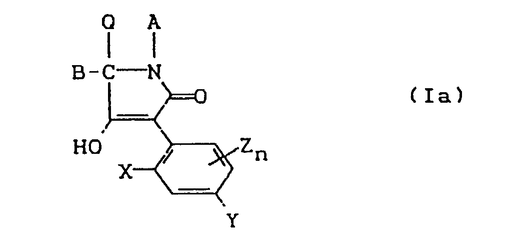 dans laquelle
A, B, Q, X, Y, Z et n ont la définition indiquée ci-dessus,
α) avec des esters d'acide chloromonothioformique ou des esters d'acide chlorodithioformique de formule générale (VI) 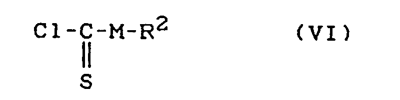 dans laquelle
M et R² ont la définition indiquée ci-dessus,
le cas échéant en présence d'un diluant et en la présence éventuelle d'un accepteur d'acide,
ou bien
β) avec le sulfure de carbone, puis avec des halogénures d'alkyle de formule générale (VII)
R²-Hal (VII)
dans laquelle
R² a la définition indiquée ci-dessus
et
Hal représente le chlore, le brome, l'iode ;
ou bien
(E) pour obtenir des composés de formule (Id) 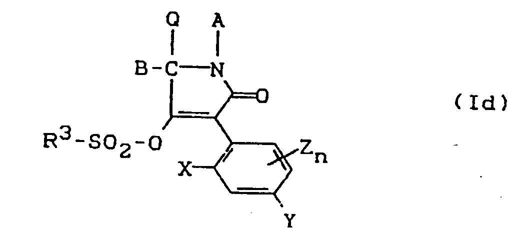 dans laquelle
A, B, Q, X, Y, Z, R³ et n ont la définition indiquée ci-dessus,
on fait réagir des composés de formule (Ia) 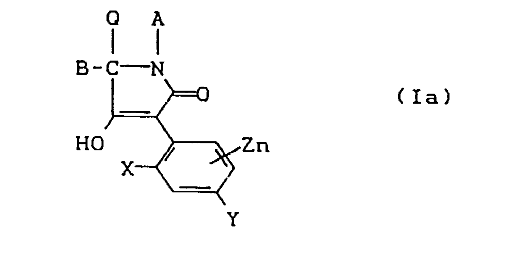 dans laquelle
A, B, Q, X, Y, Z et n ont la définition indiquée ci-dessus,
avec des chlorures d'acides sulfoniques de formule générale (VIII)
R³-SO₂-Cl (VIII)
dans laquelle
R³ a la définition indiquée ci-dessus,
le cas échéant en présence d'un diluant et en la présence éventuelle d'un accepteur d'acide ;
ou bien
(F) pour obtenir des 3-aryl-pyrrolidine-2,4-diones de formule (Ie) 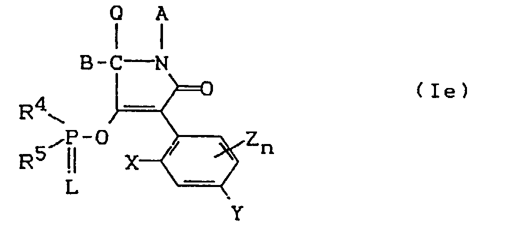 dans laquelle
A, B, Q, L, X, Y, Z, R⁴, R⁵ et n ont la définition indiquée ci-dessus,
on fait réagir des 3-aryl-pyrrolidine-2,4-diones de formule (Ia) ou leurs énols 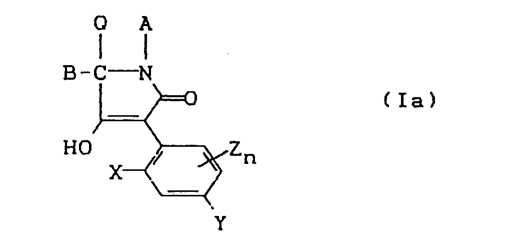 dans laquelle
A, B, Q, X, Y, Z et n ont la définition indiquée ci-dessus,
avec des composés de phosphore de formule générale (IX) 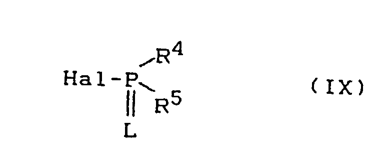 dans laquelle
L, R⁴ et R⁵ ont les définitions indiquées ci-dessus
et
Hal représente un halogène, en particulier le chlore et le brome ;
ou bien
(G) pour obtenir des composés de formule (If) 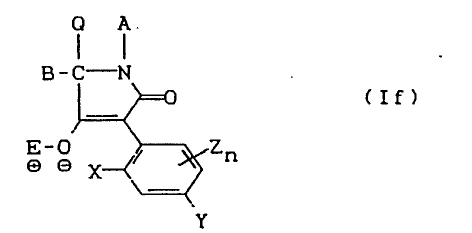 dans laquelle
A, B, Q, X, Y, Z et n ont la définition indiquée cidessus,
et
E est un équivalent d'ion métallique ou un ion ammonium,
on fait réagir des composés de formule (Ia) 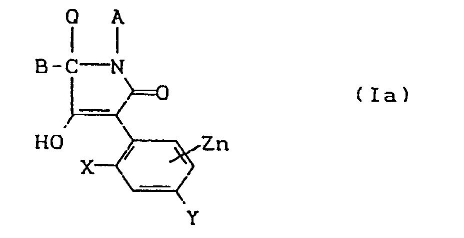 dans laquelle
A, B, Q, X, Y, Z et n ont la définition indiquée ci-dessus,
avec des hydroxydes de métaux ou des amines de formules générales (X) et (XI) 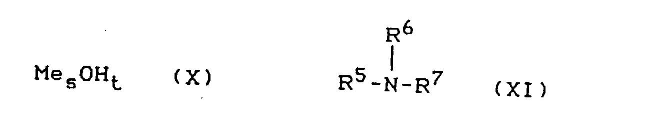 dans lesquelles
Me représente des ions de métaux monovalents ou divalents
s et t représentent les nombres 1 et 2 et
R⁵, R⁶ et R⁷ représentent, indépendamment les uns des autres, l'hydrogène et un groupe alkyle,
le cas échéant en présence d'un diluant.

5. Compositions pesticides, caractérisées par une teneur en au moins un dérivé bicyclique substitué de 3-arylpyrrolidine-2,4-dione de formule (I).

6. Procédé pour combattre des parasites animaux, caractérisé en ce qu'on fait agir sur des parasites animaux et/ou sur leur milieu des dérivés bicycliques substitués de 3-aryl-pyrrolidine-2,4-diones de formule (I).

7. Utilisation de dérivés bicycliques substitués de 3-aryl-pyrrolidine-2,4-diones de formule (I) pour combattre des parasites animaux.

8. Procédé de préparation de compositions pesticides, caractérisé en ce qu'on mélange des dérivés bicycliques substitués de 3-aryl-pyrrolidine-2,4-diones de formule (I) avec des diluants et/ou des agents tensio-actifs.

9. Compositions herbicides, caractérisées par une teneur en au moins un dérivé bicyclique substitué de 3-arylpyrrolidine-2,4-dione de formule (I).

10. Procédé pour combattre des mauvaises herbes, caractérisé en ce qu'on fait agir sur des mauvaises herbes et/ou sur leur milieu des dérivés bicycliques substitués de 3-aryl-pyrrolidine-2,4-diones de formule (I).

11. Utilisation de dérivés bicycliques substitués de 3-aryl-pyrrolidine-2,4-diones de formule (I) pour combattre des mauvaises herbes.

12. Procédé de préparation de compositions herbicides, caractérisé en ce qu'on mélange des dérivés bicycliques substitués de 3-aryl-pyrrolidine-2,4-diones de formule (I) avec des diluants et/ou des agents tensio-actifs.

## Revendications (Revendications pour l'(les) Etat(s) contractant(s) suivant(s): ES)

1. Procédé de production de dérivés bicycliques substitués de 3-aryl-pyrrolidine-2,4-diones de formule (I) 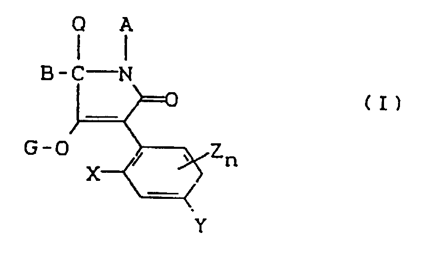 dans laquelle
A et Q forment en commun un groupement alcanediyle, alcènediyle, alcènediényle ou alcénetriényle de 3 à 6 atomes de carbone, qui est substitué dans chaque cas une à trois fois identiques ou différentes par un halogène, un groupe hydroxy, mercapto, un groupe alkyle ayant 1 à 10 atomes de carbone, alkylcarbonyloxy ayant 1 à 8 atomes de carbone dans la partie alkyle, alkoxy ayant 1 à 6 atomes de carbone, alkylthio ayant 1 à 6 atomes de carbone, cycloalkyle ayant 3 à 7 atomes de carbone ou aryle ayant 6 à 10 atomes de carbone substitué dans chaque cas éventuellement par un halogène, et qui est en outre interrompu, le cas échéant par l'un des groupements suivants : 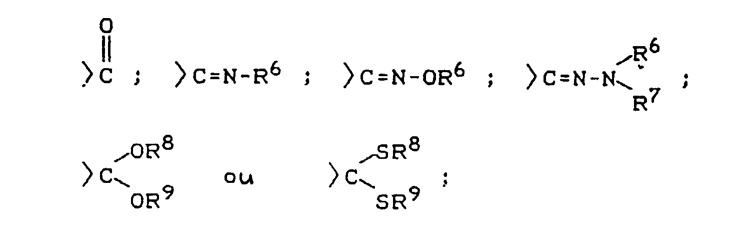
B représente l'hydrogène ou un groupe alkyle ayant 1 à 6 atomes de carbone,
X est un groupe alkyle en C₁ à C₆, un halogène ou un groupe alkoxy en C₁ à C₆,
Y est l'hydrogène, un groupe alkyle en C₁ à C₆, un halogène, un groupe alkoxy en C₁ à C₆ ou un groupe halogénalkyle en C₁ à C₃,
Z est un groupe alkyle en C₁ à C₆, un halogène ou un groupe alkoxy en C₁ à C₆,
n représente le nombre 0, 1, 2 ou 3,
G est l'hydrogène (a) ou les groupes 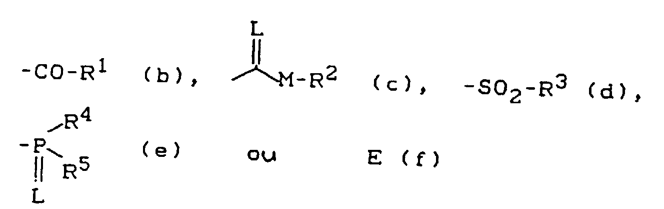
dans lesquels
E représente un équivalent d'ion métallique ou un ion ammonium,
L et M représentent l'oxygène et/ou le soufre,
R¹ est un groupe, éventuellement substitué par un halogène, alkyle en C₁ à C₂₀, alcényle en C₂ à C₂₀, (alkoxy en C₁ à C₈)-(alkyle en C₂ à C₈), (alkylthio en C₁ à C₈)-(alkyle en C₂ à C₈), poly(alkoxy en C₁ à C₈)-(alkyle en C₂ à C₈) ou cycloalkyle à noyau de 3 à 8 atomes de carbone, qui peut être interrompu par des atomes d'oxygène et/ou de soufre,
un groupe phényle éventuellement substitué par un radical halogéno, nitro, alkyle en C₁ à C₆, alkoxy en C₁ à C₆, halogénalkyle en C₁ à C₆, halogénalkoxy en C₁ à C₆,
un groupe phényl-(alkyle en C₁ à C₆) éventuellement substitué par un radical halogéno, alkyle en C₁ à C₆, alkoxy en C₁ à C₆, halogénalkyle en C₁ à C₆, halogénalkoxy en C₁ à C₆,
un groupe hétaryle éventuellement substitué par un radical halogéno et/ou alkyle en C₁ à C₆,
un groupe phénoxy-(alkyle en C₁ à C₆) éventuellement substitué par un radical halogéno et un radical alkyle en C₁ à C₆,
un groupe hétaryloxy-(alkyle en C₁ à C₆) éventuellement substitué par un radical halogéno, amino et alkyle en C₁ à C₆,
R² est un groupe, éventuellement substitué par un halogène, alkyle en C₁ à C₂₀, alcényle en C₂ à C₂₀, (alkoxy en C₁ à C₈)-(alkyle en C₂ à C₈), poly(alkoxy en C₁ à C₈)-(alkyle en C₂ à C₈),
un groupe phényle ou benzyle éventuellement substitué par un radical halogéno, nitro, alkyle en C₁ à C₆, alkoxy en C₁ à C₆, halogénalkyle en C₁ à C₆,
R³, R⁴ et R⁵ représentent, indépendamment les uns des autres, un groupe, éventuellement substitué par un halogène, alkyle en C₁ à C₈, alkoxy en C₁ à C₈, alkylamino en C₁ à C₈, di-(alkyle en C₁ à C₈)-amino, alkylthio en C₁ à C₈, alcénylthio en C₂ à C₅, alcynylthio en C₂ à C₅, cycloalkylthio en C₃ à C₇, un groupe phényle, phénoxy ou phénylthio éventuellement substitué par un radical halogéno, nitro, cyano, alkoxy en C₁ à C₄, halogénalkoxy en C₁ à C₄, alkylthio en C₁ à C₄, halogénalkylthio en C₁ à C₄, alkyle en C₁ à C₄, halogénalkyle en C₁ à C₄,
R⁶ et R⁷ représentent, indépendamment l'un de l'autre, l'hydrogène, un groupe, éventuellement substitué par un halogène, alkyle en C₁ à C₂₀, alkoxy en C₁ à C₂₀, alcényle en C₂ à C₈, (alkoxy en C₁ à C₂₀)-(alkyle en C₁ à C₂₀), un groupe phényle éventuellement substitué par un radical halogéno, halogénalkyle en C₁ à C₂₀, alkyle en C₁ à C₂₀ ou alkoxy en C₁ à C₂₀, un groupe benzyle éventuellement substitué par un radical halogéno, alkyle en C₁ à C₂₀, halogénalkyle en C₁ à C₂₀ ou alkoxy en C₁ à C₂₀, ou forment ensemble un noyau alkylène en C₂ à C₆ éventuellement interrompu par de l'oxygène,
R⁸ et R⁹ représentent, indépendamment l'un de l'autre, l'hydrogène ou un groupe alkyle ayant 1 à 6 atomes de carbone, ou bien
R⁸ et R⁹ forment ensemble un reste alcanediyle ayant 2 à 4 atomes de carbone,
caractérisé en ce que
(Aα) pour obtenir des 3-aryl-pyrrolidine-2,4-diones et leurs énols de formule (Ia) 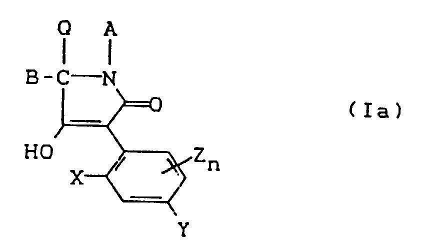 dans laquelle
A, B, Q, X, Y, Z et n ont la définition indiquée ci-dessus,
on effectue la condensation intramoléculaire d'un ester de N-acylamino-acide de formule (II) 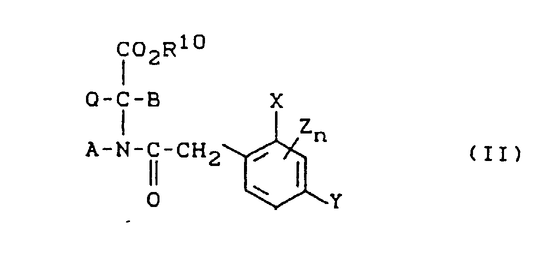 dans laquelle
A, B, Q, X, Y, Z et n ont la définition indiquée ci-dessus, et
R¹⁰ est un groupe alkyle,
en présence d'un diluant et en présence d'une base,
ou bien
(A-β) on transforme en esters ou éthers les composés, obtenus selon le procédé (Aα), de formule (Ia-a) 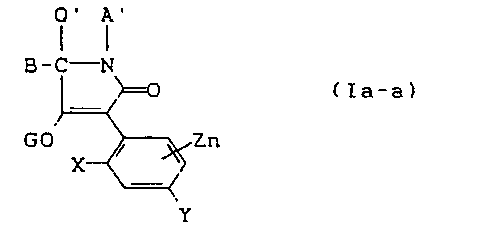 dans laquelle
B, X, Y, Z, n et G ont la définition indiquée ci-dessus et
A' et Q' forment ensemble un groupement alcanediyle, alcènediyle, alcènediényle ou alcènetriényle substitué par un radical hydroxy
au niveau du groupement hydroxy selon des procédés généraux classiques, ou bien on transforme dans une première étape le groupement hydroxy en les cétones correspondantes à l'aide de modes opératoires classiques d'oxydation et on fait réagir ces cétones dans une seconde étape selon des modes opératoires également classiques avec des thiènes, des alcools, des diols, des mercaptans ou des dithiols,
ou bien
(B) pour obtenir des composés de formule (Ib) 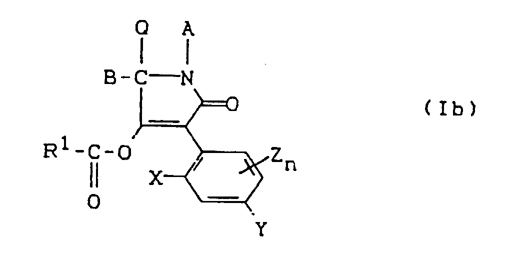 dans laquelle
A, B, Q, X, Y, Z, R¹ et n ont la définition indiquée ci-dessus,
des composés de formule (Ia), 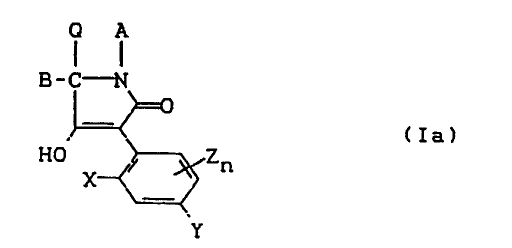 dans laquelle
A, B, Q, X, Y, Z et n ont la définition indiquée ci-dessus,
sont amenés à réagir
α) avec des halogénures d'acides de formule générale (III) 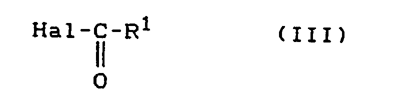 dans laquelle
R¹ a la définition indiquée ci-dessus, et
Hal représente un halogène, en particulier le chlore et le brome,
le cas échéant en présence d'un diluant et en la présence éventuelle d'un accepteur d'acide
ou bien
β) avec des anhydrides d'acides carboxyliques de formule générale (IV)
R¹-CO-O-CO-R¹ (IV)
dans laquelle
R¹ a la définition indiquée ci-dessus,
le cas échéant en présence d'un diluant et en la présence éventuelle d'un accepteur d'acide,
ou bien
(C) pour obtenir des composés de formule (Ic-1) 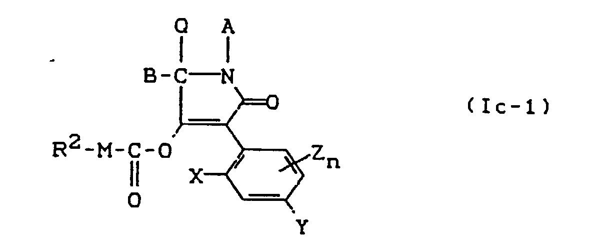 dans laquelle
A, B, Q, X, Y, Z, R² et n ont la définition indiquée ci-dessus,
et
M est l'oxygène ou le soufre,
on fait réagir des composés de formule (Ia) 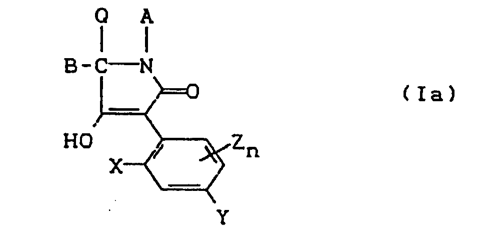 dans laquelle
A, B, Q, X, Y, Z et n ont la définition indiquée ci-dessus,
avec un ester d'acide chloroformique ou un thioester d'acide chloroformique de formule générale (V)
R²-M-CO-Cl (V)
dans laquelle
R² et M ont la définition indiquée ci-dessus,
le cas échéant en présence d'un diluant et en la présence éventuelle d'un accepteur d'acide ;
ou bien
(D) pour obtenir des composés de formule (Ic-2) 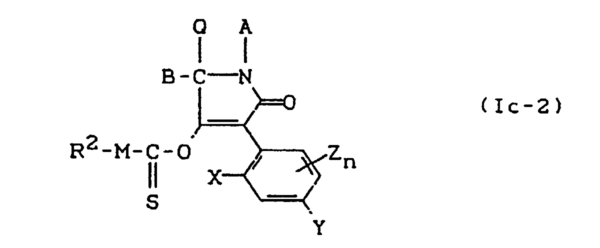 dans laquelle
A, B, Q, R², X, Y, Z et n ont la définition indiquée ci-dessus,
et
M représente l'oxygène ou le soufre,
on fait réagir des composés de formule (Ia) 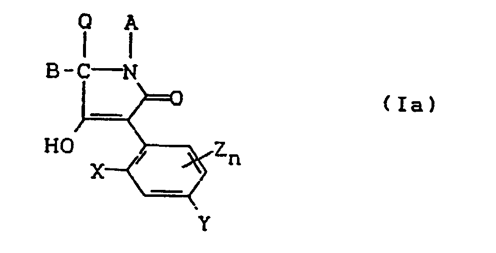 dans laquelle
A, B, Q, X, Y, Z et n ont la définition indiquée ci-dessus,
α) avec des esters d'acide chloromonothioformique ou des esters d'acide chlorodithioformique de formule générale (VI) 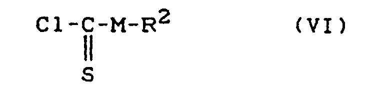 dans laquelle
M et R² ont la définition indiquée ci-dessus,
le cas échéant en présence d'un diluant et en la présence éventuelle d'un accepteur d'acide,
ou bien
β) avec le sulfure de carbone, puis avec des halogénures d'alkyle de formule générale (VII)
R²-Hal (VII)
dans laquelle
R² a la définition indiquée ci-dessus
et
Hal représente le chlore, le brome, l'iode ;
ou bien
(E) pour obtenir des composés de formule (Id) 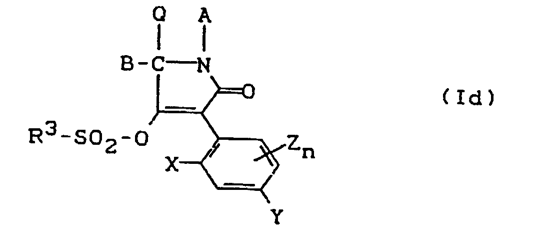 dans laquelle
A, B, Q, X, Y, Z, R³ et n ont la définition indiquée ci-dessus,
on fait réagir des composés de formule (Ia) 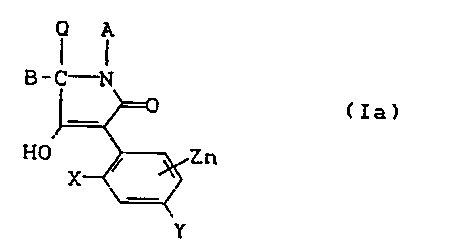 dans laquelle
A, B, Q, X, Y, Z et n ont la définition indiquée ci-dessus,
avec des chlorures d'acides sulfoniques de formule générale (VIII)
R³-SO₂-Cl (VIII)
dans laquelle
R³ a la définition indiquée ci-dessus,
le cas échéant en présence d'un diluant et en la présence éventuelle d'un accepteur d'acide ;
ou bien
(F) pour obtenir des 3-aryl-pyrrolidine-2,4-diones de formule (Ie) 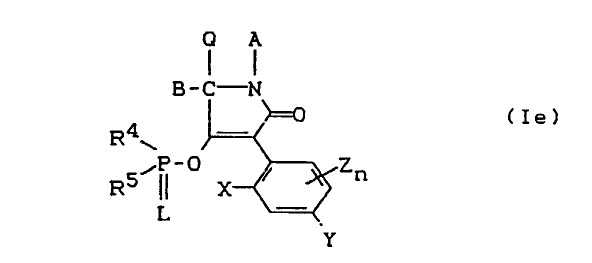 dans laquelle
A, B, Q, L, X, Y, Z, R⁴, R⁵ et n ont la définition indiquée ci-dessus,
on fait réagir des 3-aryl-pyrrolidine-2,4-diones de formule (Ia) ou leurs énols 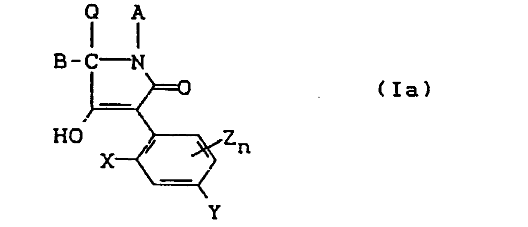 dans laquelle
A, B, Q, X, Y, Z et n ont la définition indiquée ci-dessus,
avec des composés de phosphore de formule générale (IX) 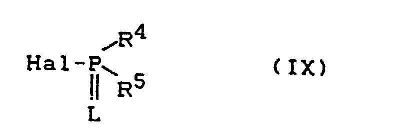 dans laquelle
L, R⁴ et R⁵ ont les définitions indiquées ci-dessus
et
Hal représente un halogène, en particulier le chlore et le brome ;
ou bien
(G) pour obtenir des composés de formule (If) 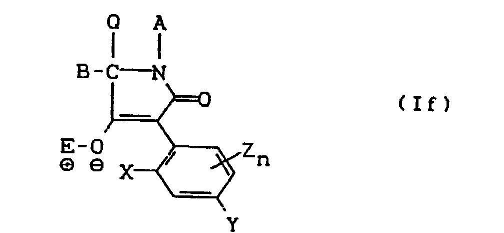 dans laquelle
A, B, Q, X, Y, Z et n ont la définition indiquée ci-dessus,
et
E est un équivalent d'ion métallique ou un ion ammonium,
on fait réagir des composés de formule (Ia) 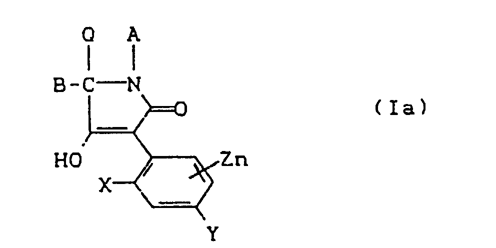 dans laquelle
A, B, Q, X, Y, Z et n ont la définition indiquée ci-dessus,
avec des hydroxydes de métaux ou des amines de formules générales (X) et (XI) 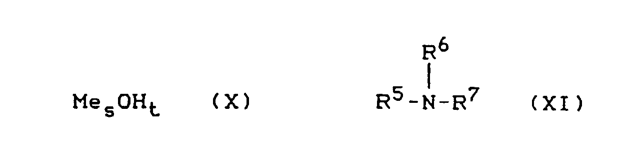 dans lesquelles
Me représente des ions de métaux monovalents ou divalents
s et t représentent les nombres 1 et 2 et
R⁵, R⁶ et R⁷ représentent, indépendamment les uns des autres, l'hydrogène et un groupe alkyle,
le cas échéant en présence d'un diluant.

2. Compositions pesticides, caractérisées par une teneur en au moins un dérivé bicyclique substitué de 3-arylpyrrolidine-2,4-dione de formule (I).

3. Procédé pour combattre des parasites animaux, caractérisé en ce qu'on fait agir sur des parasites animaux et/ou sur leur milieu des dérivés bicycliques substitués de 3-aryl-pyrrolidine-2,4-diones de formule (I).

4. Utilisation de dérivés bicycliques substitués de 3-aryl-pyrrolidine-2,4-diones de formule (I) pour combattre des parasites animaux.

5. Procédé de préparation de compositions pesticides, caractérisé en ce qu'on mélange des dérivés bicycliques substitués de 3-aryl-pyrrolidine-2,4-diones de formule (I) avec des diluants et/ou des agents tensio-actifs.

6. Compositions herbicides, caractérisées par une teneur en au moins un dérivé bicyclique substitué de 3-arylpyrrolidine-2,4-dione de formule (I).

7. Procédé pour combattre des mauvaises herbes, caractérisé en ce qu'on fait agir sur des mauvaises herbes et/ou sur leur milieu des dérivés bicycliques substitués de 3-aryl-pyrrolidine-2,4-diones de formule (I).

8. Utilisation de dérivés bicycliques substitués de 3-aryl-pyrrolidine-2,4-diones de formule (I) pour combattre des mauvaises herbes.

9. Procédé de préparation de compositions herbicides, caractérisé en ce qu'on mélange des dérivés bicycliques substitués de 3-aryl-pyrrolidine-2,4-diones de formule (I) avec des diluants et/ou des agents tensio-actifs.
